# EUROPEAN PATENT SPECIFICATION

(11) **EP 1 960 539 B1**
(45) Date of publication and mention of the grant of the patent: **19.09.2012**
(21) Application number: 06820543.4
(22) Date of filing: 15.12.2006
(51) Int. Cl.: C12Q 1/68

(54) **IDENTIFICATION OF LOSS-OF-FUNCTION MUTATIONS IN FILAGGRIN CAUSING ICHTHYOSIS VULGARIS AND PREDISPOSING TO OTHER DISEASES**
IDENTIFIZIERUNG VON ZU ICHTHYOSIS VULGARIS UND EINER VERANLAGUNG FÜR ANDERE KRANKHEITEN FÜHRENDEN FUNKTIONSVERLUSTMUTATIONEN IN FILAGGRIN
IDENTIFICATION DE MUTATIONS PERTE-DE-FONCTION DANS LA FILAGGRINE QUI ENTRAINENT ICHTHYOSIS VULGARIS ET PRÉDISPOSENT À D'AUTRES MALADIES

(30) Priority: 15.12.2005 GB 0525492
(43) Date of publication of application: 27.08.2008
(73) Proprietor: University Court of The University of Dundee, Dundee DD1 4HN (GB)
(72) Inventor: MCLEAN, William, Henry, Irwin, Dundee DD1 9SY (GB); SMITH, Frances, Jane, Dorothy, Dundee DD1 9SY (GB)
(74) Representative: Chapman, Paul Gilmour
(86) International application number: PCT/GB2006/004707
(87) International publication number: WO 2007/068946

(56) References cited:
- WO-A-99/28344
- WO-A-02/090583
- NIRUNSUKSIRI WILAS ET AL: "Reduced stability and bi-allelic, coequal expression of profilaggrin mRNA in keratinocytes cultured from subjects with Ichthyosis vulgaris" JOURNAL OF INVESTIGATIVE DERMATOLOGY, vol. 110, no. 6, June 1998 (1998-06), pages 854-861, XP002444718 ISSN: 0022-202X
- DALE B A ET AL: "CHARACTERIZATION OF TWO MONOCLONAL ANTIBODIES TO HUMAN EPIDERMAL KERATOHYALIN REACTIVITY WITH FILAGGRIN AND RELATED PROTEINS" JOURNAL OF INVESTIGATIVE DERMATOLOGY, vol. 88, no. 3, 1987, pages 306-313, XP002444719 ISSN: 0022-202X
- DATABASE NCBI SNP [Online] XP002445421 retrieved from HTTP://WWW.NCBI.NLM.NIH.GOV/SNP/SNP_SS.CGI ?SUBSNP_ID=3122822
- PALMER COLIN N A ET AL: "Common loss-of-function variants of the epidermal barrier protein filaggrin are a major predisposing factor for atopic dermatitis" NATURE GENETICS, vol. 38, no. 4, April 2006 (2006-04), pages 441-446, XP002444720 ISSN: 1061-4036
- SMITH FRANCES J D ET AL: "Loss-of-function mutations in the gene encoding filaggrin cause ichthyosis vulgaris" NATURE GENETICS, vol. 38, no. 3, March 2006 (2006-03), pages 337-342, XP002444721 ISSN: 1061-4036
- SANDILANDS AILEEN ET AL: "Prevalent and rare mutations in the gene encoding filaggrin cause ichthyosis vulgaris and predispose individuals to atopic dermatitis" JOURNAL OF INVESTIGATIVE DERMATOLOGY, vol. 126, no. 8, August 2006 (2006-08), pages 1770-1775, XP002444722 ISSN: 0022-202X
- HUFFMEIER U ET AL: "Loss-of-function variants of the filaggrin gene are not major susceptibility factors for psoriasis vulgaris or psoriatic arthritis in German patients" JOURNAL OF INVESTIGATIVE DERMATOLOGY 2007 UNITED STATES, vol. 127, no. 6, 2007, pages 1367-1370, XP002444723 ISSN: 0022-202X 1523-1747
- PRESLAND R B ET AL: "Loss of normal profilaggrin and filaggrin in flaky tail (ft/ft) mice: an animal model for the filaggrin-deficient skin disease ichthyosis vulgaris.", THE JOURNAL OF INVESTIGATIVE DERMATOLOGY DEC 2000 LNKD- PUBMED:11121144, vol. 115, no. 6, December 2000 (2000-12), pages 1072-1081, ISSN: 0022-202X
- FALLON PADRAIC G ET AL: "A homozygous frameshift mutation in the mouse Flg gene facilitates enhanced percutaneous allergen priming.", NATURE GENETICS MAY 2009 LNKD- PUBMED:19349982, vol. 41, no. 5, May 2009 (2009-05), pages 602-608, ISSN: 1546-1718
- CHAOYONG MA: "Animal models of diesease", MODERN DRUG DISCOVERY, June 2004 (2004-06), pages 30-35,

## Description

### Field of the Invention

The present invention relates to the identification of loss-of-function mutations in the filaggrin gene and their use in diagnosing ichthyosis vulgaris and/or susceptibility to other diseases including atopic dermatitis (eczema), asthma and allergies (including food allergy).

### Background to the Invention

Ichthyosis vulgaris (IV; OMIM# 146700) is the most common inherited disorder of keratinisation and one of the most frequent single gene disorders in humans. The most widely cited incidence figure is 1 in 250 based on a survey of 6051 healthy English schoolchildren¹.

The phenotypic characteristics of IV include palmar hyperlinearity, keratosis pilaris and a fine scale most markedly seen over the lower abdomen, arms and legs². Filaggrin (filament aggregating protein) is important in the formation of the stratum corneum³⁻⁵. Keratohyalin granules in the granular layer of interfollicular epidermis are predominantly composed of the 400 kDa protein profilaggrin. Following a short, unique N-terminal domain, most of the profilaggrin molecule consists of 10-12 repeats of the 324 amino acid filaggrin sequence⁶. Upon terminal differentiation of granular cells, profilaggrin is proteolytically cleaved into ∼37 kDa filaggrin peptides and the N-terminal domain containing an S100-like calcium binding domain. Filaggrin rapidly aggregates the keratin cytoskeleton, causing collapse of the granular cells into flattened anuclear squames. This condensed cytoskeleton is cross-linked by transglutaminases during formation of the cornified cell envelope (CCE). The CCE is the outermost barrier layer of the skin which not only prevents water loss but also impedes the entry of allergens and infectious agents⁷. Filaggrin is therefore a key protein in facilitating epidermal differentiation and maintaining barrier function.

Immunoblotting studies have shown that filaggrin protein was absent or markedly reduced in IV patients' skin and/or keratinocytes⁸⁻¹⁰. In addition, decreased filaggrin mRNA has been demonstrated in some individuals with IV¹¹. A recessive mouse mutant, flaky tail (*ft*), bears the histological and ultrastructural hallmarks of human IV¹² and strong genetic linkage has been obtained to the murine filaggrin locus (*FLG*)^{13,14}. Although biochemical analysis has shown defective profilaggrin processing in *ft*/*ft* homozygotes¹², any genomic mutation in the *FLG* gene has not hitherto been identified.

It is amongst the objects of the present invention to provide a method of diagnosing ichthyosis vulgaris and atopic diseases or predisposition thereto.

### Summary of the Invention

The present invention is based on the identification for the first time of mutations in the human filaggrin (*FLG*) gene which lead to a loss or partial loss of protein function.

Thus, in a first aspect, the present invention provides a genetic test for the diagnosis of ichthyosis vulgaris (IV) comprising the steps of:
detecting *in vitro* whether or not a mutation, which would lead to a loss of function or partial loss of function of the filaggrin (FLG) protein encoded by the filaggrin (*FLG*) gene, is present in the *FLG* gene of a sample of nucleic acid from a subject.

It will be appreciated that the test may be used to diagnose IV and/or to test if a subject is predisposed to developing IV. Additionally, due to an association of IV, in severe or mild forms, with other diseases, the test may be used to also detect whether or not a subject is likely to be predisposed or suffering from atopic dermatitis (eczema), asthma or allergies, such as of a contact type allergy and food allergies (for example, peanut allergy). With regards to skin conditions, low levels of filaggrin expression may lead to development of mild and/or sub-clinical disease. In this manner, the present invention may also relate to the identification and/or treatment of said mild and/or sub-clinical forms of disease. Indeed, many skin conditions go undiagnosed and as such treatments may be considered more as a cosmetic treatment.

Thus, in a further aspect, the present invention provides a genetic test for atopic dermatitis (eczema), asthma and/or allergies comprising the steps of:
detecting *in vitro* whether or not a mutation, which would lead to a loss of function or partial loss of function of the FLG protein encoded by the *FLG* gene, is present in the *FLG* gene of a sample of nucleic acid from a subject.

The sample to be tested may be any suitable sample from which nucleic acid may be obtained. Typically the nucleic acid is a sample of genomic DNA or mRNA. Conveniently the sample may be a sample of saliva, buccal scraping or blood sample. The sample may also be a tissue sample, such as a skin biopsy.

The subject may be any subject requiring to be tested and may suitably be a newborn or even a foetus. The subject may however be at any stage of life, and therefore includes neonates, children and adults. As mentioned above, said tests may be carried out in order to ascertain whether or not the subject is predisposed to developing a disease. Thus, a test may be carried out, for example, in order to test a subject's suitability for a particular job, where he/she may come into contact with agents which are known to lead in some cases to the development of eczema and/or allergy. Alternatively, said test may be carried out in order to categorise a subject and predict an "at risk" status for, for example, atopic disease. In this manner subjects may be tested so as to categorise or stratify subjects for therapeutic intervention, when appropriate, based on any results obtained, so as to prevent and/or treat atopic disease.

Moreover, ascertaining a subject's *FLG* status and therefore degree of expression or lack of expression of the FLG protein may find use in determining suitable treatment for a subject suffering or predisposed to suffering from IV and/or any of the other aforementioned diseases. For example, depending on the degree of severity, or expected degree of severity, the skilled artisan can decide on an appropriate therapeutic and/or cosmetic regime and as such tailored treatments can be based on a subjects *FLG* status.

The present invention, in one embodiment, is based on the identification of previously unidentified mutations in the *FLG* gene, which lead to a loss of function of the profilaggrin and consequently filaggrin proteins. The present invention however extends to any mutation in the *FLG* gene which leads to a loss or partial loss of function of the profilaggrin and/or filaggrin proteins.

The mutation may be an addition, deletion, substitution or inversion. Typically, the mutation effects 1 - 10 nucleotides, such as a one-base substitution, or a 2 - 10 e.g. 4-base deletion. The mutation may also be due to a translocation. By partial or total loss of profilaggrin or filaggrin protein function, is understood to mean that the mutation or mutations result in incorrect processing and/or expression of the *FLG* gene such that one or more of the filaggrin peptides normally expressed, is not functionally expressed. Typically 10 - 12 copies of the filaggrin peptide are expressed from a non-mutated *FLG* gene⁶. It is understood therefore that the mutant *FLG* genes of the present invention will result in the functional expression of less than 10-12 filaggrin peptides, typically less than 7, 5, 3 or 1 from one or both copies of the *FLG* gene, which are present in a genome.

Depending on the location and/or type of a mutation or mutations, any reduction in functional filaggrin expression can be mild, e.g. a 1 - 5 reduction in functional filaggrin peptides; significant e.g. a 7 -13 reduction in functional filaggrin peptides; or severe, e.g. a 15 - 20 reduction in functional filaggrin peptides.

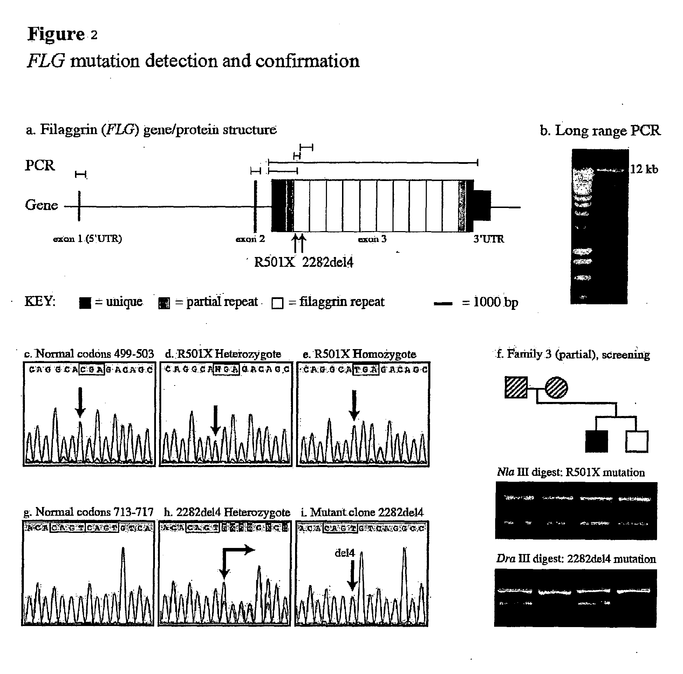

The mutation or mutations may be found in any of the exons 1, 2 and/or 3 of the *FLG* gene and may typically be found in exon 3. If the mutation or mutations is/are located in exon 3, the most detrimental mutations, with regards to functional filaggrin expression, will be found within the 5' (N-terminal) portion of the 3rd exon, such as within the first 2000 bases, e.g. mutation(s) is/are found within the unique, partial repeat, or first filaggrin repeat portion of exon 3 (see Figure 2a).

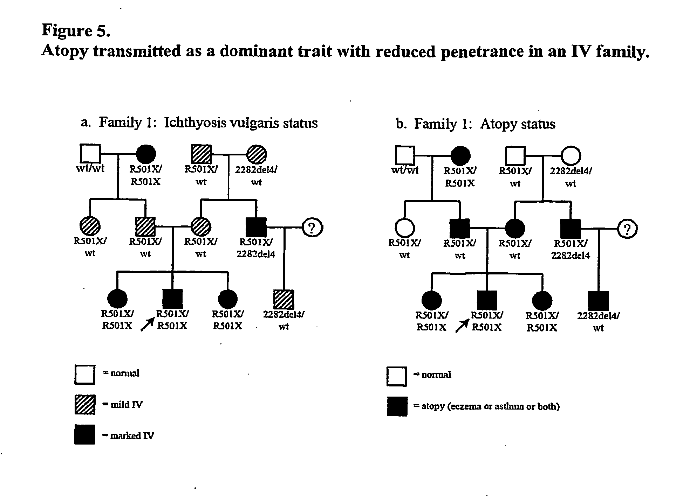

A significant number of mutations have been identified by the present inventors, which lead to a loss of function and in some cases, a total loss of function of one of the *FLG* copies. One such mutation is a 1-base substitution at position 1501 of the *FLG* gene herein (as shown in Figure 5 and SEQ ID NO.: 188). 1501C>T (numbering from initiating ATG), which results in the substitution of a cytidine by a thymidine and a corresponding amino.acid change at position 501 of an arginine to a stop codon. As this mutation occurs in the first filaggrin repeat (see Figure 2) and results in the generation of a stop codon, no functional copies of the filaggrin peptide are produced.

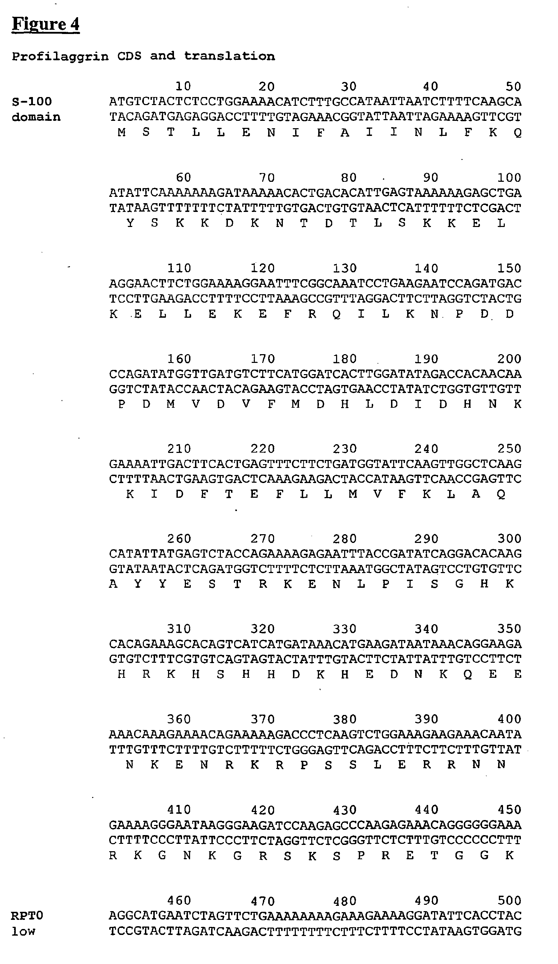

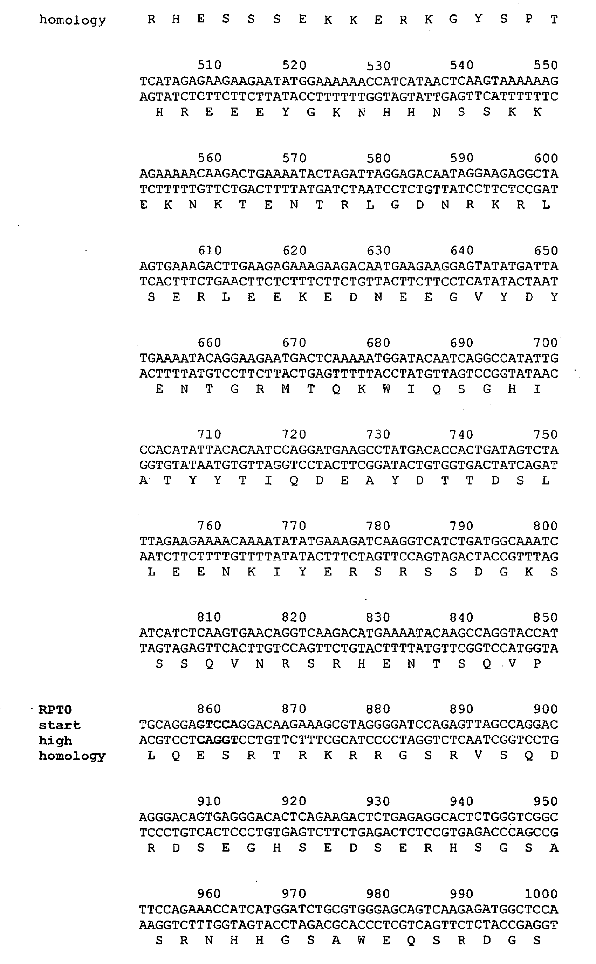

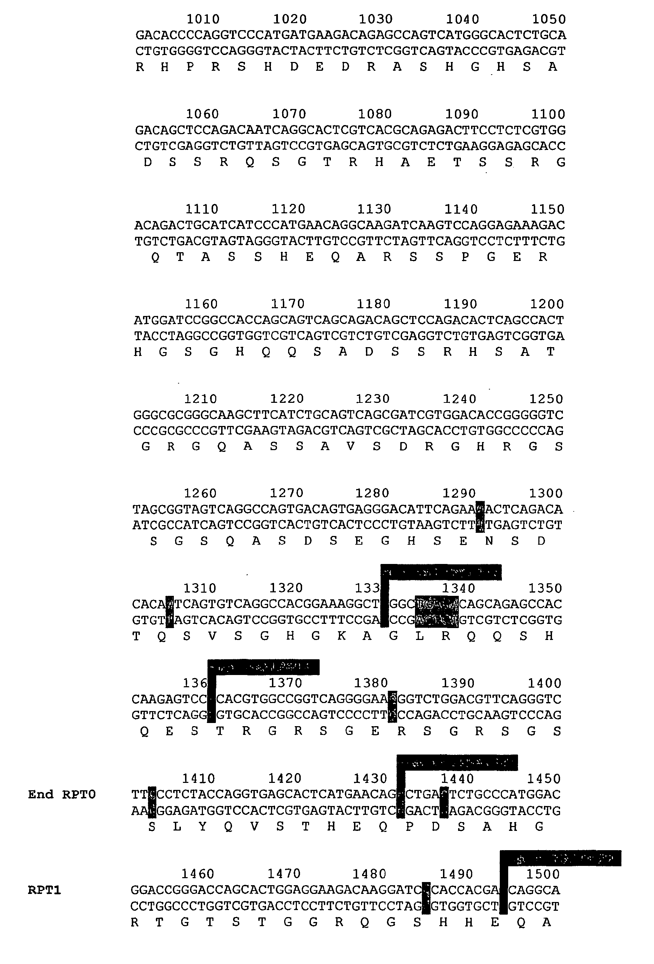

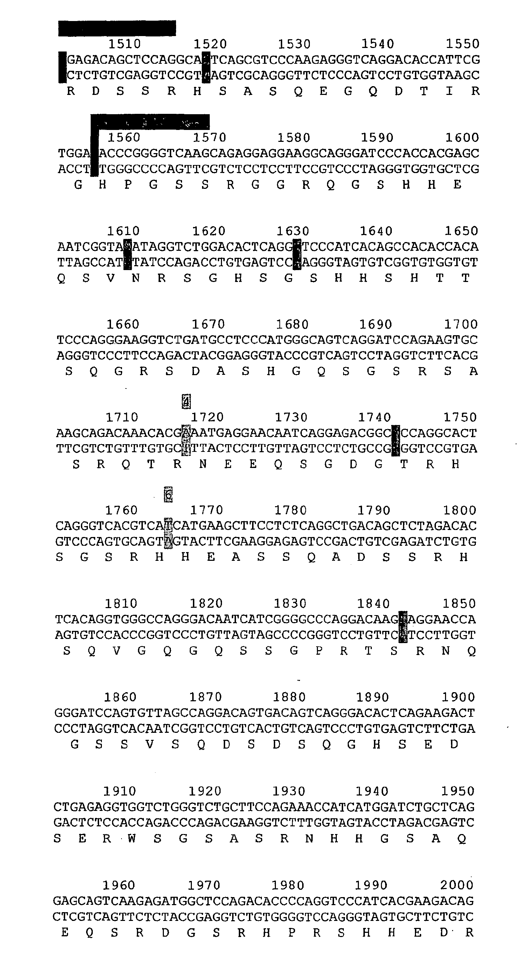

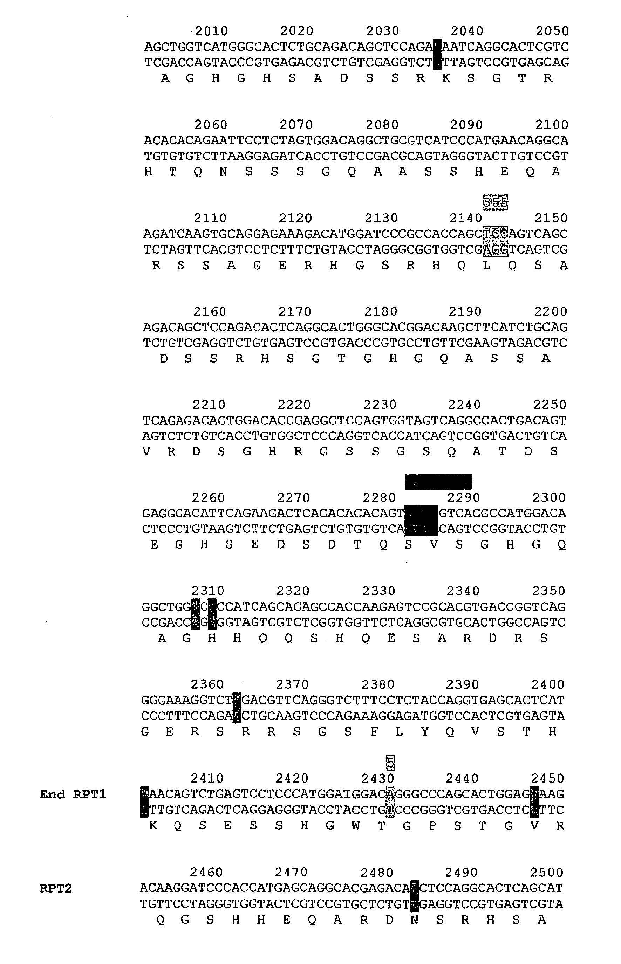

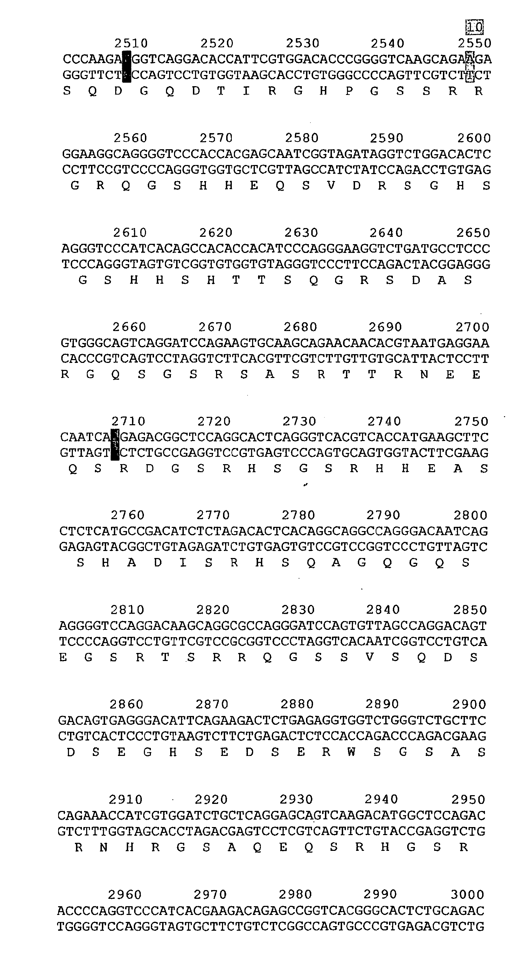

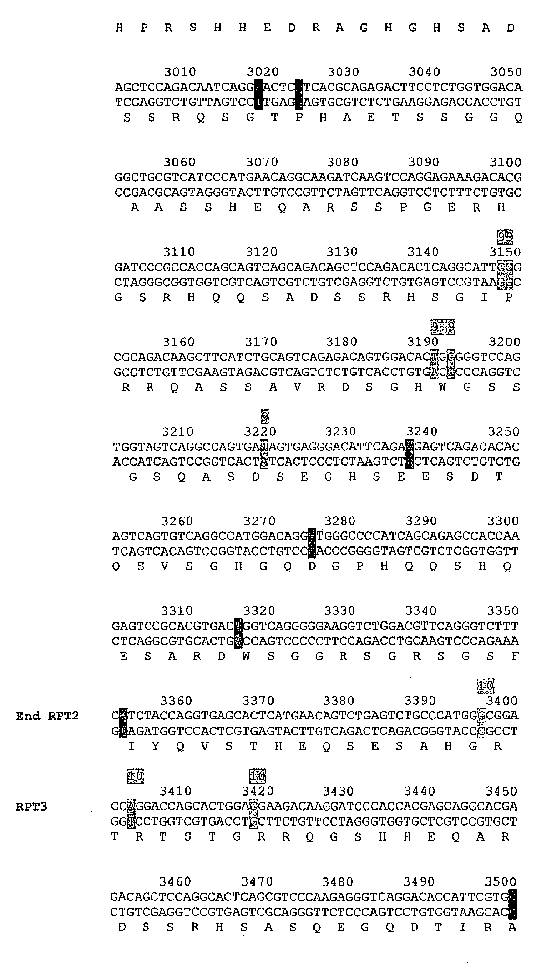

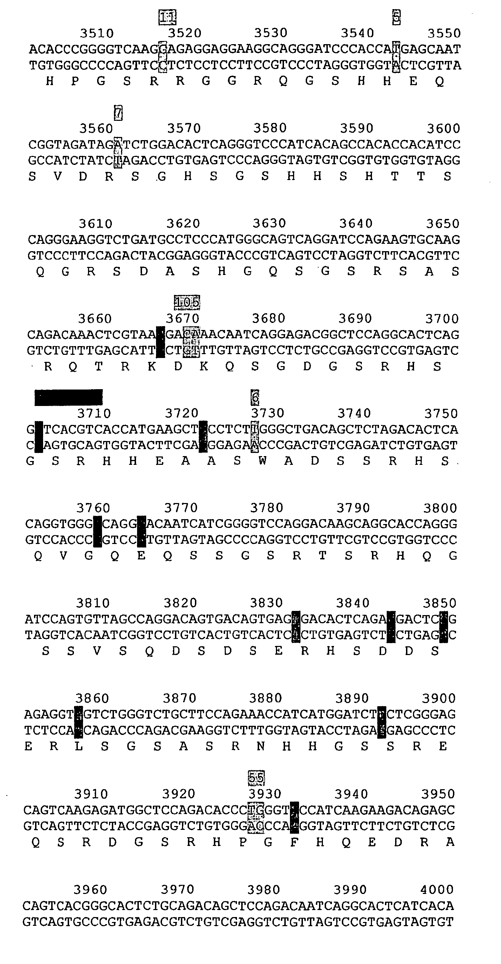

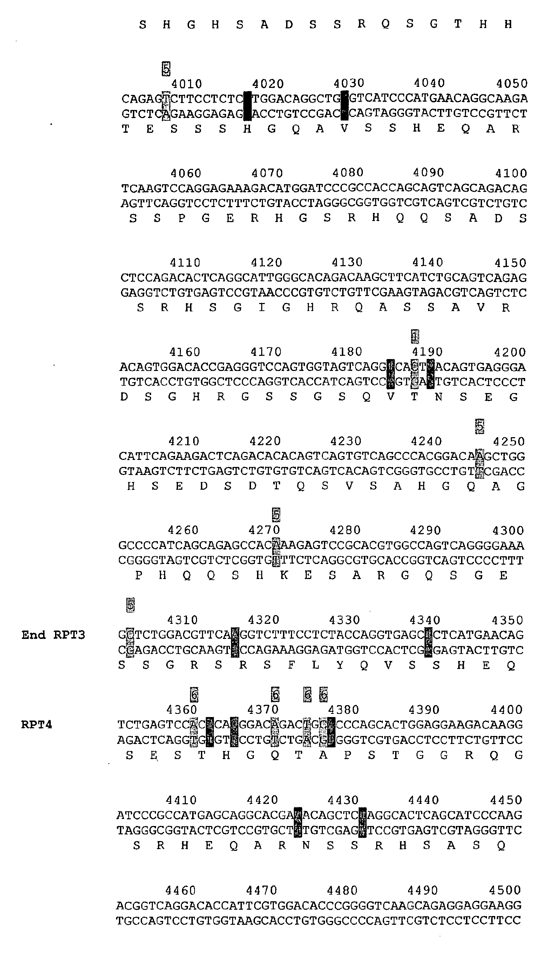

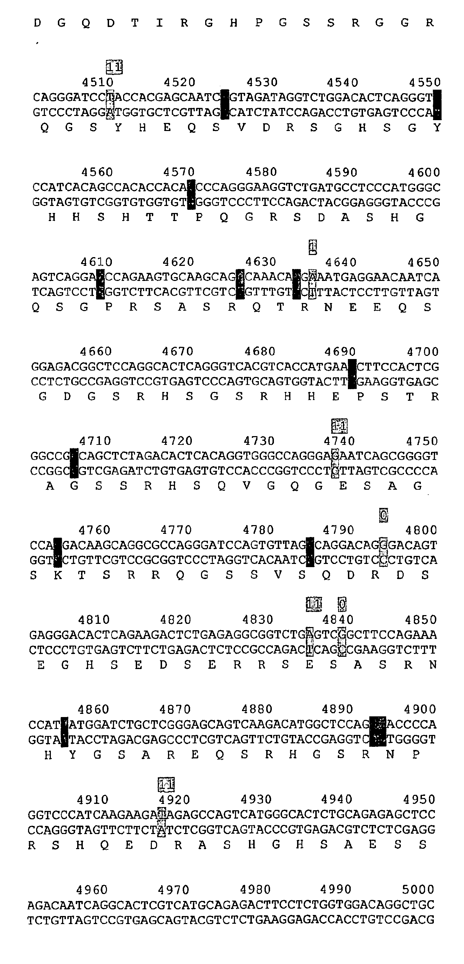

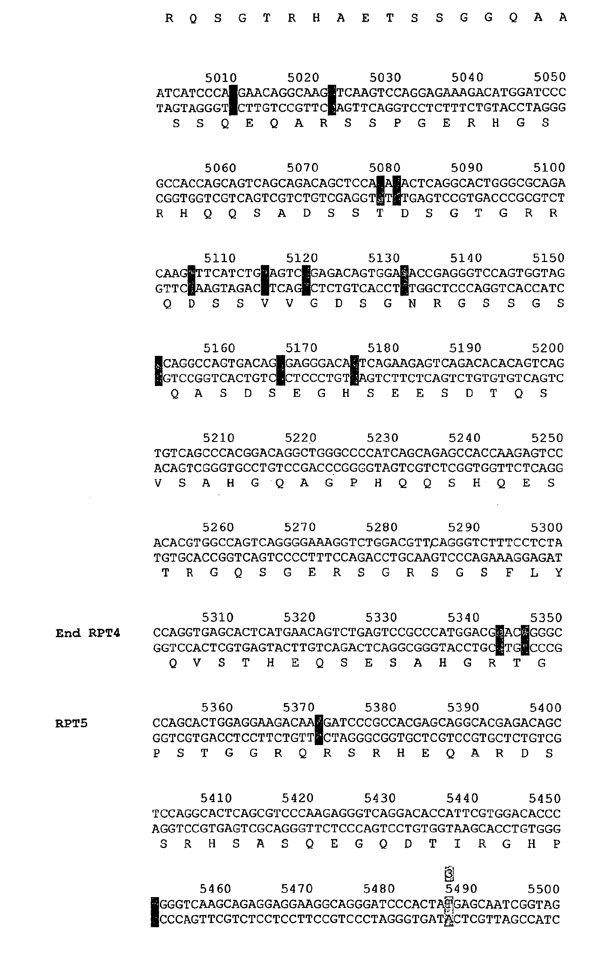

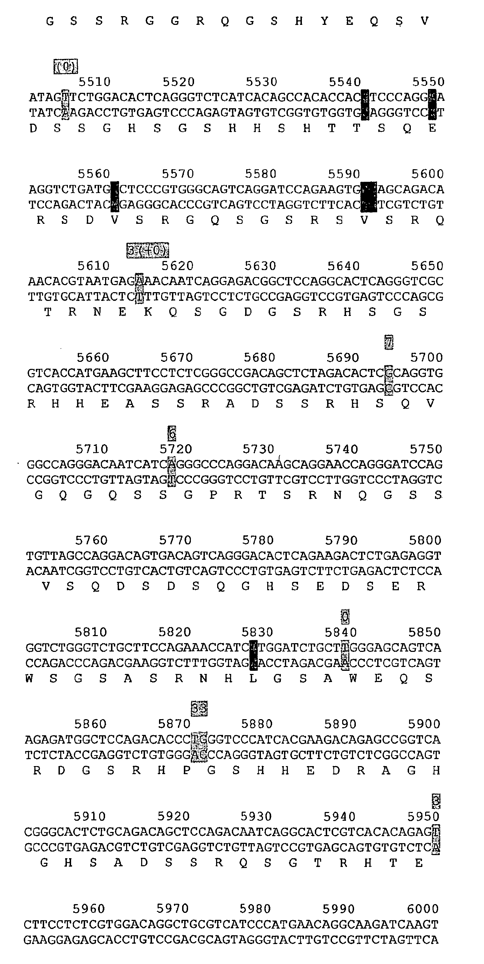

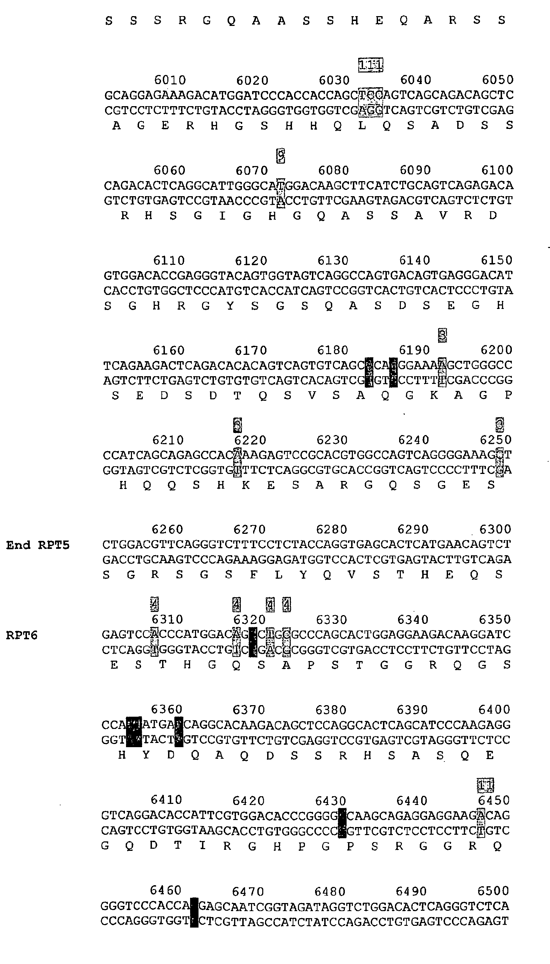

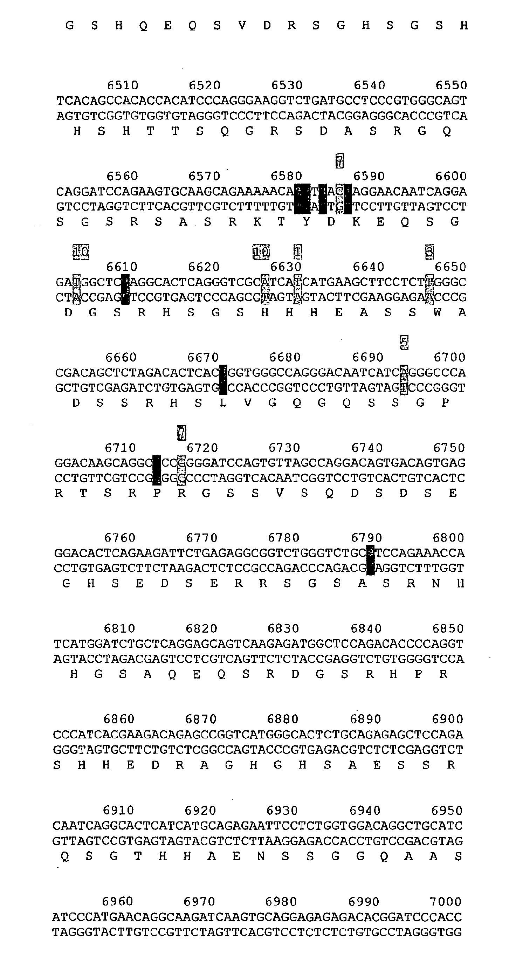

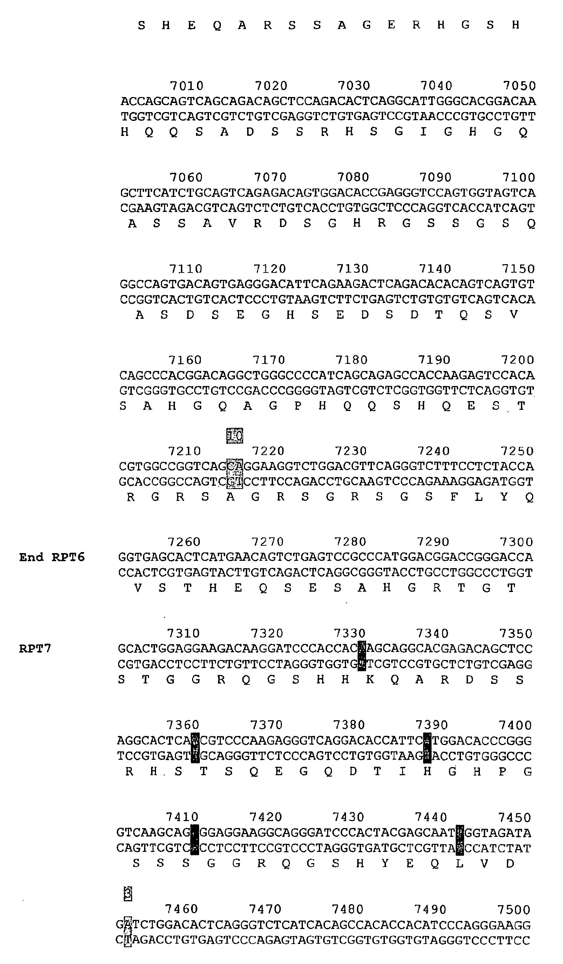

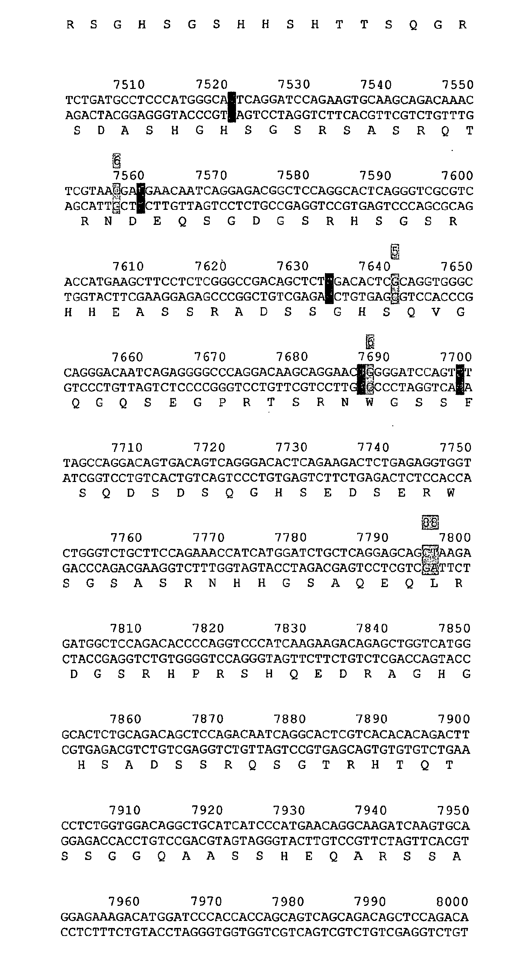

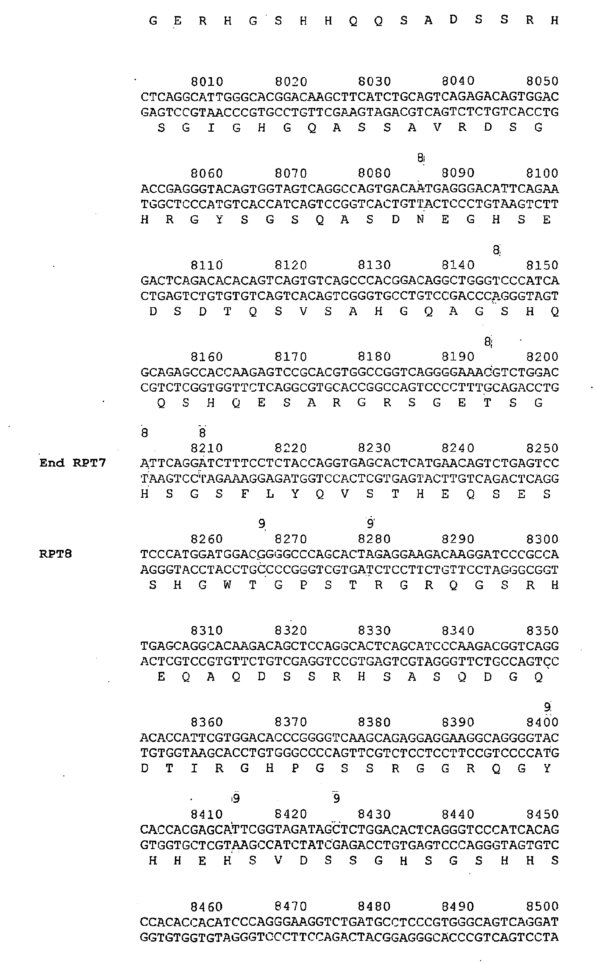

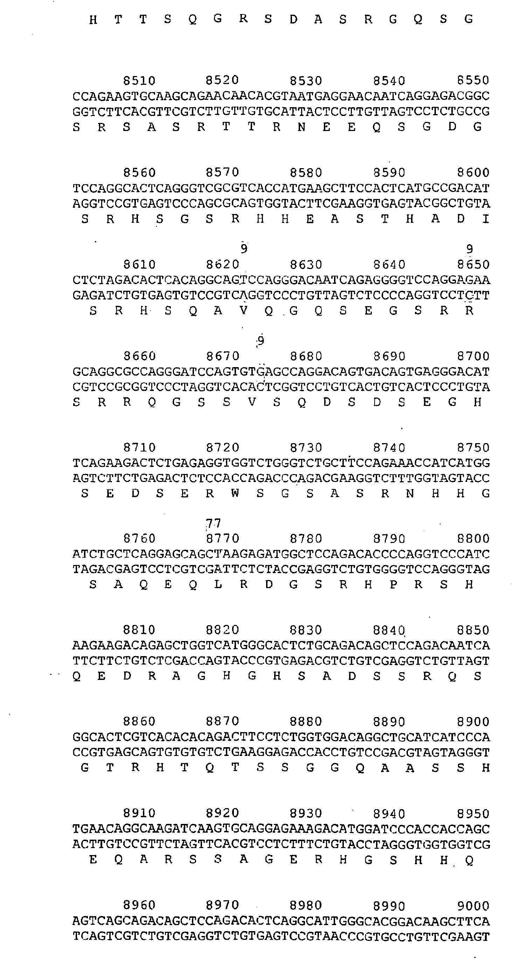

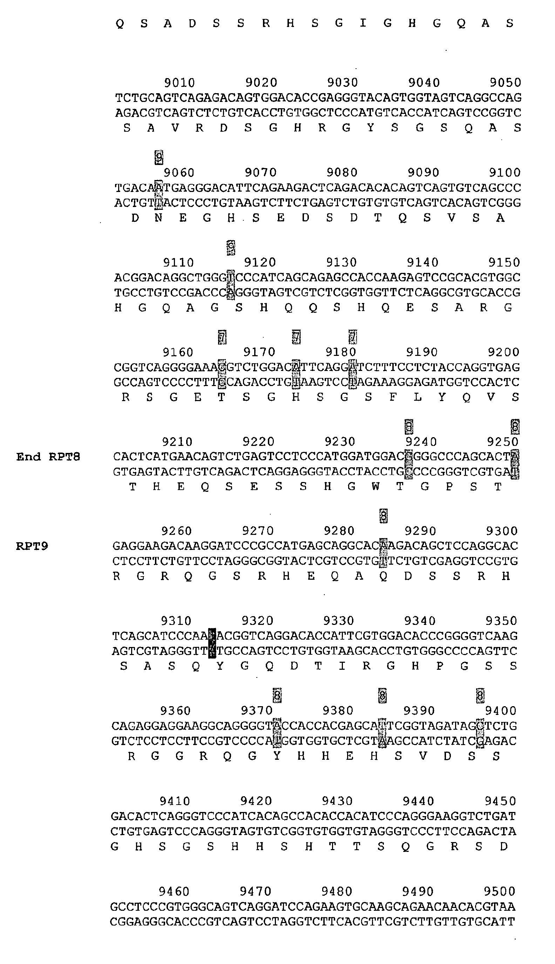

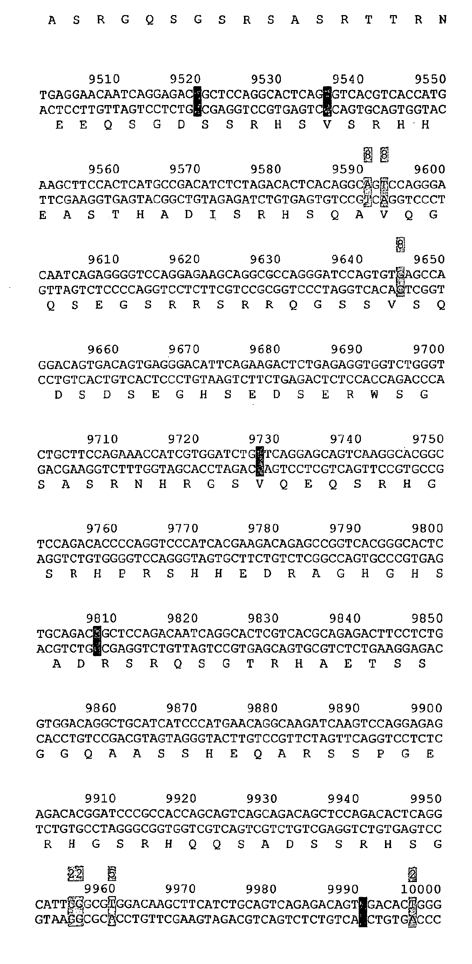

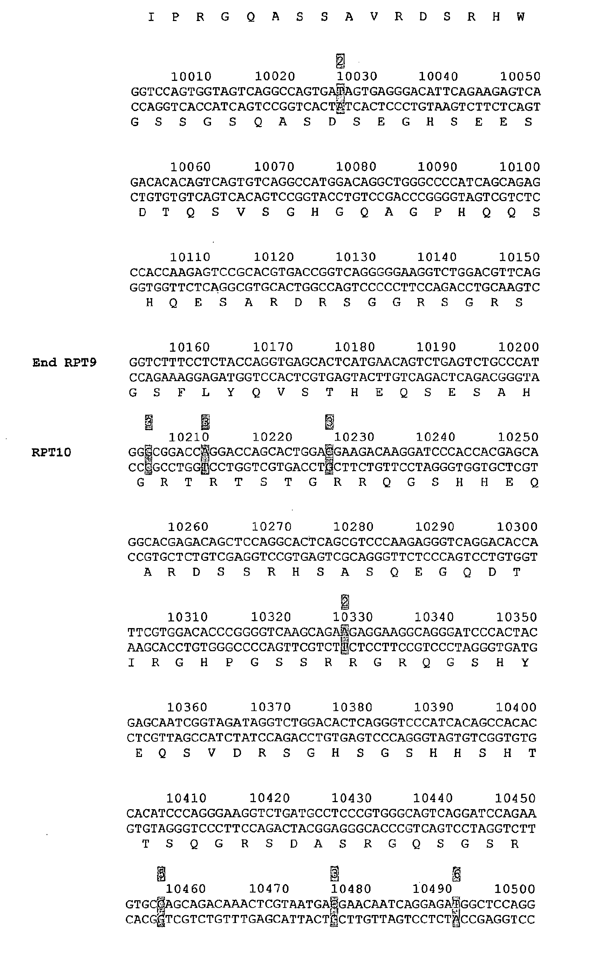

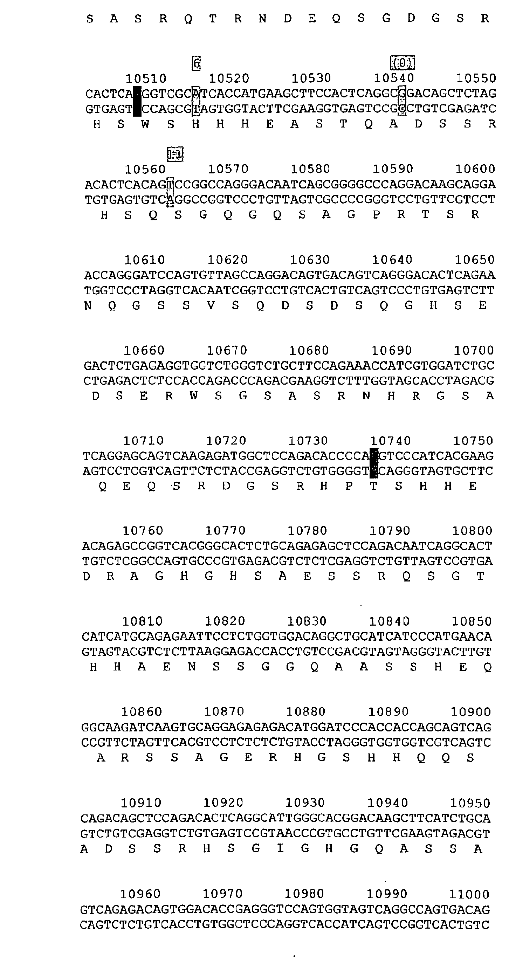

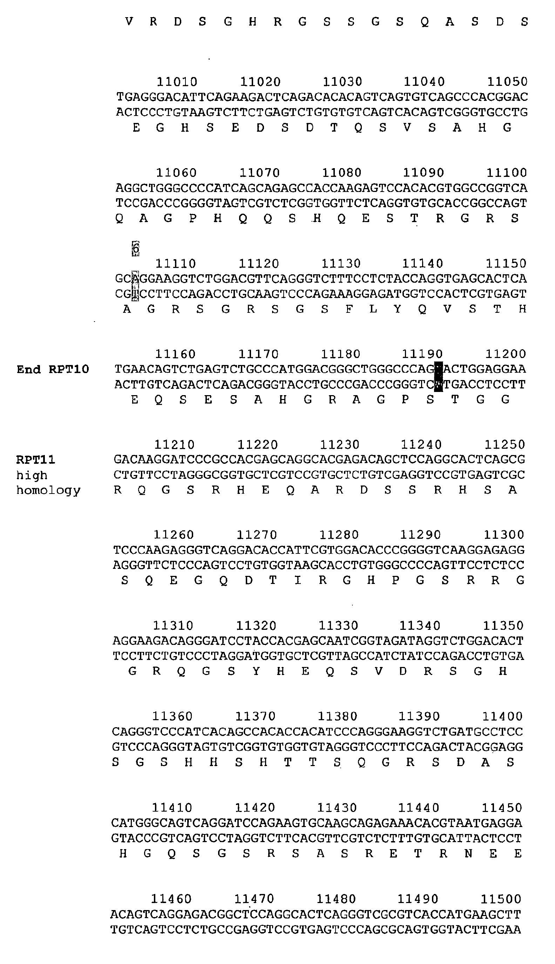

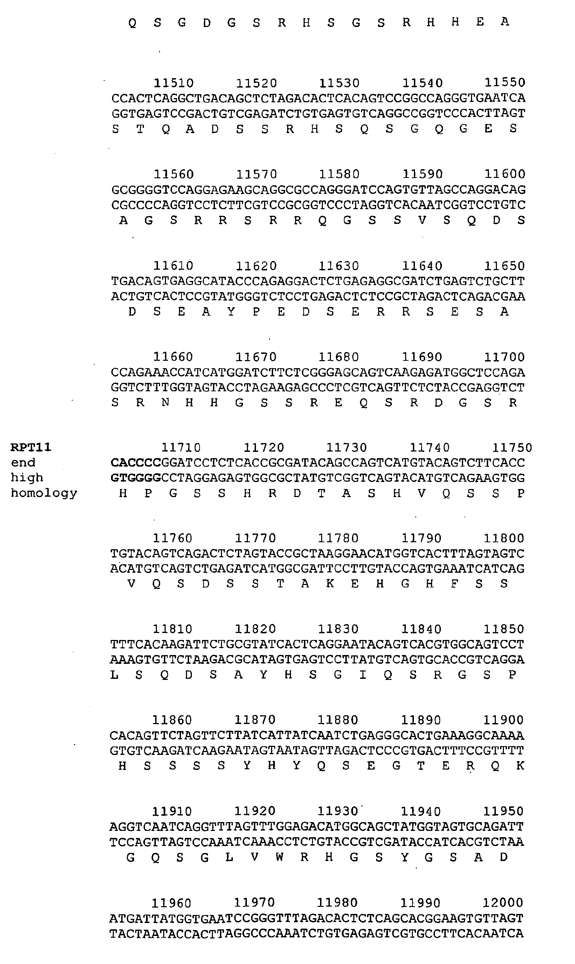

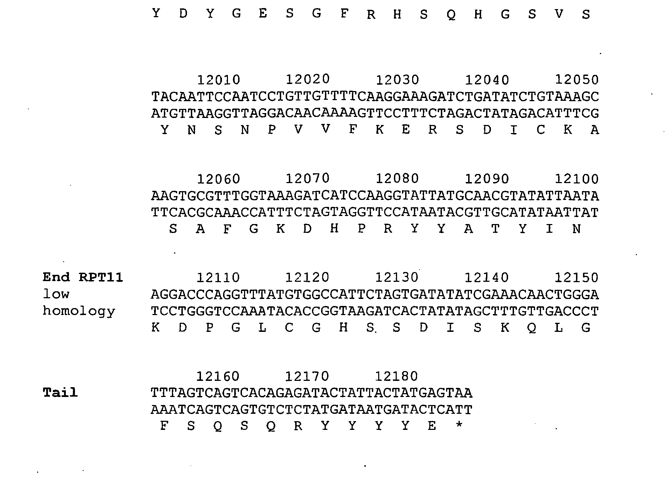

A second mutation identified is a 4-base deletion starting at position 2282 (see Figure 5 and SEQ ID NO.: 186). The mutation has been named 2282del4 and this causes a resulting frame-shift which leads to an alternative stop codon 107 bases downstream. Again, this mutation occurs in the first filaggrin repeat and as such no functional copy of a filaggrin peptide is expressed, although a truncated mutant form of the peptide may be expressed, which possesses a unique C-terminal portion (see Figure 4 and SEQ ID NO.: 187).

A third mutation is a deletion of a G in the third filaggrin repeat. The deletion is at position 3702 and is shown in Figure 5. This mutation causes a frameshift in repeat 3, such that only 2 functional copies of filaggrin from repeats 1 and 2 are made.

Further mutations which have been identified include R2477X (repeat 7), S3247X (repeat 9), R1474X (repeat 4), Q1745X (repeat 4), Q3683X (repeat 10), 11029delCA (repeat 10), E2422X (repeat 6), 5369delG (repeat 5), 7367delCA (repeat 7), 11033de14 (repeat 100, 6867delAG (repeat 6), 3321delA (repeat 2) and S2554X (repeat 7). The most prevalent and/or recurrent mutations in the European population are R510X, 2282de14, 3702delG, R2447X and S3247X.

The nomenclature used above is to be understood as follows: S3247X, for example means that there is a mutation found at codon position 3247 which results in a codon change from a codon which encodes a serine, to a stop codon. 5360delG is a deletion of a G at DNA base-pair position 5360 (numbering where the initiating ATG =1), leading to a frameshift.

Detection of a mutation in the *FLG* gene may be carried out by a variety of techniques including quantitative or semi-quantitative PCR, including real-time PCR, nucleic acid sequencing, hybridisation studies and/or restriction fragment length polymorphism (RFLP) analysis techniques, well known to the skilled addressee (see, for example, Sambrook & Russel, 2000).

Depending on where the mutation or mutations are located, it may be appropriate to amplify one or more exons or portions thereof. If the mutation(s) is/are located in exon 3, all or only a portion of exon 3 may be amplified using appropriate primers. If the mutation(s) is/are located in the first repeat, it may only be necessary to amplify the first repeat, or portion thereof comprising the mutation(s). By appropriate use of primers and optional labels, it can be possible to amplify a product and ascertain whether or not the product comprises a mutation. For example, primers may be designed which incorporate at (or very close to) the 3' terminal, a base capable of binding to the native or mutant base/sequence, such that only the native or mutant sequence will be amplified and detected. A selection of primers suitable for use in amplifying the entire exon 3, or certain specific regions of the repeated sequences of exon 3 are identified herein as SEQ ID NO.s: 1 -182.

SEQ ID NO.s 1 - 8 represent primers suitable for long range PCR and sequencing of the filaggrin repeats.

SEQ ID NO.s 9 - 12 represent primers suitable for generating short PCR fragments for detection of the R501X mutation.

SEQ ID NO.s 13 - 15 represent primers suitable for generating short PCR fragments for detection of mutation 2828de14.

SEQ ID NO.s 15 - 18 represent primers suitable for generating short PCR fragments for detection of mutation 3702delG.

SEQ ID NO.s 19 - 40 represent primers which are specific for repeat 0.

SEQ ID NO.s 41- 67 represent primers which are specific for repeat 1.

SEQ ID NO.s 68 - 93 represent primers which are specific for repeat 2.

SEQ ID NO.s 94 -136 represent primers which are specific for repeat 3.

SEQ ID NO.s 137 - 201 represent primers which are specific for repeat 4.

SEQ ID NO.s 202 - 264 represent primers which are specific for repeat 5.

SEQ ID NO.s 265 - 329 represent primers which are specific for repeat 6.

SEQ ID NO.s 330 - 377 represent primers which are specific for repeat 7.

SEQ ID NO.s 378 - 414 represent primers which are specific for repeat 8.

SEQ ID NO.s 415 - 461 represent primers which are specific for repeat 9.

SEQ ID NO.s 462 - 493 represent primers which are specific for repeat 10.

SEQ ID NO.s 494 - 497 represent primers which are specific for repeat 8.1.

SEQ ID NO.s 498 - 501 represent primers which are specific for repeat 8.2.

SEQ ID NO.s 502 - 518 represent primers which are specific for repeat 10.1.

SEQ ID NO.s 519 - 539 represent primers which are specific for repeat 10.2.

SEQ ID NO.s 540 - 544 represent primers which are specific for repeat 11.

SEQ ID NO. 545 represents a primer which is specific for the filaggrin tail.

In all cases, F at the end of a primer sequence shows that the primer is a forward primer and an R shows it is a reverse primer.

It will be appreciated that shorter or longer versions of the identified primer sequences may be used, for example, the primers may be from 12 - 50 bases in length. However, 3'-terminal base is critical for correct primer extension and so the 3'-end of any primer should be identical to the sequences as identified herein.

Labels, such as fluorescent, chemiluminescent, bioluminescent or radio-labels may be incorporated into the PCR primers so as to allow detection of the native or mutant sequence. The skilled man will appreciate that two separately labelled primers may be used in a PCR reaction, designed to facilitate amplification of a product comprising either the native or mutant sequence and the sequence, native or mutant, detected based on the particular label being present in the product. Other labelling techniques such as the TaqMan^{®} system of Applied Biosystems Inc., CA, USA, may be employed.

Of course, a fragment of DNA, which includes the portion of DNA which may include the mutation, may simply be amplified and sequenced, in order to determine whether or not the *FLG* gene comprises a mutation. Alternatively, such a fragment may be amplified and a hybridisation study carried out using an appropriate oligonucleotide and very stringent hybridisation conditions and washing conditions employed (see for example Sambrook et al, 2000¹⁵) so that only exactly matching oligonucleotides bind to the amplified fragment in the region or regions comprising the mutation(s).

It may also be appropriate to first amplify a fragment of DNA comprising the sequence which may or may not comprise a mutation(s) and thereafter detecting whether or not the fragment includes the native or mutant sequence by carrying out a further PCR reaction using primers internal to the amplified fragment, in order to detect or otherwise, a mutation(s). Such a technique is commonly known as nested PCR.

Moreover, any particular mutation may generate a new restriction site which may be detected by RFLP analysis. A fragment which would encompass a mutation which, if present, can first be amplified using appropriate primers and the fragment thereafter subjected to RFLP analysis providing the mutation or native sequence has a restriction site which is not present in the corresponding native or mutant sequence. In accordance with the present invention, the exemplary mutations identified herein result in the generation of new restriction sites which can easily be detected by first amplifying a fragment comprising the mutation and thereafter restricting the fragment obtained using the appropriate restriction enzyme - only a fragment comprising the mutant sequence will be restricted (see Examples Section fur further description).

Kits may comprise one or more of the aforementioned oligonucleotides/primers. Such kits may also comprise other reagents to facilitate, for example, sequencing, conducting PCR and/or RFLP analysis. Such kits may also comprise instructions for their use to detect one or more mutations in a filaggrin gene and optionally how to interpret whether or not a mutation may lead to development or predisposition to developing IV and/or any of the other aforementioned diseases/conditions.

The oligonucleotides/primers disclosed herein may also be used in multiplex PCR techniques, known to the skilled addressee, see for example. Kuperstein G, Jack E and Narod SA; Genet Test. 10(1):1-7 (2006)., so as to identify mutations in the filaggrin sequence.

In addition to mutations which lead to a loss of function or partial loss of function of profilaggrin/filaggrin protein, the present inventors have now identified the specific repeat sequences which can lead to exon 3 of the filaggrin gene consisting of 11 or 12 full filaggrin repeats, as opposed to the "normal" 10 repeats. The inventors have identified that repeats 8 and/or 10 can be essentially duplicated in certain individuals, in order to generate 11 or 12 filaggrin repeats. Desirably therefore, the present invention also extends to identifying the number of filaggrin repeats in a subject as well as detecting one or more mutations. Heterozygous mutant subjects who possess one mutant allele which results in no or little filaggrin expression, but have a second wild-type allele encoding 11 or 12, filaggrin repeats, may express sufficient filaggrin to not develop disease, or only a mild form of disease. For example, a carrier of a 12-repeat allele, will express 20% more filaggrin than a 10-repeat carrier and this difference in expression may be significant in terms of disease development.

It has been observed that the aforementioned 4-base deletion (2282de14) results in the expression of a unique peptide which comprises an N-terminal region corresponding to the N-terminal portion of the filaggrin peptide and a unique C-terminal region which has been expressed due to the frame-shift mutation. With respect to this unique peptide which is produced from the mutant sequence comprising the 4-base deletion, it is possible to detect the unique peptide using an appropriate binding agent, such as a specific antibody. It is appreciated that any such binding agent/antibody should be specific for the unique peptide and not therefore be capable of binding the native filaggrin peptide.

The skilled man will readily know how to obtain a suitable antibody, such as a monoclonal antibody, by, for example, producing the unique peptide recombinantly or using synthetic chemistry, coded for by the mutant sequence and raising antibodies thereto. Antibodies so produced can thereafter be screened to ascertain their specificity, such that only those antibodies which are specific for the unique peptide may be selected.

Such specifically reactive antibodies to the unique peptide can be optionally labelled and used in an immunoassay to detect for the presence of the unique peptide in a sample. Alternatively, the specifically reactive antibody can be used in an assay, such as an ELISA, to detect any of said unique peptide in a sample being tested.

Thus, in a further aspect there is provided a method of detecting a mutant peptide generated as a result of the 2284de14 mutation in the filaggrin gene, comprising the steps of:
detecting *in vitro* whether or not a mutant peptide expressed from a mutant *FLG* gene is present in the sample, by using a binding agent which is specifically reactive to said mutant peptide generated as a result of the 2284del4 mutation wherein the binding agent is not capable of binding the native filaggrin peptide.

In this aspect, the sample may preferably be a skin tissue sample. Typically, the binding agent is an antibody, monoclonal antibody or fragment thereof, such as a Fab fragment. Detection may be carried out by detecting a label, such as a fluorescent, chemiluminescent, bio-luminescent or radio-label coupled to the binding agent/antibody/fragment. Alternatively, the binding agent, antibody or antibody fragment may be unlabelled and detected by way of an antibody specific for said binding agent, antibody or antibody fragment, such as in an ELISA assay.

It will be understood that the nucleic acid and mutant peptide tests described herein may be conducted individually or together.

Identification of mutants in the *FLG* gene leading to loss or partial loss of function of the FLG protein opens up the possibility of treating prophylactically or therapeutically IV and/or any of the other aforementioned diseases by gene therapy. As such a correct non-mutant copy or copies of the *FLG* gene may be used to complement for a mutant version of the *FLG* gene present in a subject.

An *FLG* gene sequence or fragment thereof, capable of encoding one or more copies of the *FLG* protein may be used in the manufacture of a medicament. It is understood that the medicament may be used for the prophylactic or therapeutic treatment of IV and/or diseases including atopic dermatitis (eczema), asthma, psoriasis and allergies.

A *FLG* gene sequence or fragment thereof, which gene sequence or fragment thereof, is capable of expressing one or more copies of the filaggrin protein, may find application in therapy or prophylaxis.

Methods of treating prophylactically or therapeutically any of the aforementioned diseases/conditions may comprise administering to a patient suffering or predisposed to developing any of said aforementioned diseases a DNA construct comprising an *FLG* gene sequence or fragment thereof, which gene sequence or fragment thereof is capable of expressing one or more copies of the *FLG* protein, whereby expression of said one or more copies of the FLG protein treats or ameliorates said disease(s)/condition(s).

Typically, the *FLG* sequence or fragment thereof may be administered to a subject in the form of a recombinant molecule comprising said *FLG* sequence or fragment under appropriate transcriptional/translational controls to allow expression of said filaggrin protein when administered to a subject. It will be appreciated that the *FLG* sequence or fragment may be under control of a suitable promoter, such as a constitutive and/or controllable promoter. Convenient promoters include the native filaggrin promoter, or an appropriate late differentiation-specific keratin promoter.

A recombinant molecule comprising an *FLG* sequence or fragment thereof may also find application in therapy. The recombinant molecule may be in the form of a plasmid, phagemid or viral vector. Furthermore, recombinantly expressed, or chemically synthesised filaggrin or profilaggrin protein, or functionally important fragments thereof, may be produced and applied to the skin via a suitable ointment or other pharmaceutical vehicle, as a treatment or prophylatic measure for ichthyosis vulgaris and/or atopic diseases. Such a treatment may also be of cosmetic value as it may increase the barrier function and/or moisture-retention properties of the skin. Since filaggrin is prominently expressed in hair from early in development¹⁶, such a treatment may also improve cosmetic qualities of the hair, such as moisture retention, in individuals with either normal or reduced filaggrin expression.

Many different viral and non-viral vectors and methods of their delivery, for use in gene therapy, are known, such as adenovirus vectors, adeno-associated virus vectors, retrovirus vectors, lentiviral vectors, herpes virus vectors, liposomes, DNA vaccination and the like¹⁷.

A method of treating, preventing and/or ameliorating IV and/or any of the aforementioned diseases may comprise the steps of:
a) determining if expression of one or more copies of the FLG polypeptide would occur even if a subject's FLG gene comprises one or more mutations; and
b) providing a subject carries FLG alleles capable of expressing at least one or more copies of the FLG polypeptide, treating the subject using UV light in order to seek to increase *FLG* expression.

The intention is to increase expression of FLG polypeptides in the skin. This may most easily be achieved in heterozygote subjects, i.e. those subjects who have one mutant copy of the *FLG* gene and one native copy.

The expression of the *FLG* gene is known to be induced by UV light¹⁸. Thus, U V treatment of the skin of an individual carrying a heterozygous copy of a filaggrin loss-of-function mutation could increase the expression of the normal allele and thereby produce a beneficial effect.

The present invention also relates to a transgenic non-human animal which possesses one or more mutations in one or both copies of the *FLG* gene which would lead to a loss or partial loss of function of the *FLG* protein encoded by the *FLG* gene. Desirably, the *FLG* gene of the non-human animal may be replaced with a mutant human form of the gene, in order to "humanise" the non-human animal with respect to the *FLG* gene.

The transgenic animals of the present invention can be used for the development of various treatments for IV and/or the other diseases mentioned herein including the identification of various therapeutically active agents including but not limited to other proteins, peptides, peptidomimetic drugs, small molecule drugs, chemicals and nucleic acid-based agents.

The term non-human animals is used herein to include all vertebrate animals, except humans. It also includes an individual animal in all stages of development, including embryonic and foetal stages. A transgenic animal is any animal containing one or more cells bearing genetic information altered or received, directly or indirectly, by deliberate genetic manipulation at a subcellular level, such as by targeted recombination or microinjection or infection with recombinant virus. The term transgenic animal is not intended to encompass classical cross-breading or *in vitro* fertilization, but rather is meant to encompass animals in which one or more cells are altered by, or receive, a recombinant DNA molecule.

To create a transgenic non-human animal expressing a mutant form of the *FLG* gene, mutant *FLG* nucleic acid sequences are inserted into a germ line of the animal using standard techniques of oocyte microinjection or transfection or microinjection into stem cells. Preferably, it is desired to replace the endogenous gene and homologous recombination using embryonic stem cells or foetal fibroblasts can be applied.

Mice are often used for transgenic animal models because they are easy to house, relatively inexpensive, and easy to breed. However, other non-human transgenic mammals can also be made in accordance with the present invention such as but not limited to monkeys, sheep, rabbits, dogs and rats. Transgenic animals are those which carry a transgene, that is, a cloned gene introduced and stably incorporated which is passed on to successive generations.

For oocyte injection, for example in mice, one or more copies of the nucleic acid sequences encoding *FLG* can be inserted into the pronucleus of a just-fertilised mouse oocyte. This oocyte is then reimplanted into a pseudo-pregnant foster mother. The live born mice can then be screened for integrants using analysis of, for example, tail DNA for the presence of the mutant *FLG* sequences.

Methods of making transgenic mammals are known and described, e.g. in Wall, et al. (1992) J. Cell Biochem. 49(2): 113-20¹⁹; McCreath, et al. (2000) Nature 405: 1066-1069²⁰; Lai, et al. (2002) Science 295: 1089-92²¹; Hogan, et al. (1986) In: Manipulating the mouse embryo. A Laboratory Manual. Cold Spring Harbour Laboratory Press, Cold Spring Harbor, N.Y.²²; in WO 91/08216 or U.S. Pat. No. 4,736,866. The mice disclosed herein can be crossed with a hairless or nude mouse background so that skin abnormalities are visible to facilitate monitoring of disease progression and/or potential therapies.

An *in vivo* assay for identifying an agent which is useful for treating or preventing IV and/or any of the other aforementioned diseases associated with mutant *FLG* expression comprising the steps of administering a test agent to a *FLG* mutant transgenic animal; and measuring or determining whether the agent decreases or inhibits at least one sign or symptom of IV and/or any of the other aforementioned diseases which is indicative that the test agent is capable of treating or preventing IV and/or any of the other aforementioned diseases. The results of the screening assay can be compared with a control, e.g., an animal which has not been administered a test agent or an animal which has received an agent known to reduce a sign or symptom of said diseases. The route of administration of the test agent may vary. Examples of administration routes include, but are not limited, to oral, nasal, rectal, transmucosal, intestinal, parenteral, intravenous, intraperitoneal and topical.

### Examples Section

The present invention will now be further described by way of example and with reference to the Figures which show:

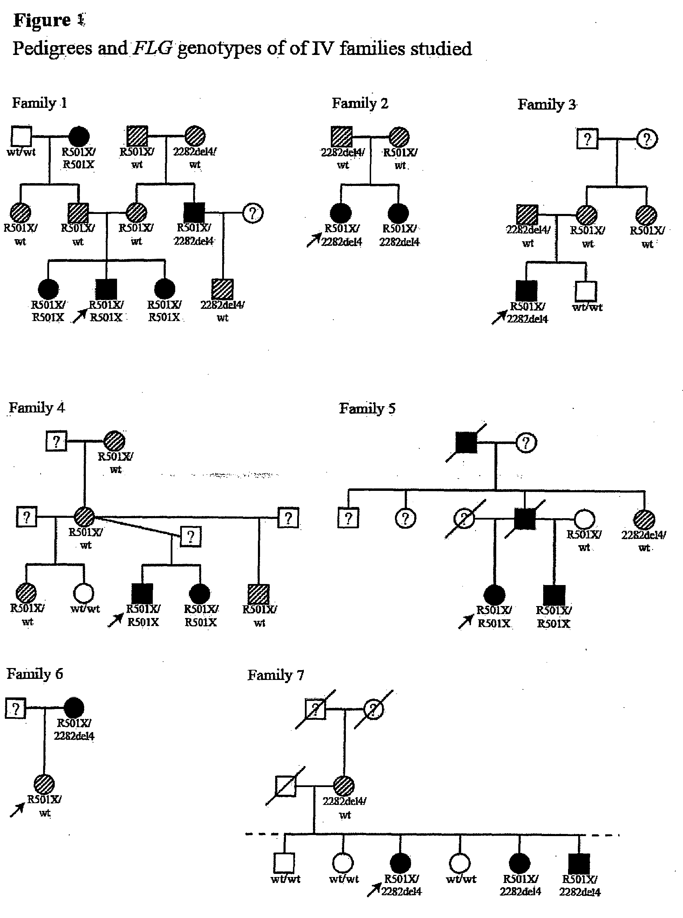

**Figure 1** **shows Pedigrees of IV families studied.**
   Pedigrees of IV families studied here where a family history was available. Families 1-3 are of Irish origin, Families 4-6 are Scottish and Family 7 is of US origin. In addition, 8 isolated IV cases were studied where a family history was not available (not shown). Of the latter, one case was Irish, 4 were Scottish and 3 were of US origin. All patients studied were White Caucasians. Black-filled symbols refer to the marked IV presentation; cross-hatched symbols refer to the very mild IV presentation; open symbols refer to no detectable IV phenotype. The genotypes for the two mutations R501X and 2282del4 are shown. Note that wt/wt refers only to the regions screened and does not preclude other sequence changes in the central regions of exon 3. Only two people with no detectable IV phenotype were found to have a filaggrin mutation: individual II-5 in Family 5, who carries R501X; and individual II-1 in Family 7, who is an obligate carrier of R501X. On this basis, we estimate the penetrance in heterozygotes as ∼90%, although this is probably an overestimate due to ascertainment bias. Many individuals in these families, who carry one or other filaggrin mutation, either heterozygously or homozygously, have, in addition to ichthyosis vulgaris, atopic dermatitis (eczema) and/or asthma and/or allergies (see also Figure 4). Thus, filaggrin mutations predispose individuals to these other conditions.
**Figure 2** **shows FLG mutation detection and confirmation.**
   (a) Schematic diagram of the filaggrin gene (*FLG*), annotated to show the corresponding protein structure. Exon 1 consists of a short 5'UTR sequence. Exon 2 and the 5' end of exon 3 encode the profilaggrin N-terminal domain. The remainder of exon 3 consists of 10-12 repeats of approximately 1 kb, each encoding a filaggrin peptide separated by linker sequences, followed by a short unique coding sequence and the 3'UTR. Upon terminal differentiation of the epidermis, each profilaggrin molecule is proteolytically cleaved to release 10 - 12 copies of filaggrin, which aggregate the keratin cytoskeleton and cause physical collapse of the granular cells to form squamous cells. The positions of PCR fragments used here and of the two null-mutations, R501X and 2282del4 in repeat 1 of exon 3, are shown.
   (b) Long-range PCR product from genomic DNA covering exon 3 and therefore all the filaggrin repeats.
   (c) Normal sequence from filaggrin repeat 1 in exon 3, corresponding to codons 499-503.
   (d) The same region of the *FLG* as seen in (c), showing heterozygous transition mutation 1502C>T resulting in nonsense mutation R501X.
   (e) The same region of *FLG* as in (c) showing a homozygous mutation resulting in a nonsense codon, R501X.
   (f) Confirmation of mutation R501X by *Nla* III restriction digest and 2282del4 by *Dra* III restriction digest from some members of Family 3.
   (g) Normal sequence from filaggrin repeat 1 in exon 3, corresponding to codons 713-717.
   (h) The same region of *FLG* as in (f), showing overlapping peaks due to a heterozygous deletion mutation, 2282de14.
   (i) The same region of the *FLG* as in (f), derived from a mutant clone confirming mutation 2282del4. This mutation leads to a premature stop codon 107 bp downstream and terminates translation within filaggrin repeat 1.

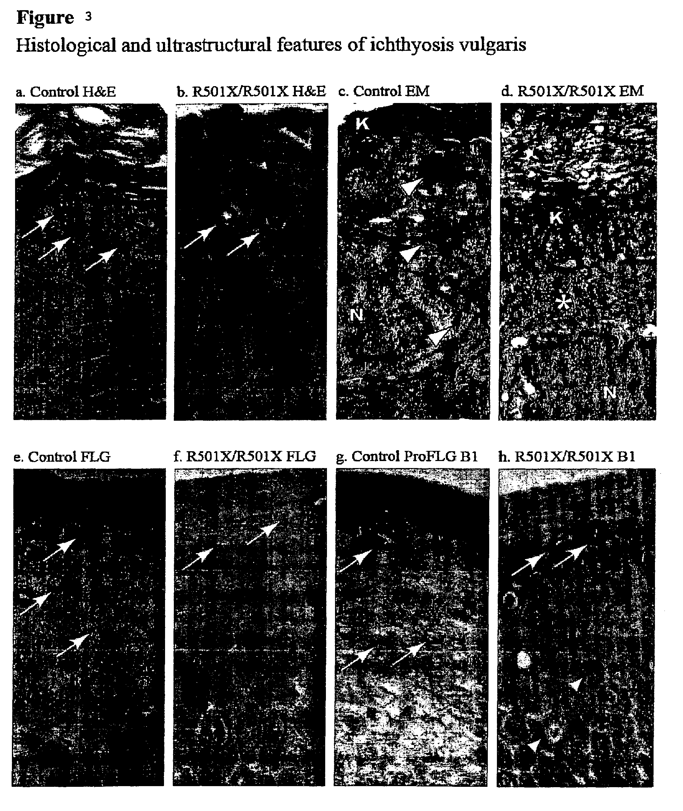

**Figure 3** **shows morphological features of filaggrin-null ichthyosis vulgaris.**
   (a) Skin biopsy from a normal (non-ichthyotic) control. Haematoxylin and eosin staining of formaldehyde-fixed paraffin-embedded tissue shows prominent keratohyalin granules in the granular cell layers of the superficial epidermis (arrows).
   (b) Skin biopsy from the proband in Family 4, who is homozygous for nonsense mutation R501X in the *FLG* gene. In contrast with the normal control seen in (a), there is a complete absence of keratohyalin granules in the upper layers of the epidermis. The degenerating nuclei seen in the uppermost living layers (arrows), indicate that this is the area where one would normally see keratohyalin granules.
   (c) Transmission electron micrograph of keratinocytes at the boundary of the granular layer and stratum corneum, from a normal individual, showing prominent keratohyalin granules (arrowheads). N = nucleus; K = keratinized material in stratum corneum. Original magnification = ∼5,600x.
   (d) Transmission electron micrograph of granular layer cells from the proband in Family 4, who is homozygous for nonsense mutation R501X in the *FLG* gene. There is a complete absence of keratohyalin granules (*). The stratum corneum is not fully cornified, as compared with the control (K), indicative of an epidermal barrier defect. N = nucleus. Original magnification = ∼5,600x.
   (e) Immunohistochemical staining of formaldehyde-fixed paraffin-embedded tissue using anti-filaggrin repeat monoclonal antibody 15C10 (Novocastra), visualized by the immunoperoxidase method. In skin biopsy material from a normal control, keratohyalin granules are strongly stained in the upper suprabasal layers of the epidermis (arrows).
   (f) Immunoperoxidase staining of skin biopsy material from the proband in Family 4, shows complete absence of staining in the upper suprabasal layers (arrows) with anti-filaggrin repeat monoclonal antibody 15C10 (Novocastra). This demonstrates that no filaggrin peptides are produced in patients homozygous for R501X, consistent with a nonsense mutation within the first filaggrin repeat.
   (g) Immunoperoxidase staining of skin biopsy material from a normal control individual with polyclonal antibody B1, raised against an epitope within the N-terminal domain of profilaggrin, showing prominent staining of keratohyalin granules (arrows).
   (h) Immunoperoxidase staining of skin biopsy material from the proband in Family 4, with profilaggrin N-terminal antibody B1. No granular staining is seen but unlike the filaggrin repeat epitope (f), there is a diffuse pattern of residual staining. This is more pronounced in the upper suprabasal cells where profilaggrin is normally expressed (arrows) but in addition there is some patchy diffuse cytoplasmic staining throughout the epidermis (arrowheads). The epitope of this antibody is upstream of the mutation and so this shows that a truncated fragment of profilaggrin is synthesized in R501X homozygotes.
Figure 4 shows complete sequence of the filaggrin repeats and identifies the positions of the mutations identified by the inventors and positions of where the specific primers identified herein bind. These specific priming sites were identified by analysis of alignments of the individual filaggrin repeats, including the novel additional repeats identified by the inventors.
Figure 5 shows the pedigrees of a family with IV and corresponding atopy transmission;
Figure 6 is a graph showing that filaggrin variants are associated with increased atopy;
Figure 7 is a graph showing increased number of positive allergens in carriers of filaggrin mutations;
Figure 8 is a graph showing filaggrin variants are stronger risk factors for eczema in the older asthmatics;
Figure 9 shows a) immunostaining of skin biopsy material from a normal control and an IV patient with the R5110X/R2447X genotype. b) immunoblotting of skin biopsy protein extracts from a normal control and IV patients with the genotypes R501X/R2447X and R501X/R501X;
Figure 10 is a schematic diagram of profilaggrin proteins encoded by size variant alleles *of FLG;*
Figure 11 shows the DNA sequence of *FLG* size variant allele *FLG⁸⁺;*
Figure 12 shows the DNA sequence of *FLG* size variant allele *FLG¹⁰⁺;* and
Figure 13 shows the sequence of a fragment from *FLG* size variant allele *FLG⁸⁺¹⁰⁺,* together with annotations as follows:
   Annotated Sequence - repeats in alternate plan and bold text
   * = unique base pair specific to this filaggrin repeat sequence
   N = base pair shared with only one other filaggrin repeat sequence, number N
   N* = base pair essentially specific due to nearby differences in repeat N.

### METHODS

### Affected individuals and phenotypes

Blood samples were obtained from 15 families with IV and normal ethnically matched controls with informed consent that complies with all principles of the Helsinki Accord.

### Long-range PCR for FLG exon 3

Primers FiILR2F (+ strand) 5' GTC ACT TAC CCC ATC AAA TC 3' and FiILR1R (- strand) 5' CCA CCA AAC TAA TGA AAT AC 3' were used to amplify approximately 12 kb of the filaggrin gene (including all of exon 3 and therefore all the repeat domains) from genomic DNA using the Expand Long Template PCR System (Roche Diagnostics, East Sussex, UK). A "hot start" was performed with 1U Expand Long Template enzyme mix (Roche). Reactions were amplified using the following extended PCR program: (92°C 5 min x1); (92°C 10 sec, 49°C 30 sec, 68°C 6 min) x10; (92°C 10 sec, 49°C 30 sec, 68°C 6 min plus 10 sec increment/cycle) x28; and (68°C 10 min) x1.

### R501X mutation analysis

A shorter PCR fragment was designed to amplify approximately 1.5 kb for mutation analysis of R501X. Primers FilF3 (+ strand) 5' GCT GAT AAT GTG ATT CTG TCT G 3' and RPT1P6 (- strand) 5' ACC TGA GTG TCC AGA CCT ATT 3' were used in High Fidelity PCR buffer (Roche) containing 1.5 mM MgCl₂, 4% DMSO and 1U High Fidelity thermostable DNA polymerase mix (Roche). Reactions were amplified under the following conditions: (94°C 5 min x1); (94°C 30 sec, 57°C 1 min, 72°C 2 min) x30; and (72°C 5 min) x1. Mutation R501X creates a new *Nla* III restriction enzyme site; this was used to confirm the mutation and screen control samples. Primers Fi1H1F3 (+ strand) 5' CAC GGA AAG GCT GGG CTG A 3' and RPT1P6 (above) were used to amplify 312 bp of genomic DNA using PCR buffer (Promega) containing 1.5 mM MgCl₂, 4% DMSO and 1U *Taq* polymerase mix (Promega). Reactions were amplified as follows: (94°C 5 min x1); (94°C 30 sec, 58°C 45 sec, 72°C 1 min) x30; and (72°C 5 min) xl. PCR products were digested with 5U *Nla* III for 4 hr at 37°C. Digests were resolved on 3% agarose gels.

### 2282del4 mutation analysis

A PCR fragment amplifying 811 bp of genomic DNA was amplified with primers RPT1P7 (+ strand) 5' AAT AGG TCT GGA CAC TCA GGT 3' and RPT2P1 (- strand) 5' GGG AGG ACT CAG ACT GTT T 3' using PCR buffer (Applied Biosystems) containing 1.5 mM MgCl₂, 4% DMSO and 1U *Taq* polymerase mix (Promega). PCR amplification conditions were: (94°C 5 min x1); (94°C 30 sec, 57°C 45 sec, 72°C 1 min 30 sec) x35; and (72°C 5 min) x1. Mutation 2282del4 creates a new *Dra* III restriction enzyme site which was used to screen samples for this mutation. PCR products were digested with 5U *Dra* III for 4 hr at 37°C. Digests were resolved on 2% agarose gels. A PCR fragment from a heterozygous individual was cloned into vector pCR2.1 (Invitrogen). Clones were screened by *Dra* III digestion and sequenced to confirm the 4 bp deletion.

### Histology and electron microscopy

Routine hematoxylin and eosin (H&E) staining was performed to evaluate morphologic features of each specimen. Immunoperoxidase staining of frozen and paraffin-embedded sections utilized the Envision system (DakoCytomation, Denmark). Antibodies used were mouse monoclonal 15C10 against an epitope in the C-terminal portion of the human filaggrin repeat unit (Novocastra, Newcastle upon Tyne, UK) and rabbit polyclonal antiserum B1 raised against the N-terminus of profilaggrin²³. For transmission electron microscopy, skin samples from patients were fixed in half-strength Karnovsky's fixative (containing 2.5% glutaraldehyde and 2% formaldehyde) then in 1.3% osmium tetroxide and processed using standard methods, largely as described previously²⁴.

### Lod score calculations

Lod scores were calculated with MLINK algorithm of LINKAGE version 5.1, using a semidominant model of the disease where heterozygotes were assigned a mild phenotype with 90% penetrance and homozygotes or compound heterozygotes were assigned as a severe phenotype with 100% penetrance. The combined mutant allele frequency was assumed to be 0.037 (Table 1). Recalculation with 50% penetrance in heterozygotes still yielded a highly significant maximum combined lod score of 7.08 at 0=0.

*FLG* consists of three exons^{25,26}. Exon 1 (15 bp) consists only of 5'UTR sequences and exon 2 (159 bp) contains the initiation codon. Exon 3 is unusually large (12,753 bp) and codes for most of the N-terminal domain and all filaggrin repeats (Figure 2a). The number of filaggrin repeats varies from 10-12 in the population⁶. The homology between the repeats at the DNA level is almost 100%, making conventional PCR-based sequencing for the internal regions of this exon almost impossible. No sequence changes were found in exons 1 or 2 in five IV families. The present inventors developed long-range PCR conditions to amplify a 12 kb genomic fragment covering exon 3 and therefore all the repeat domains (Figure 2b). Full sequencing of this fragment is on-going, but initial sequencing has revealed a homozygous nonsense mutation R501X near the start of repeat 1 in three affected individuals from Family 1 (Figure 2c - e). Using a smaller PCR fragment, segregation of R501X was confirmed in Family 1 and in addition, this mutation was identified in the other 14 IV kindreds studied. The mutation creates a new *Nla* III restriction enzyme site; this was used to confirm the mutation and screen populations (Figure 2f). By this means, the mutation was found to be present at relatively high allele frequencies in Irish, Scottish and North American Caucasian populations (combined frequency, 0.027; see Table 1).

In 3 families, IV patients with a very pronounced phenotype were homozygous for R501X (Figure 1). In other families and isolated cases, individuals with the marked IV phenotype were found to be heterozygous for R501X. Further sequencing in these cases revealed a second mutation, 2282del4, in exon 3 (Figure 2g - i). This leads to a premature termination codon 107 bp downstream and, like R501X, stops protein translation within the first filaggrin repeat (Figure 2a). Mutation 2282del4 creates a *Dra* III restriction enzyme site which was used to screen IV families and control samples (Figure 2f; Table 1). This mutation segregated in 10 of the IV families studied (Figure 1). Of the 8 "sporadic" cases of clinically significant IV where family history was not available, 4 were homozygous for R501X and the remaining 4 were R501X/2282del4 compound heterozygotes. Interestingly, part of the US family previously reported to show significant linkage to the *FLG* locus²⁷, was studied using freshly obtained high-quality DNA required for analysis of exon 3. The severely affected individuals in Family 7 were compound heterozygous for R501X/2282del4 (Figure 1), consistent with the linkage data previously reported²⁷. The semidominant mode of inheritance is best exemplified in Family 1 where there are multiple examples of IV patients with the very mild presentation as well as examples of R501X homozygotes and R501X/2282del4 compound heterozygotes with the full IV phenotype. In the studied series of families there were only two individuals who were heterozygous for a null-mutation (both R501X) and have no obvious phenotype (Families 5 & 7; Figure 1). On the basis of these small numbers, the penetrance in heterozygotes appears to be about 90%, however, this may be an overestimate due to ascertainment bias. The allele frequency for 2282del4 in US, Irish and Scottish Caucasians was found to be ∼0.01 (Table 1). Using the determined allele frequencies and assuming mildly affected heterozygotes and severely affected homozygotes, the maximum combined 2-point lod score for families 1-7 (Figure 1), was 8.11 at θ=0.

Skin biopsy material from an R501X homozygote (proband, Family 4) was subjected to histological and ultrastructural analysis. The granular layer was found to be absent by conventional histology (Figure 3a & b) and electron microscopy showed complete absence of keratohyalin (Figure 3c & d). Immunohistochemistry showed that an epitope conserved in all filaggrin repeat peptides was completely absent in the R501X homozygote (Figure 3e & f). In contrast, an epitope in the N-terminal domain of profilaggrin, encoded by sequences upstream of filaggrin repeat 1, was still present, albeit in an abnormal, diffuse distribution (Figure 3g & h). Immunohistochemical analysis of an R501X/2282del4 compound heterozygote gave identical results (not shown). This confirms that either R501X or 2282del4 result in complete loss of filaggrin peptide production and so functionally, these are null-alleles.

Since profilaggrin is the major component of keratohyalin granules, this explains the absent granular layer associated with the more severe cases of IV²⁷ (Figure 3). The presence of a truncated profilaggrin peptide in IV epidermis (Figure 3b) is consistent with previous studies demonstrating that a peptide containing the unique N-terminal domain and a small amount of filaggrin sequence is stable *in vitro²³.* In normal epidermis, the N-terminal Ca²⁺-binding domain is cleaved from profilaggrin by a proprotein convertase, and subsequently localizes to different cell compartments including the nucleus^{28,29}. Similar processing of the truncated peptide may occur in IV epidermis.

Here the inventors have shown that in three Caucasian populations, IV appears to be predominantly caused by two frequent null-mutations in *FLG,* leading to loss of filaggrin production and impaired epidermal barrier formation. In the IV families studied, most R501X mutations are in linkage disequilibrium with the same 156 bp allele of a microsatellite in intron 2 of *FLG* (data not shown), suggesting that, in human evolutionary terms, these are ancient mutations. Further analysis of polymorphisms near *FLG* will determine the approximate age of the mutations. Genetic drift may explain why these mutations have become so prevalent. Alternatively, a heterozygote advantage might explain the high frequencies of these alleles. One obvious hypothesis is that impaired barrier function leads to elevated exposure to bacterial or other antigens, leading to greater innate immunity. This "natural cutaneous vaccination" might allow heterozygotes to better survive when challenged by pandemic plagues or other pathogens. This should be testable using *ft* mice¹² or engineered filaggrin null-mice.

Regarding the inheritance pattern and incidence of IV; the very subtle heterozygote phenotype probably does often not come to clinical attention unless specifically sought, as was the case here. Assuming a combined null-allele frequency of ∼0.037 (Table 1), and a pronounced heterozygote phenotype, then 1 in 14 people would have IV, which is clearly not the case. With this allele frequency, 1 in 730 should be homozygous or compound heterozygous and have marked IV. The subtlety of the heterozygote phenotype, combined with incomplete penetrance and seasonal variation², probably explains the reported incidence of 1 in 250¹. With these high mutant allele frequencies, IV families will also frequently appear to have dominant or pseudo-dominant inheritance with reduced penetrance (Figure 1). By Southern analysis, polymorphism in the number of filaggrin repeats has been shown in humans (10-12 repeats)⁶ and mice (12-20 repeats)³⁰. The inventors also observed this size variation using long-range PCR and determined the sequences of the longer variant alleles (described below). It is possible that a heterozygote for a null-mutation might carry an expanded exon 3 on their other allele, lessening the overall effect of the mutation. This might explain the phenotypically normal heterozygotes seen in Families 5 and 7 (Figure 1). Due to their relatively high population frequencies, filaggrin null-mutations may themselves be modifying factors in other ichthyotic skin conditions, including congenital ichthyoses, Netherton syndrome or disorders due to defects in suprabasal keratins, where intra- and interfamilial phenotypic variation is well documented³¹⁻³⁴. The association of IV with the atopic diathesis is well established; 37-50% of people with IV have atopic diseases^{1,36} and conversely around 8% of atopic dermatitis patients have classical features of IV^{1,35}. Thus, filaggrin may be a factor in very common skin disorders known to have a major genetic component.

In the IV families studied, many filaggrin-null or heterozygous individuals also had atopic dermatitis (AD; "eczema") and/or asthma. An example is shown in Figure 5, where 3/6 filaggrin-null heterozygotes and 5/5 homozygotes had atopic disease. The inventors therefore sought to examine the role of these variants in common atopy associated with asthma. The two filaggrin variants were genotyped in a cohort of 800 schoolchildren with unknown disease status (population cohort) and in 550 school children and adolescents with physician-diagnosed asthma from the Dundee BREATHE study. The frequency of carriers of R501X was 5.2% and the 2282del4 variant was present in 3.6% of the schoolchildren, giving a combined carrier frequency of 8.8%. Both filaggrin variants were over-represented in the asthmatic cohort, with carriers of either allele demonstrating a dominant risk (Table 2; combined genotype OR= 1.94 95%CI= 1.35-2.80, p= 0.0005). Homozygotes for both variants were observed in the asthma cohort, as were two compound heterozygotes. AD is known to be co-associated with asthma^{1,36} and since filaggrin is a major epidermal structural protein, one would expect a stronger association with AD/asthma with filaggrin defects. Consistent with this, 75% of all the children in the asthma cohort carrying a filaggrin null-allele had AD, in contrast to only 46.7% of those without these filaggrin variants (Table 3; OR= 2.81 95%CI= 1.64-4.81, p=0.0001). This observation appears to be related to allergen exposure, as the risk was largely seen in individuals routinely exposed to animals (OR 5.2 95%CI= 2.36-11.50, p=0.000006).

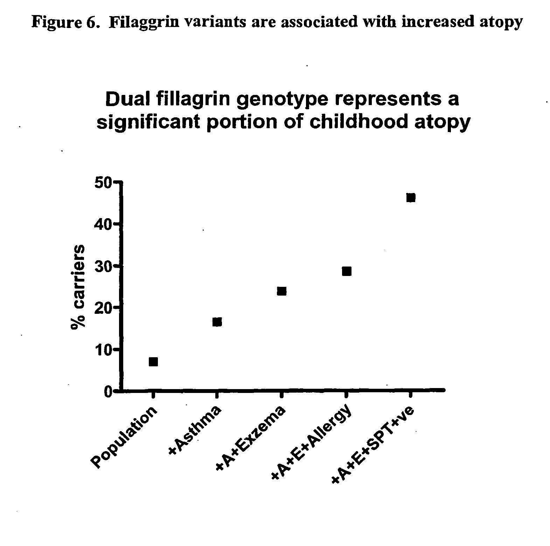

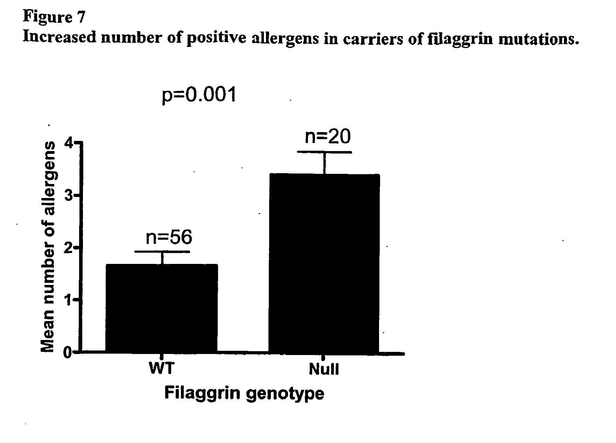

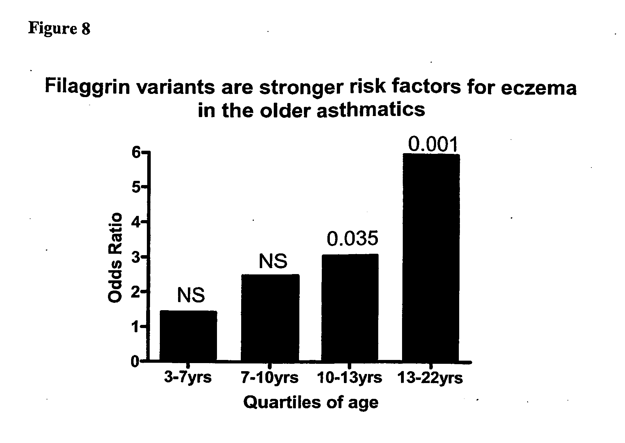

These data suggested that a barrier function defect may lead to greater risk of allergy and this hypothesis was supported by the observation that a significantly greater number of the children with asthma had been referred to an allergy clinic for allergy testing if they carried a filaggrin null variant (OR= 1.78 95%CI=1.08-2.95, p= 0.034). Even more significantly, every individual tested that carried the null variants (n=20) was positive for at least one allergen, whereas 26 out of the 56 non-carrier individuals tested negative for allergens (p=0.00006). This striking atopic phenotype resulted in a systematic increase of the carrier frequency of the null variants as we increased the definition of the degree of atopy of the cohort (Figure 6), with 46.7% of all the individuals that had asthma, AD and immunologically-verified allergy having the filaggrin null variants (n=75). In this group, the variant was still further associated with an increased number of positive allergens, with the mean number of allergens for a null variant carrier being substantially greater that that for the wild type individuals (Figure 7). This demonstrates that a substantial fraction of common, complex atopic disease can be accounted for by a single pair of variants in the filaggrin gene. Given the population carrier frequency of both of these variants (∼9%), these observations will have a huge impact in the understanding of atopy and may lead to the development of novel treatments for asthma and allergy. Asthma and allergy can wane during adolescence and the temporal features of childhood atopy are known as the "atopic march". Here the inventors show that individuals haploinsufficient for filaggrin represent a population of persistent atopy, where the observed risk of eczema increases consistently through age in the asthmatic cohort, consistent with the atopic march (Figure 8).

### Nut and food allergy

The asthma cohort was analysed for people with a recorded allergy to peanuts and/or other nuts. 23 individuals had a proven nut allergy and of these, 8 carried a filaggrin-null mutation (either 2282del4 or R501X), i.e. 35% were carriers. In a population control cohort, 60 out of 621 individuals carried a filaggrin-null mutation, i.e. 9.7%. Thus, the filaggrin mutation carrier frequency is greatly increased in people with asthma and nut allergy. Fisher's exact test gave a two-sided P value of 0.0008, which is considered extremely significant (odds ratio = 5.520 95%CI 2.248-13.554). Thus, filaggrin mutations are a highly significant risk factor for nut allergy and asthma. In addition, atopy is strongly associated with food allergy especially cow's milk, hen's egg, banana, kiwi, white fish, wheat, prawns/shrimp and strawberries³⁷⁻³⁹. Since nut allergy is regarded as the most reliable marker for food allergy, this strongly infers that filaggrin mutations are associated with food allergies in general.

### A comprehensive mutation detection strategy for FLG.

Using primers ending on bases that had been determined by detailed sequence alignments to be absolutely specific for a given filaggrin repeat, or in a few cases, ending on bases that are shared by only two filaggrin repeats, a series of overlapping PCR fragments was generated that span exon 3 of the *FLG* gene in its entirety. These fragments were fully sequenced using the amplification primers and/or internal primers that again ended on unique bases. In some individuals, the identification of single nucleotide polymorphisms was able to show that the overlapping PCR fragments were amplifying both alleles, thus demonstrating the specificity and utility of this sequencing strategy. Using this method, individuals with a severe IV phenotype predictive of homozygous or compound heterozygous mutations were sequenced. These patients were from European populations, predominantly Irish and Scottish with some Dutch and Austrian individuals. A number of novel loss-of-function mutations were identified, all of which lead to premature termination codons, either as nonsense mutations or frameshift mutations. In many cases, these were inherited in *trans* with the more common mutations R501X or 2282del4. Specifically, these further mutations were 3702delG (repeat 3), R2447X (repeat 7), S3247X (repeat 9), R1474X (repeat 4), Q1745X (repeat 4), Q3683X (repeat 10), 11029delCA (repeat 10) in Irish and Scottish patients; E2422X (repeat 6), 5360delG (repeat 5), 7267delCA (repeat 7) and 11033del4 (repeat 10) in Dutch patients; and 6867delAG (repeat 6) in an Austrian patient. The IV phenotype of the patients carrying these more 3' mutations were essentially indistinguishable to patients carrying R501X or 2282del4 mutations in repeat 1. Thus, premature termination codon mutations essentially anywhere in the profilaggrin molecule appear to have similar or equivalent pathogenicity. The following mutations were recurrent and/or prevalent in the European population R501X, 2282de14, 3702delG, R2447X and S3247X. The Dutch and Austrian mutations were not detected in 188 Irish AD patients and may be population-specific or very rare. The remaining variants were not tested for prevalence.

### Mutations in the 3' half of FLG exon 3 are essentially functional null alleles

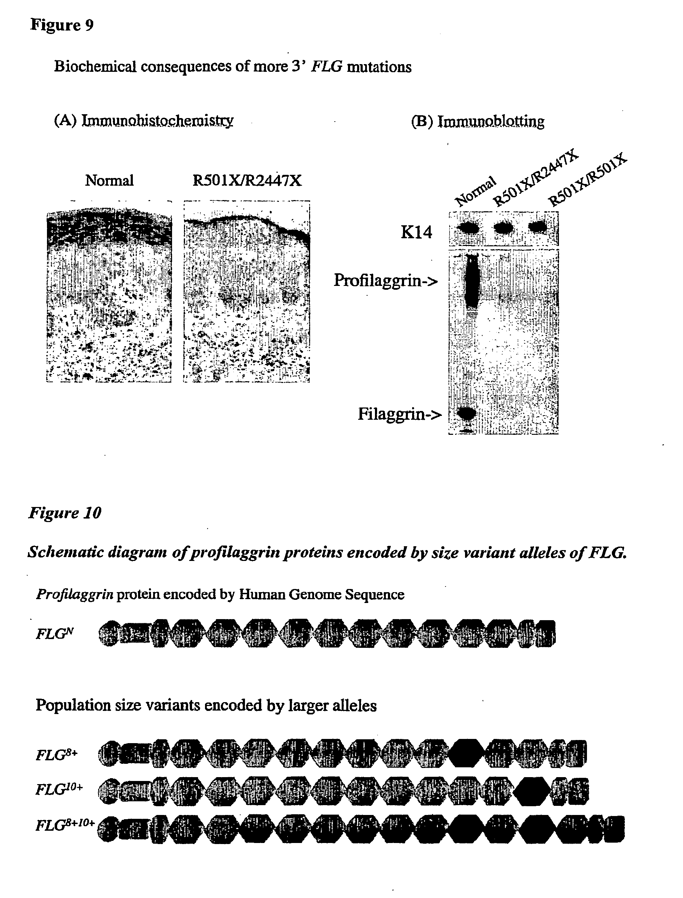

To determine the biochemical consequences of the more 3' mutations, biopsy material was obtained from two patients with the compound heterozygote genotype R501X/R2447X. Immunostaining of skin sections with Novocastra 15C10 antibody against the filaggrin repeat domain showed that this patient has an identifiable but very restricted granular cell layer in the upper epidermis **(****Figure 9A****).** The more quantitative technique of immunoblotting, using protein extracts from this biopsy, revealed that only a very small quantity of a truncated profilaggrin molecule is expressed and importantly, this is not processed into mature filaggrin **(****Figure 9B****).** Essentially identical results were obtained for an R501X/11033del4 compound heterozygote (not shown). Thus, more 3' *FLG* mutations lead to greatly reduced expression of truncated profilaggrin and complete loss of mature filaggrin and therefore, are essentially filaggrin functional null alleles.

### Mutations in more 3' repeats and repeat 1 of the FLG gene predispose to AD

A cohort of 188 Irish paediatric cases of moderate-severe AD were genotyped for 5 mutations found to be prevalent and/or recurrent in this population, R501X, 2282del4, 3702delG, R2447X and S3247X. Comparison of allele frequencies was made to an unselected Irish control population of 736 individuals genotyped for the same 5 *FLG* variants. Pearson chi-square analysis revealed that all 5 mutations are independently associated with the AD phenotype, giving individual statistically significant P values of <0.05 (**Table 4**). Combining the data for all 5 genotypes gave an extremely significant *P* value of 2.12 x 10⁻⁵¹. About 48% of the patients in the AD cohort carried one or more of the 5 filaggrin variants. Thus, a wide range of *FLG* mutations contribute to genetic predisposition to atopy and this is a major gene in early-onset moderate-to-severe AD.

### A different spectrum of FLG mutations predispose to atopy in non-European populations

The European-specific mutations R501X and 2282de14 were found to be absent from 253 Japanese individuals. We therefore sequenced the *FLG* gene in four Japanese families with IV and identified two novel mutations, 3321delA and S2554X.

We screened 143 Japanese AD patients for these null *FLG* mutations and identified them in 8 AD patients (5.6%), including S2554X in 6 patients (4.2%) and 3321delA in 2 patients (1.4%). Both null variants were absent from 156 Japanese non-atopic and non-ichthyotic controls, giving a statistically significant association between the *FLG* mutations and AD (Chi-square *P* value 0.0015).

Thus, in non-European populations, in this case Japan, there appears to be a distinct set of prevalent/recurrent *FLG* mutations that contribute to genetic predisposition to atopy. It is likely that other human populations will have their own spectrum of *FLG* mutations.

### Size variants of the filaggrin gene (FLG)

It has been reported previously that exon 3 of the *FLG* gene is variable in size in the human population and these variant alleles were predicted, on the basis of their size, to consist of 10, 11 or 12 full filaggrin repeats in addition to the two partial repeats (Gan et al., 1990)⁴⁰. However, the positions of these insertions within *FLG* exon 3 and the precise DNA sequences encoding these additional filaggrin repeats remained unknown.

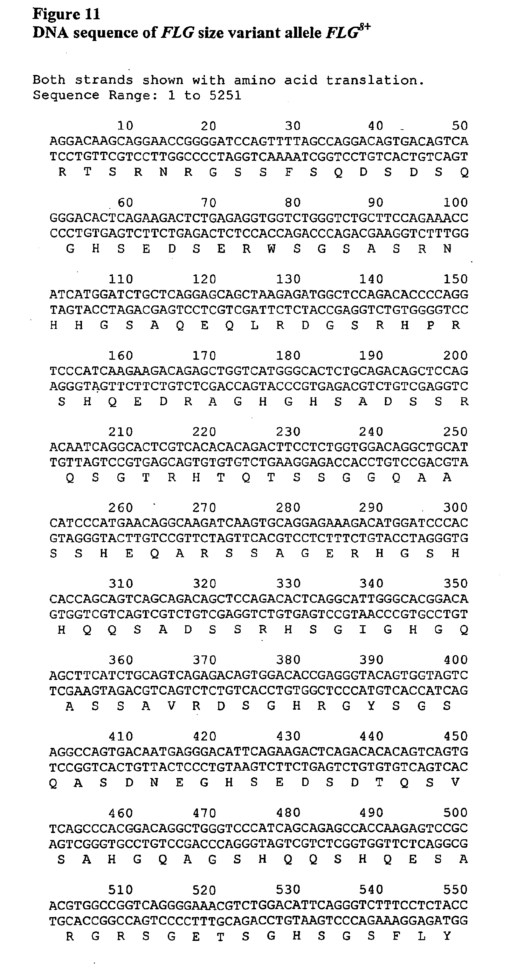

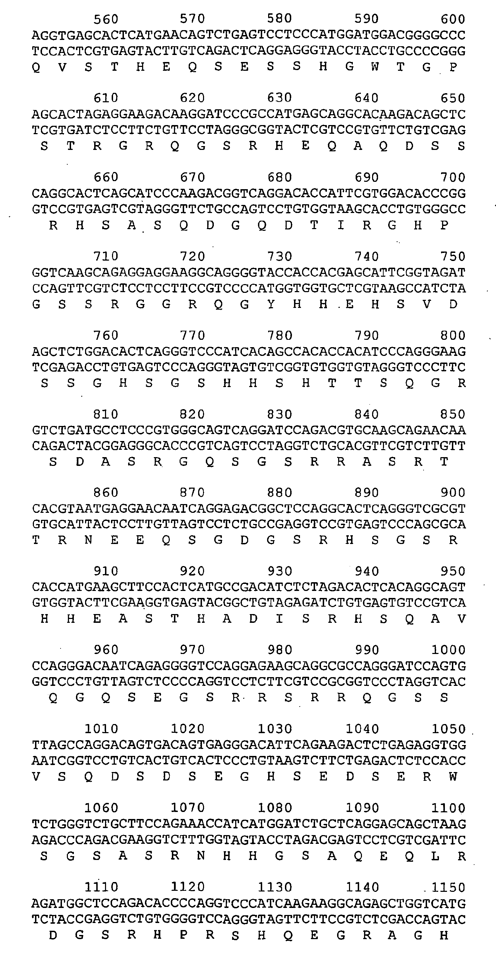

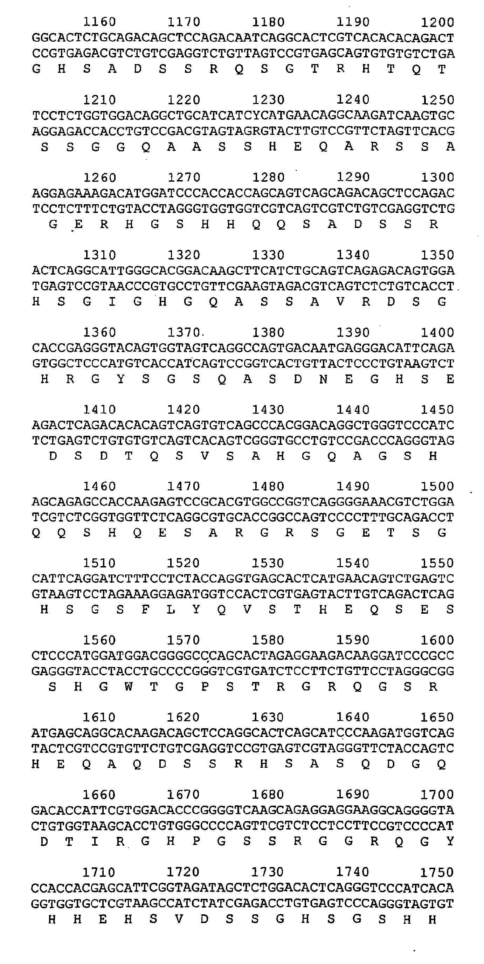

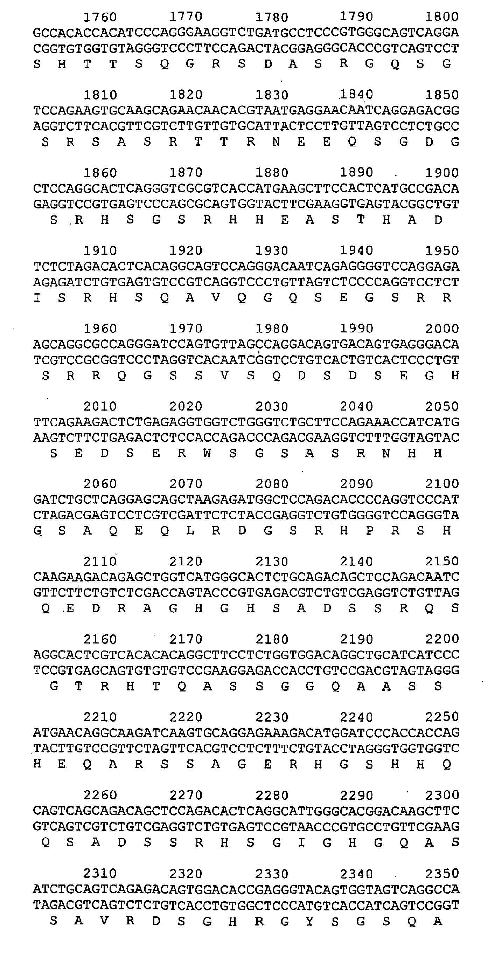

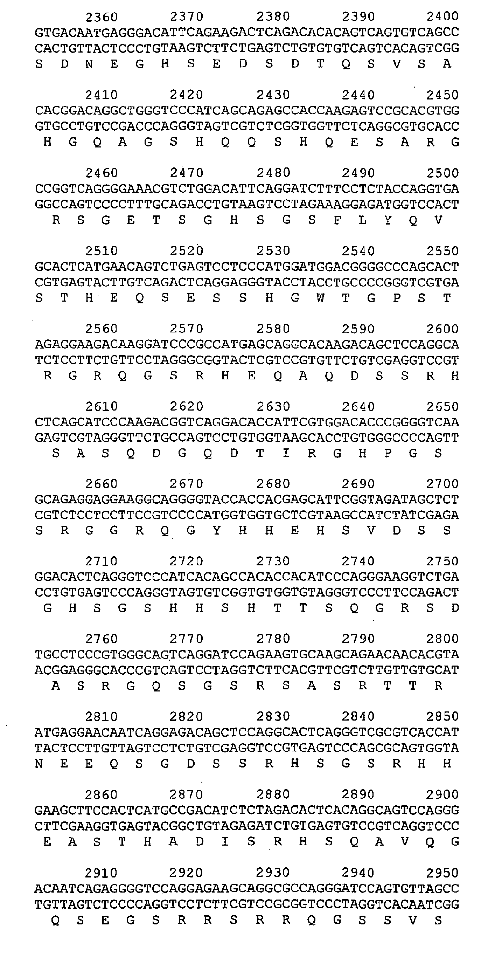

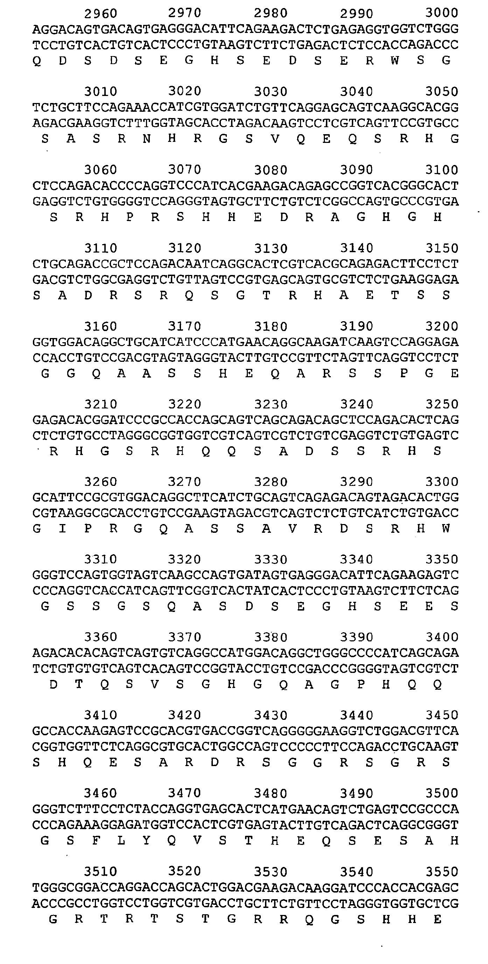

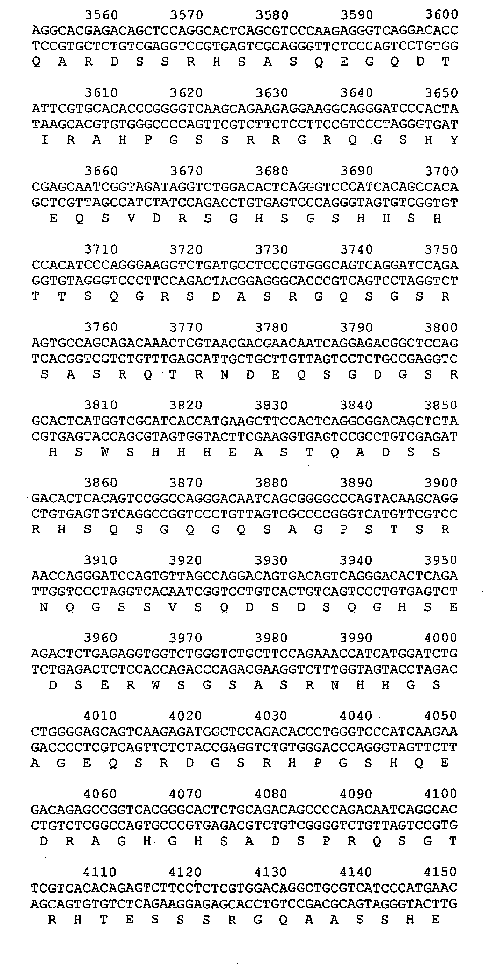

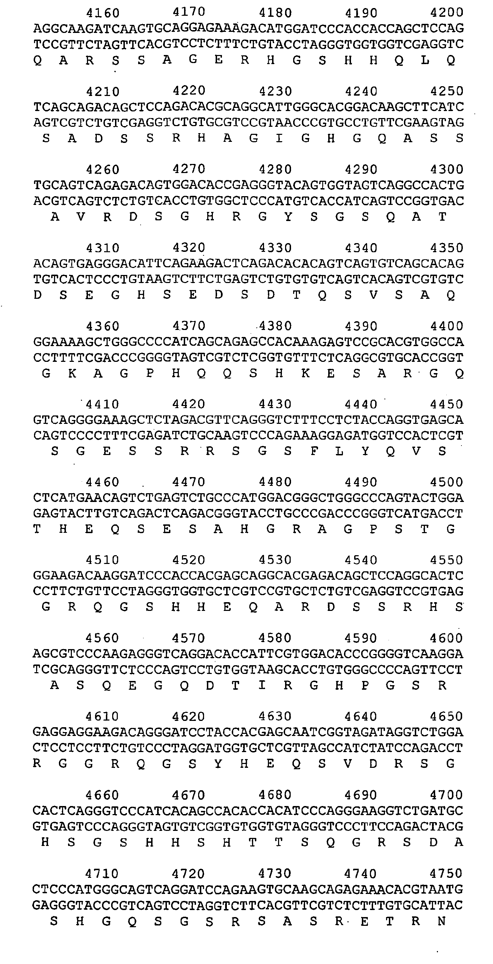

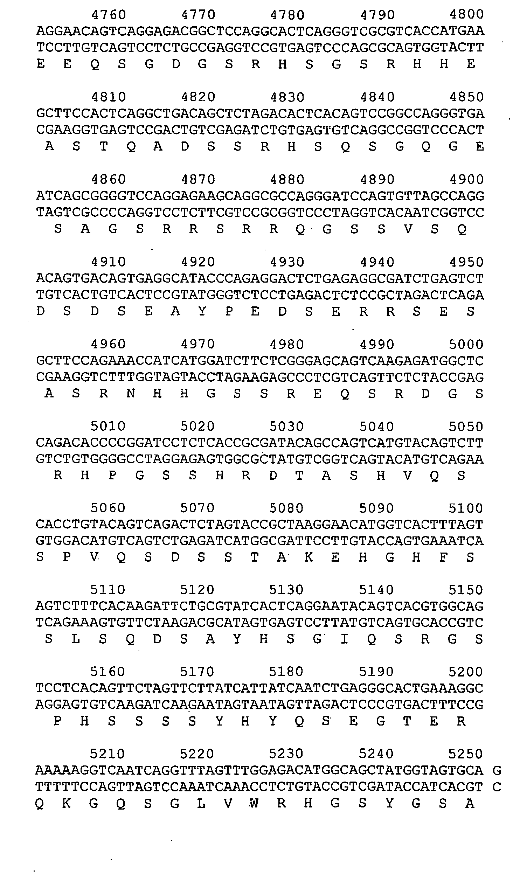

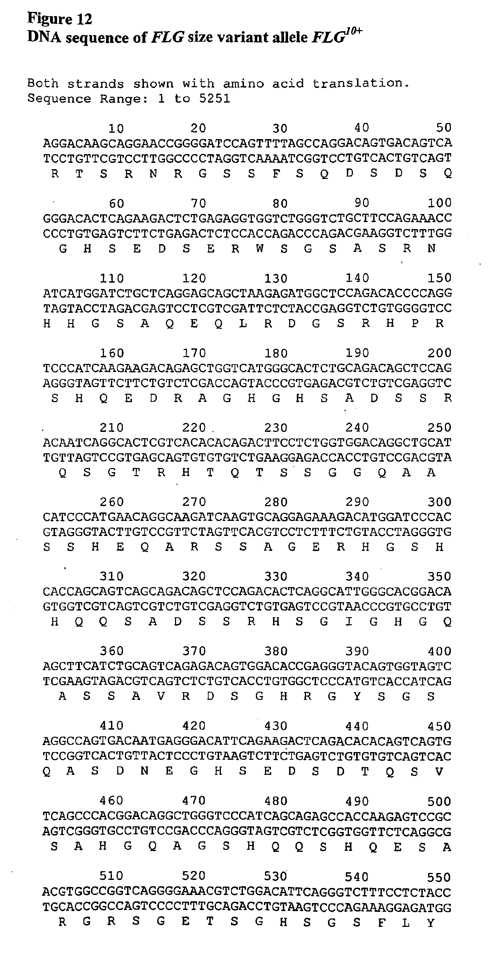

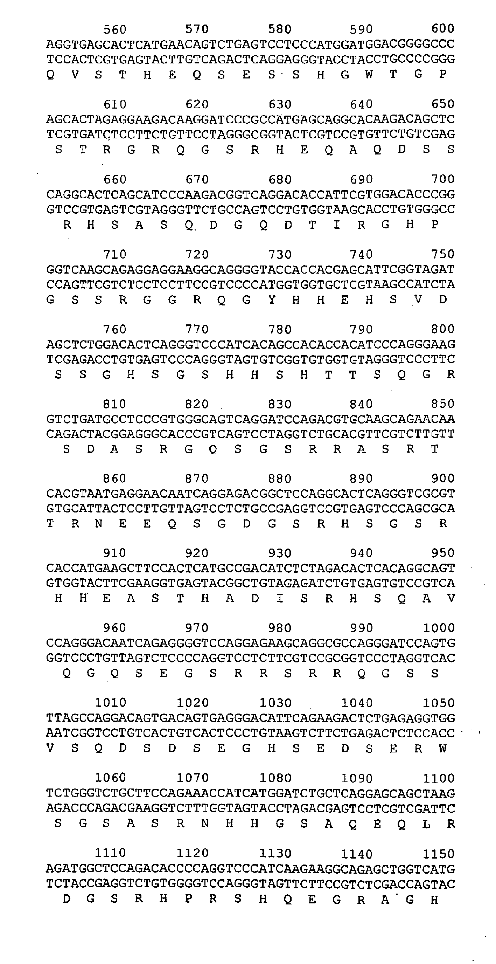

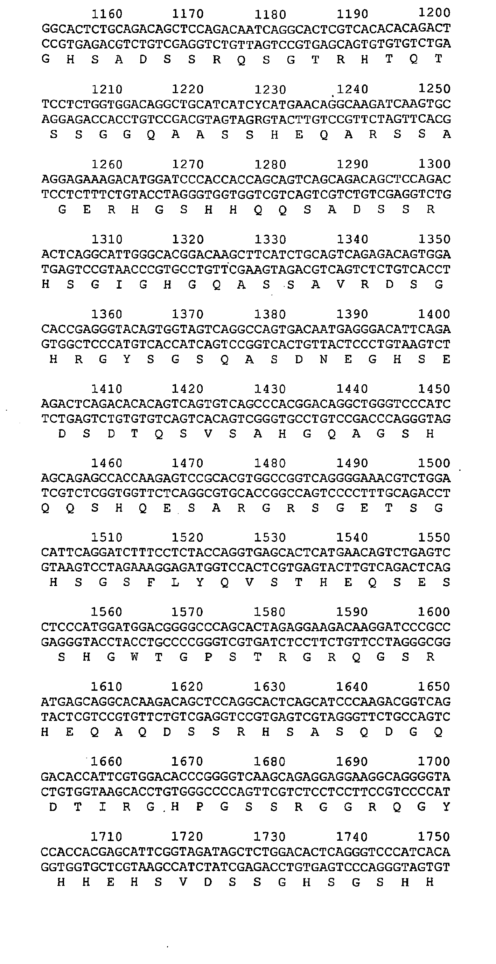

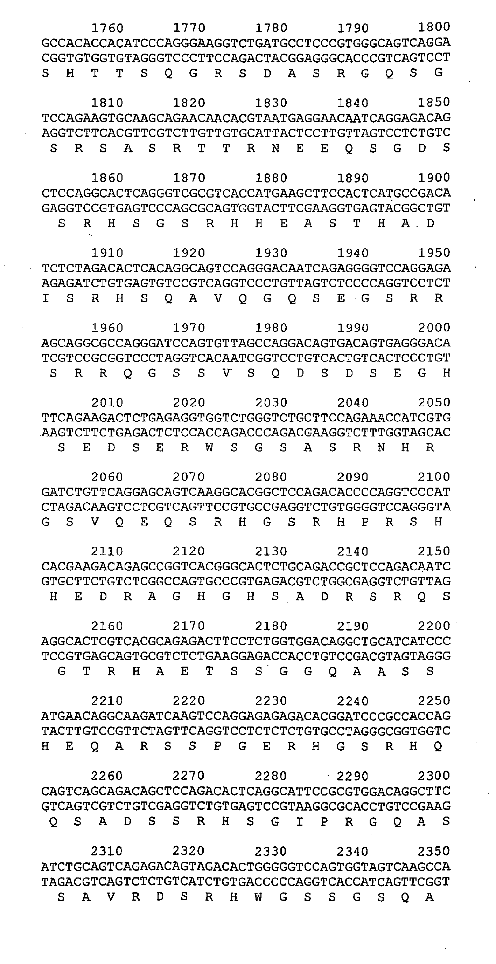

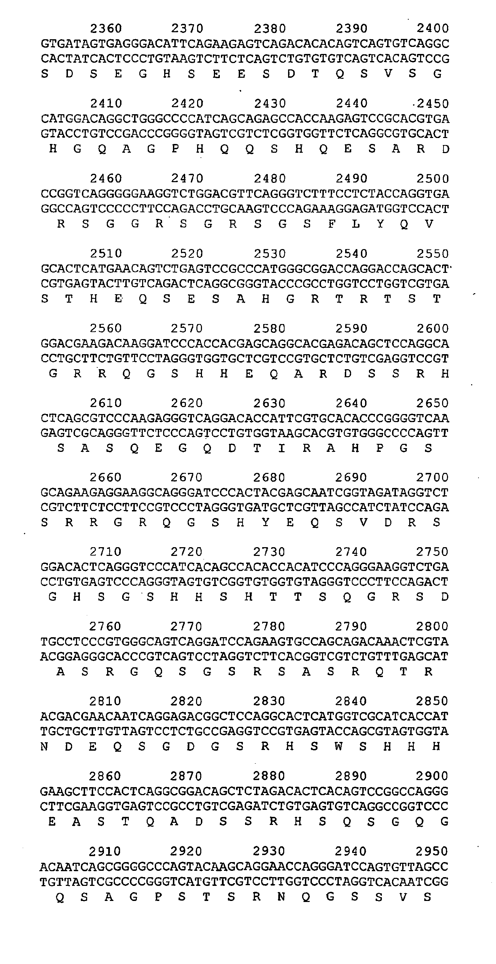

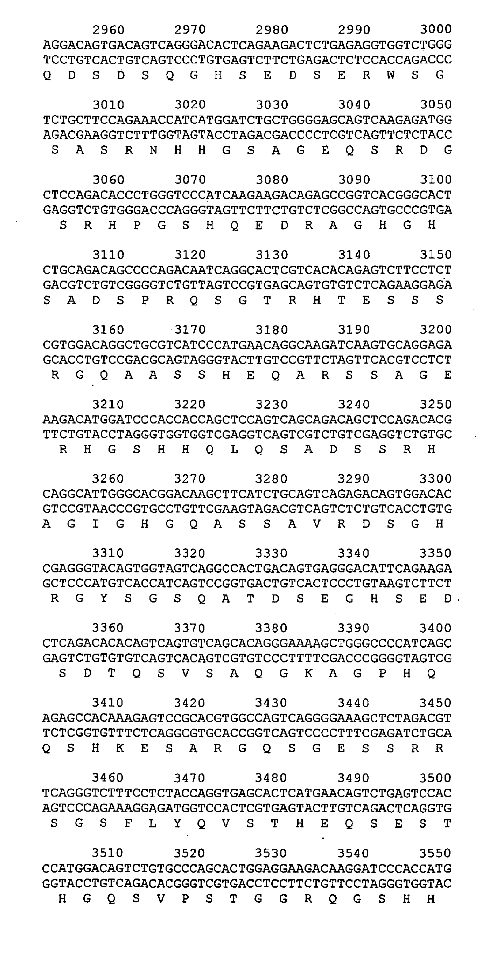

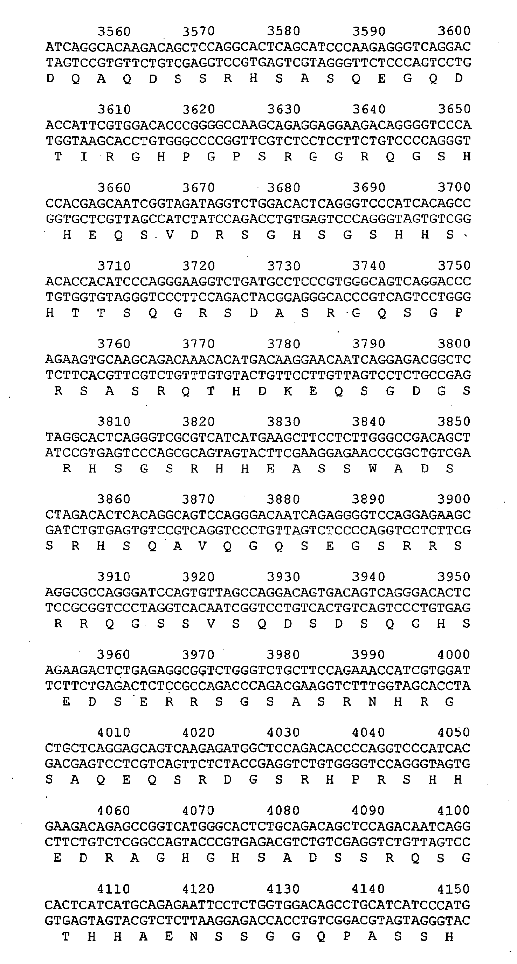

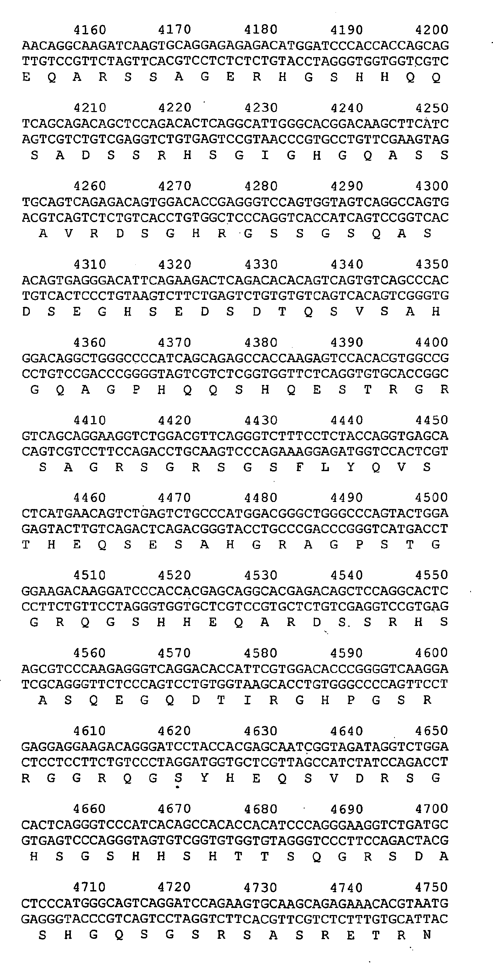

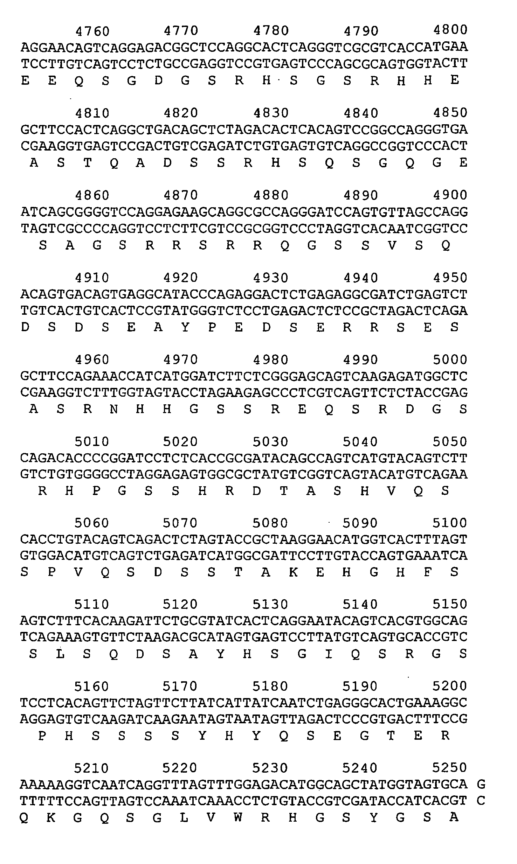

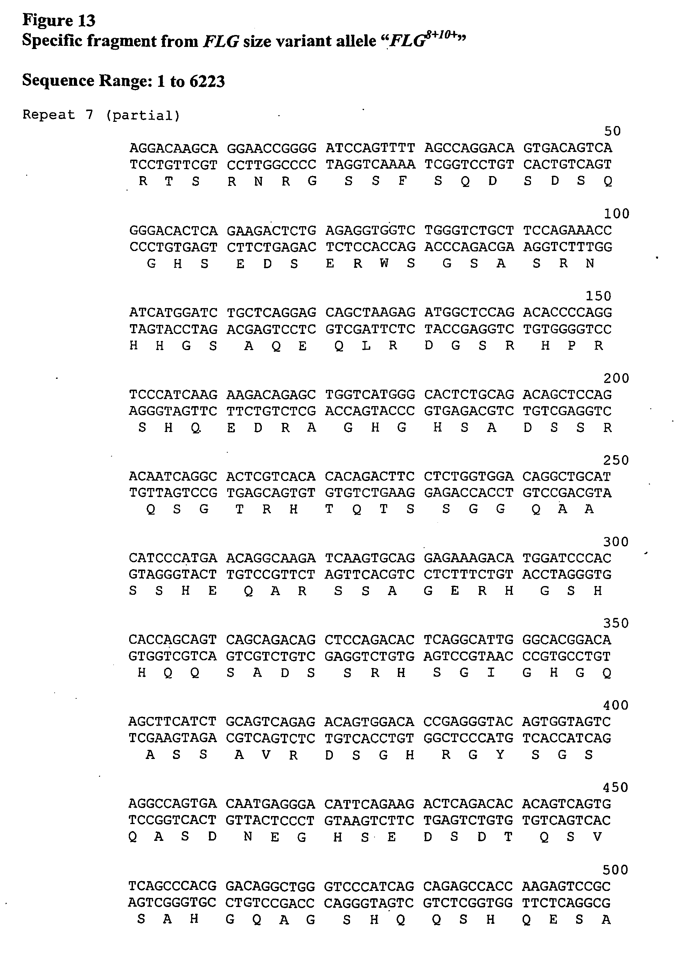

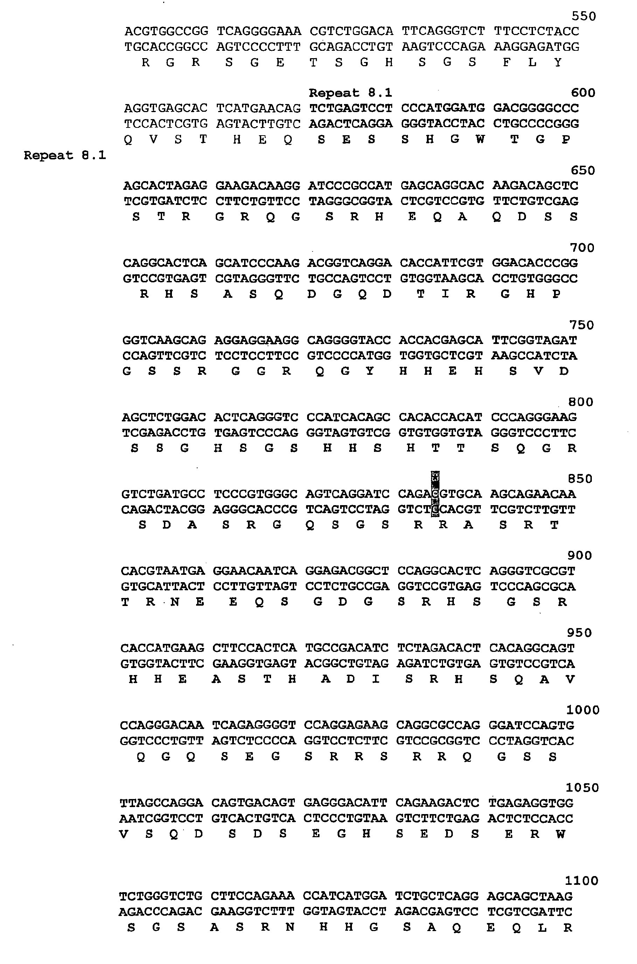

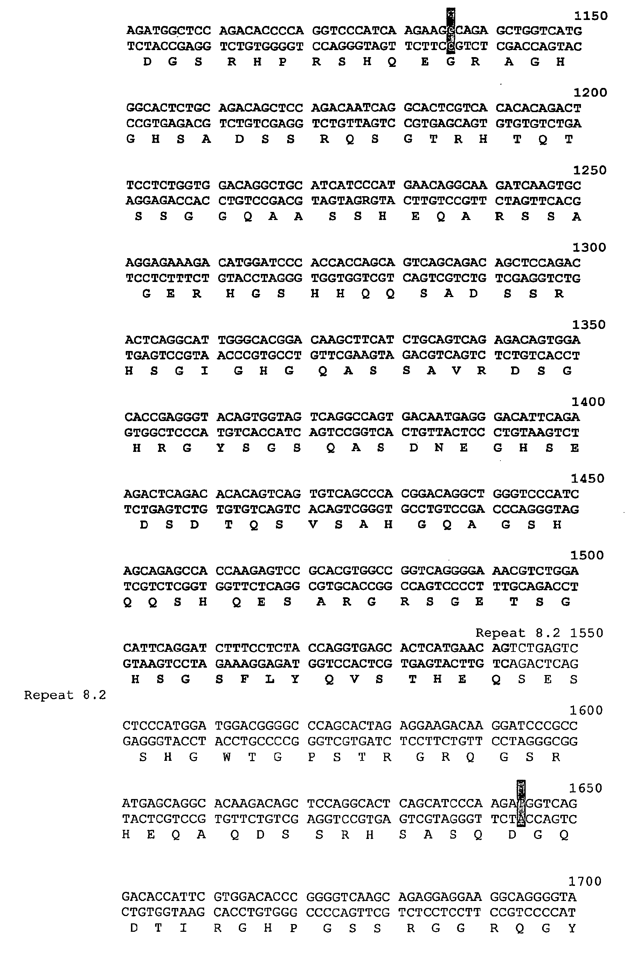

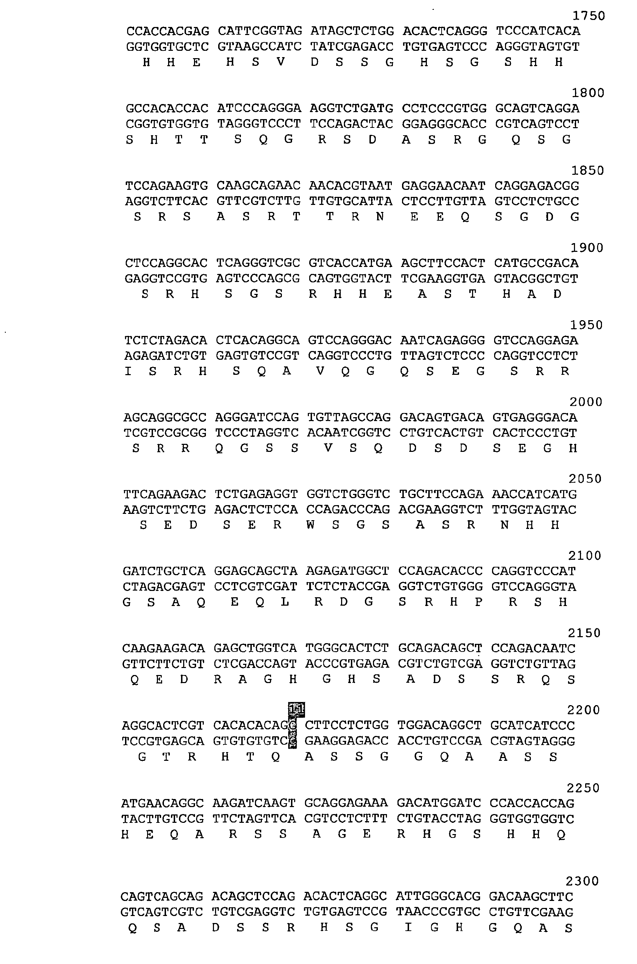

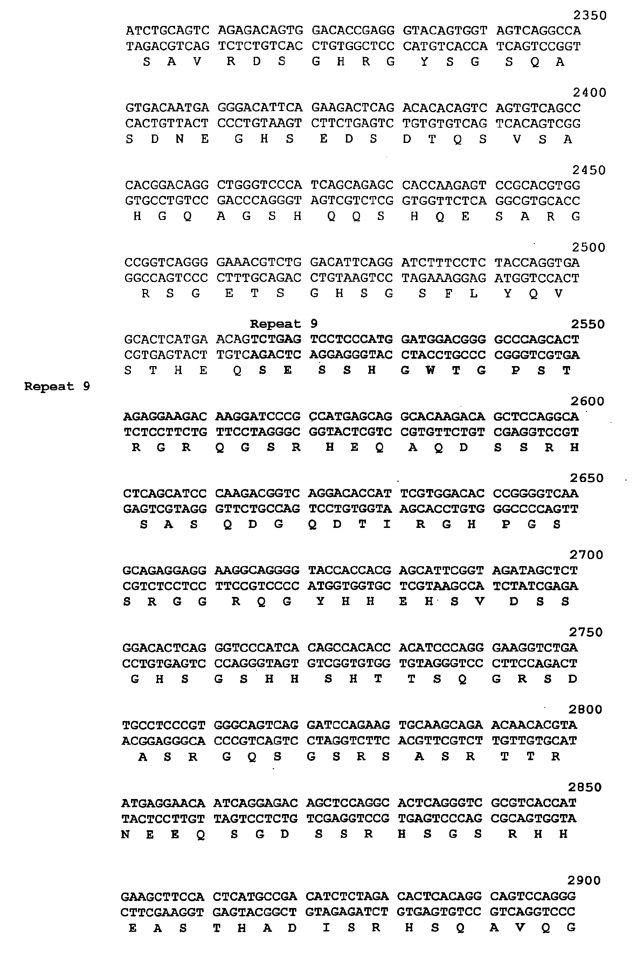

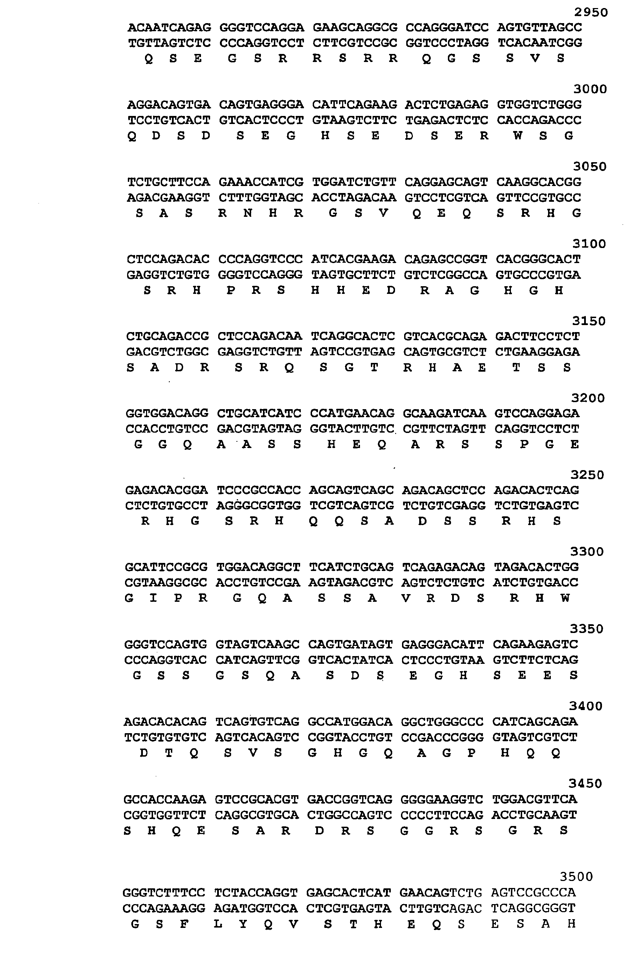

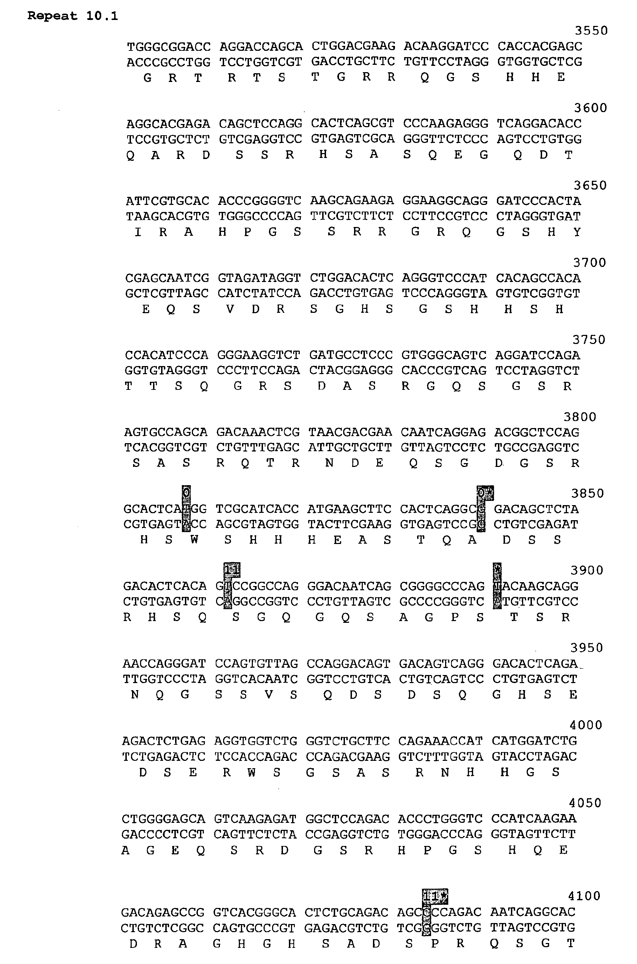

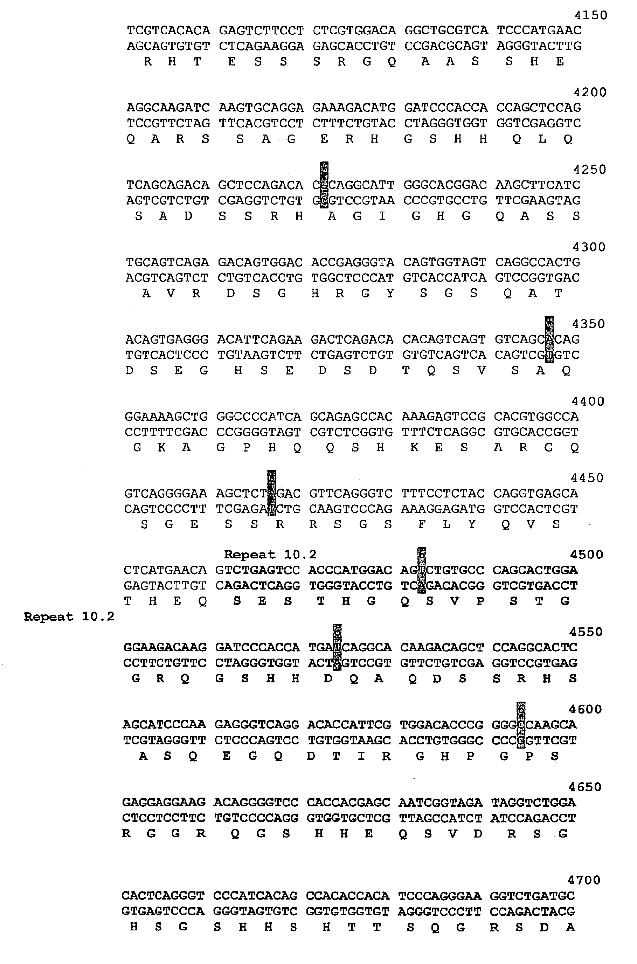

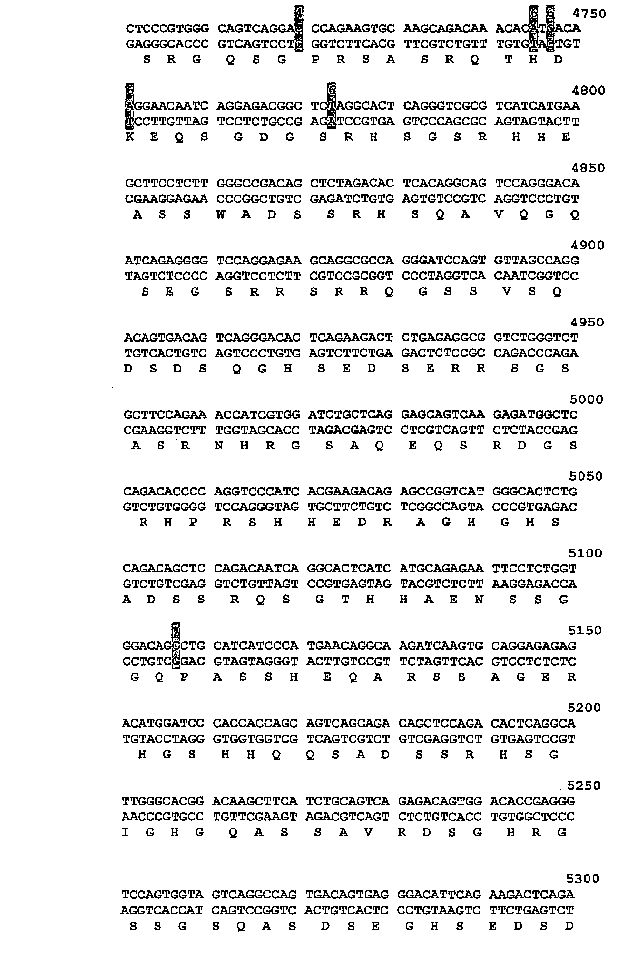

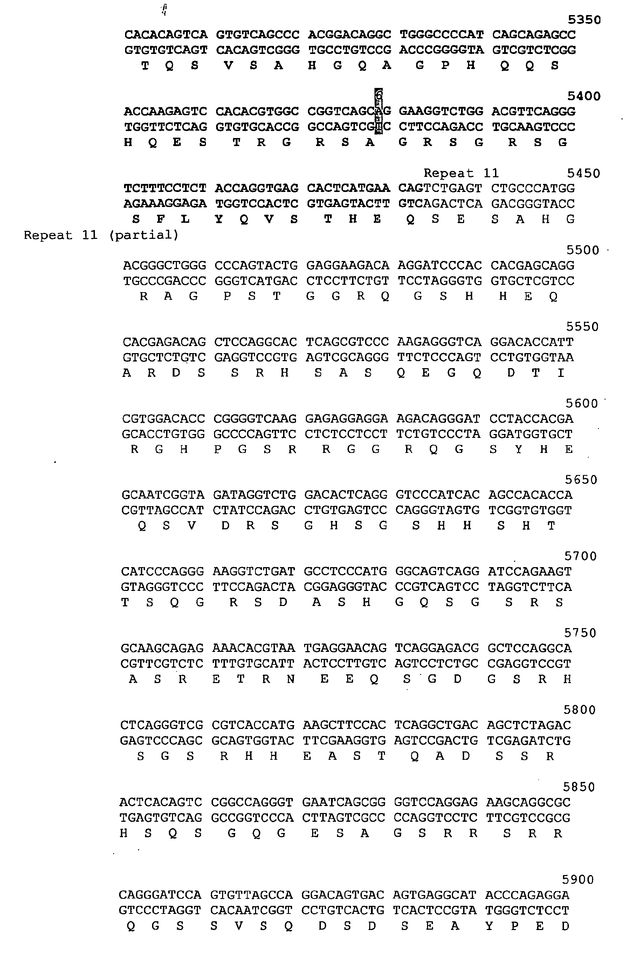

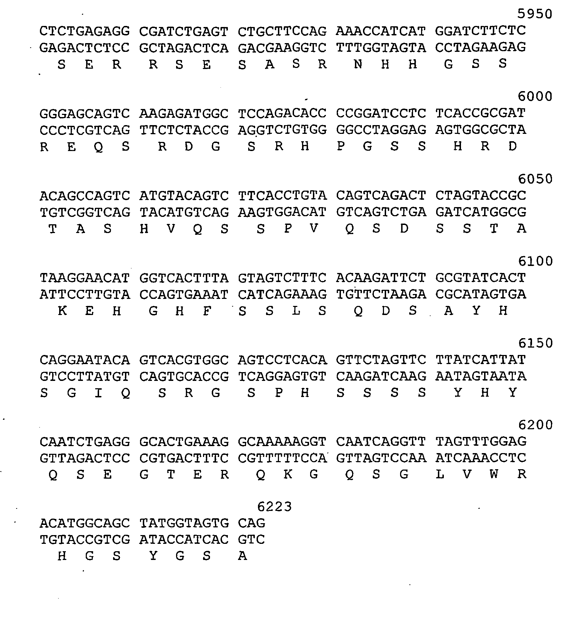

Using specific PCR primers located in repeat 7 and repeat 11, DNA fragments were generated from unrelated individuals that, from the public sequence of the *FLG* gene (Human Genome, March 2006 Assembly, hg18), would be predicted to be ∼4.2 kb in size. For convenience, this allele is designated as *FLG^{N}.* In some individuals, additional bands of ∼5.2 and/or ∼6.2 kb were observed. These larger alleles were cloned into plasmid vector pCR3.1 to allow full sequencing of these variant alleles. This revealed that some individual alleles contain a duplication of repeat 8, designated as *FLG⁸⁺.* Another allele consisted of what was essentially a duplication of repeat 10, which was designated as *FLG¹⁰⁺.* Both copies of the repeat 10 sequences on this allele showed some sequence divergence from the published genome sequence. Similarly, there were a smaller number of sequence differences between the two repeat 8 copies on the *FLG⁸⁺* allele. In some individuals, a larger allele was present consisting of the duplicated repeat 8 and the duplicated repeat 10, which was designated as *FLG⁸⁺¹⁰⁺.* The size variants are shown diagrammatically in **Figure 10**, compared to the hg18 genome database sequence, labelled *FLG^{N}.* The raw sequences of the duplicated regions of these alleles are shown in **Figures 11-13**. A fully annotated sequence of the *FLG⁸⁺¹⁰⁺*, representing all the novel sequence data generated here, with the positions of the previously known and novel filaggrin repeat sequences, is shown in **Figure 13****.**

By alignment of these additional repeat sequences with all the existing filaggrin repeats, a number of priming sites were identified that would allow specific amplification, sequencing and mutation detection within these novel alleles. Forward and reverse primers ending on these specific bases are listed in the sequence listing as SEQ ID Nos. **494 - 539.** It is recognised that the length of these primers may be varied and still allow specific PCR amplification, sequencing, or mutation analysis, provided the 3' end of the primer ends on these specific bases or very close to them. The specific bases within the duplicated repeat regions are also annotated on **Figure** 13.

It is recognised that some individuals with ichthyosis vulgaris and/or atopic disease may carry loss-of-function mutations within the newly identified sequences that constitute these new size variant alleles. It is also recognised that size variation may modulate the phenotype of heterozygous carriers of a loss-of-function or other mutation in the *FLG* gene, i.e. a heterozygous carrier of the R501X mutation may carry a second wild-type allele encoding either 10, 11 or 12 filaggrin repeats. It is recognised that the size of the heterozygous wild-type allele may influence the phenotype observed, for example, a carrier of the 12-repeat (*FLG*^{*8+10*+}) allele will express 20% more filaggrin than a R501X carrier carrying a 10-repeat wild-type allele in *trans.* Thus, detection of these size variants may be of prognostic value in ichthyosis vulgaris and atopic disease.

### REFERENCES

1. Wells, R.S. and Kerr CB, Br Med J., 1:947-949 (1966).
2. Judge, M.R., McLean, W.H.I. & Munro, C.S. Disorders of keratinization. in Rook's Textbook of Dermatology, Vol. 2 (eds. Bums, T., Breathnach, S., Cox, C. & Griffiths, C.) 34.54-34.56 (Blackwell Scientific Publishing, Oxford, 2004).
3. Steinert, P.M., Cantieri, J.S., Teller, D.C., Lonsdale-Eccles, J.D. & Dale, B.A. Characterization of a class of cationic proteins that specifically interact with intermediate filaments. Proc Natl Acad Sci 78, 4097-4101 (1981).
4. Dale, B.A., Resing, K.A. & Lonsdale-Ecccles, J.D. Filaggrin : a keratin filament associated protein. Ann. NY Acad. Sci. 455, 330-342 (1985).
5. Listwan, P. & Rothnagel, J.A. Keratin bundling proteins. Methods Cell Biol 78, 817-27 (2004).
6. Gan, S.Q., McBride, O.W., Idler, W.W., Markova, N. & Steinert, P.M. Organization, structure, and polymorphisms of the human profilaggrin gene. Biochemistry 29, 9432-40 (1990).
7. Candi, E., Schmidt, R. & Melino, G. The cornified envelope: a model of cell death in the skin. Nat Rev Mol Cell Biol 6, 328-40 (2005).
8. Fleckman, P., Holbrook, K.A., Dale, B.A. & Sybert, V.P. Keratinocytes cultured from subjects with ichthyosis vulgaris are phenotypically abnormal. J Invest Dermatol 88, 640-5 (1987).
9. Pena Penabad, C. et al. Differential patterns of filaggrin expression in lamellar ichthyosis. Br J Dermatol 139, 958-64 (1998).
10. Sybert, V.P., Dale, B.A. & Holbrook, K.A. Ichthyosis vulgaris: identification of a defect in synthesis of filaggrin correlated with an absence of keratohyaline granules. J Invest Dermatol 84, 191-4 (1985).
11. Nirunsuksiri, W., Zhang, S.H. & Fleckman, P. Reduced stability and bi-allelic, coequal expression of profilaggrin mRNA in keratinocytes cultured from subjects with ichthyosis vulgaris. J Invest Dermatol 110, 854-61 (1998).
12. Presland, R.B. et al. Loss of normal profilaggrin and filaggrin in flaky tail (ft/ft) mice: an animal model for the filaggrin-deficient skin disease ichthyosis vulgaris. J Invest Dermatol 115, 1072-81 (2000).
13. Lane, P.W. Two new mutations in linkage group XVI of the house mouse. Flaky tail and varitint-waddler-J. JHered 63, 135-40 (1972).
14. Rothnagel, J.A. et al. Characterization of the mouse loricrin gene: linkage with profilaggrin and the flaky tail and soft coat mutant loci on chromosome 3. Genomics 23, 450-6 (1994).
15. Sambrook & Russel; "Molecular Cloning - A Laboratory Manual", (2000); Cold Spring Harbor Laboratory Press.
16. Dale BA, Holbrook KA, Kimball JR, Hoff M, Sun TT., Expression of epidermal keratins and filaggrin during human fetal skin development., J Cell Biol. 1985 Oct; 101(4):1257-60.
17. Gene Therapy (2004) 11, S57 - 63.
18. Lee DS, Quan G, Choi JY, Kim SY, Lee SC.Chronic ultraviolet radiation modulates epidermal differentiation as it up-regulates transglutaminase 1 and its substrates. Photodermatol Photoimmunol Photomed. 2005 Feb;21(1):45-52.
19. Wall, et al. (1992) J. Cell Biochem. 49(2): 113-20.
20. McCreath, et al. (2000) Nature 405: 1066-1069.
21. Lai, et al. (2002) Science 295:1089-92.
22. Hogan, et al. (1986) In: Manipulating the mouse embryo. A Laboratory Manual. Cold Spring Harbour Laboratory Press, Cold Spring Harbor, N.Y.
23. Pearton, D.J., Dale, B.A. & Presland, R.B. Functional analysis of the profilaggrin N-terminal peptide: identification of domains that regulate nuclear and cytoplasmic distribution. J Invest Dermatol 119, 661-9 (2002).
24. Eady, R.A.J. Transmission electron microscopy. in Methods in Skin Research (eds. Skerrow, D. & Skerrow, C.J.) 1-36 (John Wiley & Sons, Chichester, 1985).
25. Presland, R.B., Haydock, P.V., Fleckman, P., Nirunsuksiri, W. & Dale, B.A. Characterization of the human epidermal profilaggrin gene. Genomic organization and identification of an S-100-like calcium binding domain at the amino terminus. J Biol Chem 267, 23772-81 (1992).
26. Markova, N.G. et al. Profilaggrin is a major epidermal calcium-binding protein. Mol Cell Biol 13, 613-25 (1993).
27. Compton, J.G., DiGiovanna, J.J., Johnston, K.A., Fleckman, P. & Bale, S.J. Mapping of the associated phenotype of an absent granular layer in ichthyosis vulgaris to the epidermal differentiation complex on chromosome 1. Exp Dermatol 11, 518-26 (2002).
28. Presland, R.B. et al. Evidence for specific proteolytic cleavage of the N-terminal domain of human profilaggrin during epidermal differentiation. J Invest Dermatol 108, 170-8 (1997).
29. Ishida-Yamamoto, A., Takahashi, H., Presland, R.B., Dale, B.A. & Iizuka, H. Translocation of profilaggrin N-terminal domain into keratinocyte nuclei with fragmented DNA in normal human skin and loricrin keratoderma. Lab Invest 78, 1245-53 (1998).
30. Rothnagel, J.A. & Steinert, P.M. The structure of the gene for mouse filaggrin and a comparison of the repeating units. J. Biol. Chem. 265, 1862-1865 (1990).
31. Bale, S.J., Compton, J.G., Russell, L.J. & DiGiovanna, J.J. Genetic heterogeneity in lamellar ichthyosis. J Invest Dermatol 107, 140-1 (1996).
32. Bitoun, E. et al. Netherton syndrome: disease expression and spectrum of SPINK5 mutations in 21 families. J Invest Dermatol 118, 352-61 (2002).
33. Smith, F.J.D. et al. Genomic organization and fine mapping of the keratin 2e gene (KRT2E): K2e V1 domain polymorphism and novel mutations in ichthyosis bullosa of Siemens. J. Invest. Dermatol. 111, 817-821 (1998).
34. McLean, W.H.I. et al. Mutations in the rod 1A domain of keratins 1 and 10 in bullous congenital ichthyosiform erythroderma (BCIE). J. Invest. Dermatol. 102, 24-30 (1994).
35. Tay, Y.K., Khoo, B.P. & Goh, C.L. The epidemiology of atopic dermatitis at a tertiary referral skin center in Singapore. Asian Pac J Allergy Immuno/ 17, 137-41 (1999).
36. Koukkanen K., Ichthyosis vulgaris. A clinical and histopathological study of patients and their close relatives in the autosomal dominant and sex-linked forms of the disease. Acta Derm Venereol Suppl (Stockh). 1969;62:1-72.
37. Werfel T, Breuer K., Curr Opin Allergy Clin Immunol. 2004 Oct; 4(5): 379-85. Role of food allergy in atopic dermatitis.
38. Sicherer S.H., Sampson H.A., J. Allergy Clin Immunol. 1999 Sep; 104 (3 Pt 2): S114-22. Food hypersensitivity and atopic dermatitis: pathophysiology, epidemiology, diagnosis and management.
39. Ellman L.K., Chatchatee P., Sicherer S.H. & Sampson H.A. Pediatr Allergy Immunol. 2002 Aug; 13(4): 205-8. Food hypersensitivity in two groups of children and young adults with atopic dermatitis evaluated a decade apart.
40. Gan S.Q., McBride O.W., Idler W.W., Markova N., Steinert P.M. Organization, structure, and polymorphisms of the human profilaggrin gene. Biochemistry 1990; 29: 9432-40.

**Table 1**

| Allele frequencies of *FLG* mutations R501X and 2282del4 | | |
|---|---|---|
| **Population** | **Allele Frequency R501X** | **Allele Frequency 2282del4** |
| Irish Caucasian | 0.041 (n = 97) | 0.005 (n = 91) |
| Scottish Caucasian | 0.021 (n = 145) | 0.012 (n =166) |
| US Caucasian | 0.024 (n =124) | 0.011 (n = 133) |
| | | |
| Combined | 0.027 (n = 366) | 0.41 (n = 390) |

**Table 2: Both R501X and 2282del4 are overrepresented in a childhood asthma cohort.**

| R501X | | 2282del4 | | Combined | |
|---|---|---|---|---|---|
| Population | Asthma | Population | Asthma | Population | Asthma |
| 720 | 484 | 714 | 494 | 585 | 390 |
| 38 | 46 | 27 | 35 | 52 | 69 |
| 5 | 2 | 0 | 2 | 6 | 6 |
| 763 | 532 | 741 | 531 | 643 | 465 |
| | p=0.04 | | p=0.0038 | | p=00045 |

**Table 3 Characteristics of asthmatic children with and without filaggrin variants**

| | WT n=390 | NULLcarriers n=75 | p |
|---|---|---|---|
| SEX (%Male) | 59.2 | 59.0 | 0.522 |
| Age | 10.2(2.7-21.2) | 10.3(4.1-22) | |
| BMI | 19.1 | 18.8 | |
| PEFR% | 92.8 | 90.6 | |
| FEV1% | 98.1 | 96.8 | |
| FVC% | 97.1 | 97.0 | |
| Eczema | 46.7 | 73.3 | 0.0001 |
| Perennial Rhinitis | 29 | 26.7 | 0.784 |
| Seasonal Rhinitis | 14.8 | 13.3 | 0.861 |
| Cold air trigger | 41.0 | 44.4 | 0.596 |
| Exercise trigger | 36.1 | 44.4 | 0.213 |
| Viral trigger | 44.9 | 46.8 | 0.787 |
| Tested for allergy | 16.6 | 30.1 | 0.035 |

**Table 4: Case control association study for 5 FLG mutations (188 Irish AD patients versus 736 Irish population controls)**

| Genotype | R501X Population | AD | 2282del4 Population | AD | R2447X Population | AD |
|---|---|---|---|---|---|---|
| AA | 717 | 137 | 717 | 152 | 734 | 181 |
| Aa | 19 | 51 | 19 | 35 | 2 | 7 |
| aa | 0 | 0 | 0 | 1 | 0 | 0 |
| Totals | 736 | 188 | 736 | 188 | 736 | 18 |
| | | p=7.8X10⁻³⁰ | | P=7.8X10⁻¹⁷ | | p=1.7X10⁻⁵ |
| | | | | | | |

| Genotype | S3247X Population | Asthma | 3702delG Population | AD | Combined Population | AD |
|---|---|---|---|---|---|---|
| AA | 720 | 177 | 735 | 186 | 680 | 103 |
| Aa | 16 | 11 | 1 | 2 | 55 | 62 |
| aa | 0 | 0 | 0 | 0 | 1 | 23 |
| | 736 | 188 | 736 | 188 | 736 | 188 |
| | | p=0.008 | | p=0.046 | | p=2.12X10⁻⁵ |

## Claims

1. A genetic test for the diagnosis of ichthyosis vulgaris (IV) and/or to test if a subject is predisposed to developing IV comprising the step of:
detecting *in* vitro whether or not a mutation, which would lead to a loss of function or partial loss of function of the filaggrin (FLG) protein encoded by the filaggrin (*FLG*) gene, is present in the *FLG* gene of a sample of nucleic acid from a subject.

2. The test according to claim 1 wherein the test is used to also detect whether or not a subject is likely to be predisposed or suffering from atopic dermatitis, asthma, or allergies, such as of a contact type allergy and food allergies.

3. A genetic test for atopic dermatitis, asthma, and/or allergies comprising the step of:
detecting *in* vitro whether or not a mutation, which would lead to a loss of function or partial loss of function of the FLG protein encoded by the *FLG* gene, is present in the *FLG* gene of a sample of nucleic acid from a subject.

4. The test according to any of claims 1 to 3 wherein the mutation(s) is selected from the group consisting of R501X, 2282de14, 3702de1G, R2447X (repeat 7), S3247X (repeat 9), R1474X (repeat 4), Q3683X (repeat 10), 11029delCA (repeat 10), E2422X (repeat 6), 5360de1G (repeat 5), 7267de1CA (repeat 7), 11033de14 (repeat 10), 6867delAG (repeat 6), 3321delA (repeat 2) and S2554X (repeat 7).

5. The test according to any preceding claim wherein the mutation(s) is/are detected by quantitative or semi-quantitative PCR, including real-time PCR, nucleic acid sequencing, hybridisation studies and/or restriction fragment length polymorphism (RFLP) analysis techniques.

6. Use of an oligonucleotide identified as SEQ ID. Nos. 1 - 545 in a test according to any preceding claim.

7. Use of an oligonucleotide which having at its 3' position the 3' base identified from any one of SEQ ID. Nos. 1 - 545 and which is approximately 12-50 nucleotides in length, in a test according to any of claims 1-5.

8. A binding agent or antibody which is capable of specifically binding to the peptide generated as a result of the 2282de14 mutation in the filaggrin gene, wherein the binding agent is not capable of binding the native filaggrin peptide.

9. Use of the binding agent or antibody of claim 8 for detecting the peptide generated as a result of the 2282de14 mutation in the filaggrin gene.

10. A method of detecting a mutant peptide generated as a result of the 2284de14 mutation in the filaggrin gene, comprising the steps of:
detecting *in* vitro whether or not the mutant peptide expressed from the mutant *FLG* gene comprising the 2284de14 is present in a sample, by using the binding agent or antibody of claim 8.

11. A transgenic non-human animal which possesses one or more mutations in one or both copies of the *FLG* gene which would lead to a loss or partial loss of function of the *FLG* protein encoded by the *FLG* gene, wherein the one or more mutations are selected from the group consisting of R501X, 2282del4, 3702delG, R2447X (repeat 7), S3247X (repeat 9), R1474X (repeat 4), Q3683X (repeat 10), 11029delCA (repeat 10), E2422X (repeat 6), 5360delG (repeat 5), 7267delCA (repeat 7), 11033del4 (repeat 10), 6867delAG (repeat 6), 3321delA (repeat 2) and S2554X (repeat 7).

12. An *in vivo* assay for identifying an agent which is useful for treating or preventing IV, atopic dermatitis, asthma, and/or allergies associated with mutant *FLG* expression comprising the steps of administering a test agent to the transgenic animal of claim 11; and measuring or determining whether the agent decreases or inhibits at least one sign or symptom of said disease/condition which is indicative that the test agent is capable of treating or preventing said disease/condition.

## Patentansprüche

1. Genetischer Test zur Diagnose von Ichthyosis vulgaris (IV) und/oder zur Untersuchung, ob eine Person für die Entwicklung von IV prädisponiert ist, wobei das Verfahren den folgenden Schritt aufweist:
Nachweis in vitro, ob in dem FLG-Gen einer Nucleinsäureprobe von einer Person eine Mutation anwesend oder nicht anwesend ist, die zu einem Funktionsverlust oder teilweisen Funktionsverlust des durch das Filaggrin(FLG)-Gen codierten Filaggrin(FLG)-Proteins führen würde.

2. Test nach Anspruch 1, wobei der Test angewandt wird, um außerdem zu ermitteln, ob oder nicht eine Person wahrscheinlich für atopische Dermatitis, Asthma oder Allergien prädisponiert ist oder daran leidet, wie z. B. eine Kontaktallergie und Nahrungsmittelallergien.

3. Genetischer Test auf atopische Dermatitis, Asthma und/oder Allergien, der den folgenden Schritt aufweist:
Nachweis in vitro, ob in dem FLG-Gen einer Nucleinsäureprobe von einer Person eine Mutation anwesend ist oder nicht, die zu einem Funktionsverlust oder teilweisen Funktionsverlust des durch das FLG-Gen codierten FLG-Proteins führen würde.

4. Test nach einem der Ansprüche 1 bis 3, wobei die Mutation(en) aus der Gruppe ausgewählt ist (sind), die aus R501X, 2282del4, 3702delG, R2447X (Sequenzwiederholung 7), S3247X (Sequenzwiederholung 9), R1474X (Sequenzwiederholung 4), Q3683X (Sequenzwiederholung 10), 11029delCA (Sequenzwiederholung 10), E2422X (Sequenzwiederholung 6), 5360delG (Sequenzwiederholung 5), 7267delCA (Sequenzwiederholung 7), 11033del4 (Sequenzwiederholung 10), 6867delAG (Sequenzwiederholung 6), 3321delA (Sequenzwiederholung 2) und S2554X (Sequenzwiederholung 7) besteht.

5. Test nach einem der vorstehenden Ansprüche, wobei die Mutation(en) durch quantitative oder semiquantitative PCR, einschließlich Echtzeit-PCR, Nucleinsäure-Sequenzierung, Hybridisierungsuntersuchungen und/oder Restriktionsfragmentlängenpolymorphismus (RFLP)-Analysetechniken nachgewiesen wird (werden).

6. Verwendung eines als SEQ ID Nummern 1 - 545 identifizierten Oligonucleotids in einem Test nach einem der vorstehenden Ansprüche.

7. Verwendung eines Oligonucleotids, in dessen 3'-Position die 3'-Base aus einer der SEQ ID Nummern 1 - 545 identifiziert wird und dessen Länge etwa 12 - 50 Nucleotide beträgt, in einem Test nach einem der Ansprüche 1-5.

8. Binder oder Antikörper, der spezifisch an das Peptid binden kann, das als Ergebnis der 2282de14-Mutation in dem Filaggrin-Gen erzeugt wird, wobei der Binder das native Filaggrin-Peptid nicht binden kann.

9. Verwendung eines Binders oder Antikörpers nach Anspruch 8 zum Nachweis des Peptids, das als Ergebnis der 2282de14-Mutation in dem Filaggrin-Gen erzeugt wird.

10. Verfahren zum Nachweis eines mutierten Peptids, das als Ergebnis der 2284de14-Mutation in dem Filaggrin-Gen erzeugt wird, wobei das Verfahren die folgenden Schritte aufweist:
Nachweis in vitro, ob das von dem mutierten FLG-Gen, das 2284de14 aufweist, exprimierte mutierte Peptid in einer Probe anwesend ist oder nicht, unter Verwendung des Binders oder Antikörpers nach Anspruch 8.

11. Transgenes nichthumanes Tier, das in einer oder beiden Kopien des FLG-Gens eine oder mehrere Mutationen besitzt, die zu einem Funktionsverlust oder teilweisen Funktionsverlust des durch das FLG-Gen codierten FLG-Proteins führen würden, wobei die eine oder die mehreren Mutationen aus der Gruppe ausgewählt sind, die aus R501X, 2282de14, 3702delG, R2447X (Sequenzwiederholung 7), S3247X (Sequenzwiederholung 9), R1474X (Sequenzwiederholung 4), Q3683X (Sequenzwiederholung 10), 11029delCA (Sequenzwiederholung 10), E2422X (Sequenzwiederholung 6), 5360delG (Sequenzwiederholung 5), 7267delCA (Sequenzwiederholung 7), 11033del4 (Sequenzwiederholung 10), 6867delAG (Sequenzwiederholung 6), 3321delA (Sequenzwiederholung 2) und S2554X (Sequenzwiederholung 7) besteht.

12. In-vivo-Assay zur Identifikation eines Wirkstoffs, der bei der Behandlung oder Verhütung von IV, atopischer Dermatitis, Asthma und/oder mit der Expression von mutiertem FLG assoziierten Allergien verwendbar ist, wobei der Assay die folgenden Schritte aufweist: Verabreichen eines Testwirkstoffs an das transgene Tier nach Anspruch 11 und Messen oder Ermitteln, ob der Wirkstoff mindestens ein Anzeichen oder Symptom der Erkrankung/des Leidens vermindert oder hemmt, was darauf schließen lässt, dass der Testwirkstoff die Erkrankung/das Leiden behandeln oder verhüten kann.

## Revendications

1. Test génétique de diagnostic d'ichtyose vulgaire (IV) et/ou de dépistage de la prédisposition d'un sujet à développer IV, comprenant les étapes consistant à:
détecter *in vitro* si une mutation, qui conduirait à une perte de fonction ou à une perte partielle de fonction de la protéine filaggrine (FLG) codée par le gène de la filaggrine (FLG), est présente ou non dans le gène de la FLG d'un échantillon d'acide nucléique d'un sujet.

2. Test selon la revendication 1, où le test est utilisé aussi pour détecter si un sujet est susceptible ou non d'être prédisposé à/ou de souffrir de dermatite atopique, d'asthme ou d'allergies telles qu'une allergie du type de contact et des allergies alimentaires.

3. Test génétique pour la dermatite atopique, l'asthme et/ou des allergies, comprenant l'étape consistant à:
détecter *in vitro* si une mutation, qui pourrait conduire à une perte de fonction ou à une perte partielle de fonction de la protéine FLG codée par le gène de la FLG, est présente ou non dans le gène de la FLG d'un échantillon d'acide nucléique d'un sujet.

4. Test selon l'une quelconque des revendications 1 à 3, dans lequel la/les mutation(s) est/sont sélectionnée(s) parmi le groupe consistant en R501X, 2282de14, 3702delG, R2447X (répétition 7), S3247X (répétition 9), R1474X (répétition 4), Q3683X (répétition 10), 11029delCA (répétition 10), E2422X (répétition 6), 5360delG (répétition 5), 7267delCA (répétition 7), 11033del4 (répétition 10), 6867delAG (répétition 6), 3321delA (répétition 2) et S2554X (répétition 7).

5. Test selon l'une quelconque des revendications précédentes, dans lequel la/les mutation(s) est/sont détectée(s) par PCR quantitative ou semi-quantitative, y compris PCR en temps réel, séquençage d'acides nucléiques, études d'hybridation et/ou techniques d'analyse du polymorphisme de la longueur des fragments de restriction (RFLP).

6. Utilisation d'un oligonucléotide identifié en tant que SEQ ID NO: 1 - 545 dans un test selon l'une quelconque des revendications précédentes.

7. Utilisation d'un oligonucléotide qui ayant en sa position 3' la base en 3' identifiée parmi l'une quelconque des SEQ ID NO: 1- 545 et qui fait approximativement 12 - 50 nucléotides de long, dans un test selon l'une quelconque des revendications 1-5.

8. Agent de liaison ou anticorps capable de se lier spécifiquement au peptide produit comme un résultat de la mutation 2282de14 dans le gène de la filaggrine, où l'agent de liaison n'est pas capable de se lier au peptide natif de la filaggrine.

9. Utilisation d'un agent de liaison ou d'un anticorps selon la revendication 8, pour détecter le peptide produit comme un résultat de la mutation 2282de14 dans le gène de la filaggrine.

10. Procédé de détection d'un peptide mutant produit comme un résultat de la mutation 2284de14 dans le gène de la filaggrine, comprenant les étapes consistant à:
détecter *in vitro* si le peptide mutant exprimé du gène FLG mutant comprenant 2284de14, est présent ou non dans un échantillon, en utilisant l'agent de liaison ou l'anticorps selon la revendication 8.

11. Animal transgénique non humain qui possède une ou plusieurs mutations dans une copie du gène de la FLG ou dans les deux, qui pourraient conduire à une perte ou à une perte partielle de la fonction de la protéine FLG codée par le gène de la FLG, où l'une ou les plusieurs mutations sont sélectionnées parmi le groupe consistant en R501X, 2282del4, 3702delG, R2447X (répétition 7), S3247X (répétition 9), R1474X (répétition 4), Q3683X (répétition 10), 11029delCA (répétion 10), E2422X (répétition 6), 5360delG (répétition 5), 7267delCA (répétition 7), 11033del4 (répétition 10), 6867delAG (répétition 6), 3321delA (répétition 2) et S2554X (répétition 7).

12. Essai *in vitro* d'identification d'un agent qui est utile dans le traitement ou la prévention d'IV, de la dermatite atopique, de l'asthme et/ou d'allergies associées à l'expression de FLG mutant, comprenant les étapes consistant à administrer un agent de test à l'animal transgénique selon la revendication 11; et à mesurer ou à déterminer si l'agent réduit ou inhibe au moins un signe ou un symptôme de ladite maladie/condition, ce qui est indicatif du fait que l'agent de test est capable de traiter ou de prévenir ladite maladie/condition.


### SEQUENCE LISTING

<110> The University court of The university of Dundee McLean, Irwin smith, Frances J D
<120> Filaggrin
<130> P14417GB
<160> 558
<170> PatentIn version 3.1
<210> 1
   <211> 22
   <212> DNA
   <213> Artificial Sequence FILF3
<400> 1
   gctgataatg tgattctgtc tg 22
<210> 2
   <211> 22
   <212> DNA
   <213> Artificial Sequence H1F2
<400> 2
   gccacatatt acacaatcca gg 22
<210> 3
   <211> 19
   <212> DNA
   <213> Artificial Sequence H1F3
<400> 3
   cacggaaagg ctgggctga 19
<210> 4
   <211> 20
   <212> DNA
   <213> Artificial Sequence H1F4
<400> 4
   ggtgagcact catgaacagc 20
<210> 5
   <211> 20
   <212> DNA
   <213> Artificial Sequence H1F5
<400> 5
   ggtgagcact catgaacagt 20
<210> 6
   <211> 21
   <212> DNA
   <213> Artificial Sequence LR3F
<400> 6
   cctctgtgac ttccctctgt a 21
<210> 7
   <211> 20
   <212> DNA
   <213> Artificial Sequence LR2F
<400> 7
   gtcacttacc ccatcaaatc 20
<210> 8
   <211> 20
   <212> DNA
   <213> Artificial Sequence LR1R
<400> 8
   ccaccaaact aatgaaatac 20
<210> 9
   <211> 21
   <212> DNA
   <213> Artificial Sequence 501.F1
<400> 9
   actggaggaa gacaaggatc g 21
<210> 10
   <211> 18
   <212> DNA .
   <213> Artificial Sequence 501.R1
<400> 10
   ccctcttggg acgctgaa 18
<210> 11
   <211> 19
   <212> DNA
   <213> Artificial Sequence 501.F2
<400> 11
   tggaggaaga caaggatcg 19
<210> 12
   <211> 17
   <212> DNA
   <213> Artificial Sequence 501.R2
<400> 12
   cctcttggga cgctgaa 17
<210> 13
   <211> 19
   <212> DNA
   <213> Artificial Sequence DEL4.R1
<400> 13
   gtggctctgc tgatggtga 19
<210> 14
   <211> 22
   <212> DNA
   <213> Artificial Sequence DEL4.F1
<400> 14
   cgtcacacac agaattcctc ta 22
<210> 15
   <211> 16
   <212> DNA
   <213> Artificial Sequence DEL4.F2
<400> 15
   tcccgccacc agctcc 16
<210> 16
   <211> 21
   <212> DNA
   <213> Artificial Sequence DELG.F1
<400> 16
   gcaagcagac aaactcgtaa g 21
<210> 17
   <211> 28
   <212> DNA
   <213> Artificial Sequence DELG.R1
<400> 17
   gtttcttctc ggagtcgtct gagtgtct 28
<210> 18
   <211> 27
   <212> DNA
   <213> Artificial Sequence DELG.R2
<400> 18
   gtttcttcag acaacctctc ggagtcg 27
<210> 19
   <211> 21
   <212> DNA
   <213> Artificial Sequence FILr0.F1
<400> 19
   gggaaaaggc atgaatctag t 21
<210> 20
   <211> 20
   <212> DNA
   <213> Artificial Sequence RPT0 P1F
<400> 20
   cagtgaggga cattcagaaa 20
<210> 21
   <211> 20
   <212> DNA
   <213> Artificial Sequence RPT0 P2F
<400> 21
   cagaaaactc agacacacaa 20
<210> 22
   <211> 20
   <212> DNA
   <213> Artificial Sequence RPT0 P3F
<400> 22
   ggccacggaa aggctgggct 20
<210> 23
   <211> 20
   <212> DNA
   <213> Artificial Sequence RPT0 P4F
<400> 23
   gccacggaaa ggctgggctg 20
<210> 24
   <211> 20
   <212> DNA
   <213> Artificial Sequence RPT0 P5F
<400> 24
   ccacggaaag gctgggctga 20
<210> 25
   <211> 20
   <212> DNA
   <213> Artificial Sequence RPT0 P6F
<400> 25
   cacggaaagg ctgggctgag 20
<210> 26
   <211> 20
   <212> DNA
   <213> Artificial Sequence RPT0 P7F
<400> 26
   acggaaaggc tgggctgaga 20
<210> 27
   <211> 20
   <212> DNA
   <213> Artificial Sequence RPT0 P8F
<400> 27
   ggccacggaa aggctaggct 20
<210> 28
   <211> 20
   <212> DNA
   <213> Artificial Sequence RPT0 P9F
<400> 28
   gccacggaaa ggctaggctg 20
<210> 29
   <211> 20
   <212> DNA
   <213> Artificial Sequence RPT0 P10F
<400> 29
   ccacggaaag gctaggctga 20
<210> 30
   <211> 20
   <212> DNA
   <213> Artificial Sequence RPT0 P11F
<400> 30
   cacggaaagg ctaggctgag 20
<210> 31
   <211> 20
   <212> DNA
   <213> Artificial Sequence RPT0 P12F
<400> 31
   acggaaaggc taggctgaga 20
<210> 32
   <211> 20
   <212> DNA
   <213> Artificial sequence RPT0 P13F
<220>
   <221> misc_feature
   <222> (1)..(20)
   <223> x = Inosine
<400> 32
   ggccacggaa aggctnggct 20
<210> 33
   <211> 20
   <212> DNA
   <213> Artificial Sequence RPT0 P14F
<220>
   <221> misc_feature
   <222> (1)..(20)
   <223> n = Inosine
<400> 33
   gccacggaaa ggctnggctg 20
<210> 34
   <211> 20
   <212> DNA
   <213> Artificial Sequence RPT0 P15F
<220>
   <221> misc_feature
   <222> (1)..(20)
   <223> n= Inosine
<400> 34
   ccacggaaag gctnggctga 20
<210> 35
   <211> 20
   <212> DNA
   <213> Artificial Sequence RPT0 P16F
<220>
   <221> misc_feature
   <222> (1)..(20)
   <223> n = Inosine
<400> 35
   cacggaaagg ctnggctgag 20
<210> 36
   <211> 20
   <212> DNA
   <213> Artificial Sequence RPT0 P17F
<220>
   <221> misc_feature
   <222> (1)..(20)
   <223> n = Inosine
<400> 36
   acggaaaggc tnggctgaga 20
<210> 37
   <211> 20
   <212> DNA
   <213> Artificial Sequence RPT0 P18F
<400> 37
   acgtggccgg tcaggggaac 20
<210> 38
   <211> 20
   <212> DNA
   <213> Artificial Sequence RPT0 P19F
<400> 38
   tctggacgtt cagggtcttc 20
<210> 39
   <211> 20
   <212> DNA
   <213> Artificial Sequence RPT0 P20F
<400> 39
   ggtgagcact catgaacagc 20
<210> 40
   <211> 20
   <212> DNA
   <213> Artificial Sequence RPT0 P21F
<400> 40
   ggtgagcact catgaacagt 20
<210> 41
   <211> 22
   <212> DNA
   <213> Artificial Sequence FILr1.F1
<400> 41
   caggccatgg acaggctggt ca 22
<210> 42
   <211> 20
   <212> DNA
   <213> Artificial Sequence RPT1 P1R
<400> 42
   cgtccatggg cagagtcagg. 20
<210> 43
   <211> 20
   <212> DNA
   <213> Artificial Sequence RPT1 P2R
<400> 43
   cgtccatggg cagagtcaga 20

<210> 44
   <211> 20
   <212> DNA
   <213> Artificial Sequence RPT1 P3F
<400> 44
   gcactcatga acagcctgac 20
<210> 45
   <211> 20
   <212> DNA
   <213> Artificial Sequence RPT1 P4F
<400> 45
   ctggaggaag acaaggatcg 20
<210> 46
   <211> 20
   <212> DNA
   <213> Artificial Sequence RPT1 P5R
<400> 46
   ctcgtgcctg ctcgtggtgc 20
<210> 47
   <211> 21
   <212> DNA
   <213> Artificial Sequence RPT1 P6F
<400> 47
   gcacgagaca gctccaggca t 21
<210> 48
   <211> 20
   <212> DNA
   <213> Artificial Sequence RPT1 P7R
<400> 48
   gaccctcttg ggacgctgaa 20
<210> 49
   <211> 21
   <212> DNA
   <213> Artificial Sequence RPT1 P8F
<400> 49
   cccaccacga gcaatcggta a 21
<210> 50
   <211> 21
   <212> DNA
   <213> Artificial Sequence RPT1 P9R
<400> 50
   acctgagtgt ccagacctat t 21
<210> 51
   <211> 21
   <212> DNA
   <213> Artificial Sequence RPT1P10F
<400> 51
   aataggtctg gacactcagg t 21
<210> 52
   <211> 20
   <212> DNA
   <213> Artificial Sequence RPT1P11R
<400> 52
   tggtgtggct gtgatgggaa 20
<210> 53
   <211> 20
   <212> DNA
   <213> Artificial Sequence RPT1P12F
<400> 53
   ggaacaatca ggagacggca 20
<210> 54
   <211> 20
   <212> DNA
   <213> Artificial Sequence RPT1P13R
<400> 54
   cgtgaccctg agtgcctggt 20
<210> 55
   <211> 20
   <212> DNA
   <213> Artificial Sequence RPT1P14F
<400> 55
   catcggggcc caggacaagt 20
<210> 56
   <211> 20
   <212> DNA
   <213> Artificial Sequence RPT1P15R
<400> 56
   cactggatcc ctggttccta 20
<210> 57
   <211> 18
   <212> DNA
   <213> Artificial Sequence HR1B
<400> 57
   ctgcagacag ctccagaa 18
<210> 58
   <211> 20
   <212> DNA
   <213> Artificial Sequence RPT1P16R
<400> 58
   gtgtgacgag tgcctgattt 20
<210> 59
   <211> 18
   <212> DNA
   <213> Artificial Sequence HR1A
<400> 59
   acacacagaa ttcctcta 18
<210> 60
   <211> 20
   <212> DNA
   <213> Artificial Sequence RPT1P17R
<400> 60
   gatgacgcag cctgtccact 20
<210> 61
   <211> 20
   <212> DNA
   <213> Artificial Sequence RPT1 P18F
<400> 61
   caggccatgg acaggctggt 20
<210> 62
   <211> 21
   <212> DNA
   <213> Artificial Sequence RPT1 P19F
<400> 62
   aggccatgga caggctggtc a 21
<210> 63
   <211> 24
   <212> DNA
   <213> Artificial Sequence RPT1P20R
<400> 63
   actcttggtg gctctgctga tggt 24
<210> 64
   <211> 24
   <212> DNA
   <213> Artificial Sequence RPT1P21R
<400> 64
   tcttggtggc tctgctgatg gtga 24
<210> 65
   <211> 20
   <212> DNA
   <213> Artificial Sequence RPT1 P22F
<400> 65
   ccggtcaggg gaaaggtctc 20
<210> 66
   <211> 20
   <212> DNA
   <213> Artificial Sequence RPT1 P23R
<400> 66
   aggaaagacc ctgaacgtcg 20
<210> 67
   <211> 20
   <212> DNA
   <213> Artificial Sequence RPT1 P24F
<400> 67
   ctaccaggtg agcactcata 20
<210> 68
   <211> 19
   <212> DNA
   <213> Artificial Sequence RPT2P20F
<400> 68
   ccagacaatc aggaactcc 19
<210> 69
   <211> 21
   <212> DNA
   <213> Artificial Sequence RPT2P20F
<400> 69
   gaagtctctg cgtgaggagt t 21
<210> 70
   <211> 21
   <212> DNA
   <213> Artificial Sequence DelA.F1
<400> 70
   agtgagggac attcagagga g 21
<210> 71
   <211> 28
   <212> DNA
   <213> Artificial Sequence GS-DelA.F1
<400> 71
   gtttcttagt gagggacatt cagaggag 28
<210> 72
   <211> 20
   <212> DNA
   <213> Artificial Sequence DelA.F2
<400> 72
   gtgtcaggcc atggacagga 20
<210> 73
   <211> 27
   <212> DNA
   <213> Artificial Sequence GS-DelA.F2
<400> 73
   gtttcttgtg tcaggccatg gacagga 27
<210> 74
   <211> 21
   <212> DNA
   <213> Artificial Sequence FAMdelA.R
<220>
   <221> misc_feature
   <222> (1)..(21)
   <223> carboxyfluorescein labelled adenine
<400> 74
   ntgagtgctc acctggtaga t 21
<210> 75
   <211> 19
   <212> DNA
   <213> Artificial Sequence RPT2 P1R
<400> 75
   gggaggactc agactgttt 19
<210> 76
   <211> 20
   <212> DNA
   <213> Artificial Sequence RPT2 P2F
<400> 76
   catgagcagg cacgagacaa 20
<210> 77
   <211> 20
   <212> DNA
   <213> Artificial Sequence RPT2 P3R
<400> 77
   ggatgctgag tgcctggagt 20
<210> 78
   <211> 20
   <212> DNA
   <213> Artificial Sequence RPT2 P4F
<400> 78
   ggcactcagc atcccaagat 20
<210> 79
   <211> 20
   <212> DNA
   <213> Artificial Sequence RPT2 P5R
<400> 79
   cacgaatggt gtcctgacca 20
<210> 80
   <211> 20
   <212> DNA
   <213> Artificial Sequence RPT2 P6F
<400> 80
   acgtaatgag gaacaatcaa 20
<210> 81
   <211> 20
   <212> DNA
   <213> Artificial Sequence RPT2 P7R
<400> 81
   gagtgcctgg agccgtctct 20
<210> 82
   <211> 21
   <212> DNA
   <213> Artificial Sequence RPT 2 P8F
<400> 82
   gacagctcca gacaatcagg a 21
<210> 83
   <211> 20
   <212> DNA
   <213> Artificial Sequence RPT2 P9F
<400> 83
   tccagacaat caggaactcc 20
<210> 84
   <211> 20
   <212> DNA
   <213> Artificial Sequence RPT2 P10R
<400> 84
   aagtctctgc gtgaggagtt 20
<210> 85
   <211> 20
   <212> DNA
   <213> Artificial Sequence RPT2 P11R
<400> 85
   agaggaagtc tctgcgtgag 20
<210> 86
   <211> 20
   <212> DNA
   <213> Artificial Sequence RPT2 P12F
<400> 86
   atagtgaggg acattcagag 20
<210> 87
   <211> 20
   <212> DNA
   <213> Artificial Sequence RPT2 P13R
<400> 87
   ctgactgtgt gtctgactcc 20
<210> 88
   <211> 19
   <212> DNA
   <213> Artificial Sequence RPT2 P14F
<400> 88
   tgtcaggcca tggacagga 19
<210> 89
   <211> 20
   <212> DNA
   <213> Artificial Sequence RPT2 P15R
<400> 89
   gctctgctga tggggcccat 20
<210> 90
   <211> 20
   <212> DNA
   <213> Artificial Sequence RPT2 P16F
<400> 90
   ccaagagtcc gcacgtgact 20
<210> 91
   <211> 21
   <212> DNA
   <213> Artificial Sequence RPT2 P17R
<400> 91
   tccagacctt ccccctgacc a 21

<210> 92
   <211> 21
   <212> DNA
   <213> Artificial Sequence RPT2 P18F
<400> 92
   ctggacgttc agggtctttc a 21
<210> 93
   <211> 20
   <212> DNA
   <213> Artificial Sequence RPT 2 P19R
<400> 93
   tgagtgctca cctggtagat 20
<210> 94
   <211> 21
   <212> DNA
   <213> Artificial Sequence FILr3.F1
<400> 94
   gggtcaggac accattcgtg c 21
<210> 95
   <211> 21
   <212> DNA
   <213> Artificial Sequence FILr3.F2
<400> 95
   gcaagcagac aaactcgtaa g 21
<210> 96
   <211> 21
   <212> DNA
   <213> Artificial Sequence FAMDelG.F1
<220>
   <221> misc_feature
   <222> (1)..(21)
   <223> Carboxyfluorescein labelled guanine
<400> 96
   ncaagcagac aaactcgtaa g 21
<210> 97
   <211> 22
   <212> DNA
   <213> Artificial Sequence FILDR
<400> 97
   gaatgtccct cactgttagt ga 22
<210> 98
   <211> 21
   <212> DNA
   <213> Artificial Sequence R1474X.F
<400> 98
   ctttcctcta ccaggtgagc t 21
<210> 99
   <211> 20
   <212> DNA
   <213> Artificial Sequence RPT3 P1F
<400> 99
   ggtcaggaca ccattcgtgc 20
<210> 100
   <211> 20
   <212> DNA
   <213> Artificial Sequence RPT3 P2R
<400> 100
   tctccttgac cccgggtgtg 20
<210> 101
   <211> 20
   <212> DNA
   <213> Artificial Sequence RPT3 P3F
<400> 101
   gtgcacaccc ggggtcaagg 20
<210> 102
   <211> 20
   <212> DNA
   <213> Artificial Sequence RPT3 P4R
<400> 102
   atccctgcct tcctcctctc 20
<210> 103
   <211> 20
   <212> DNA
   <213> Artificial Sequence RPT3 P5F
<400> 103
   caagcagaca aactcgtaag 20
<210> 104
   <211> 20
   <212> DNA
   <213> Artificial Sequence RPT3 P6R
<400> 104
   cgtctcctga ttgtttgtcc 20
<210> 105
   <211> 20
   <212> DNA
   <213> Artificial Sequence RPT3 P7F
<400> 105
   gtcacgtcac catgaagctg 20
<210> 106
   <211> 20
   <212> DNA
   <213> Artificial Sequence RPT3 P8R
<400> 106
   gagctgtcag cccaagaggc 20
<210> 107
   <211> 20
   <212> DNA
   <213> Artificial Sequence RPT3 P9F
<400> 107
   ctagacactc acaggtggga 20
<210> 108
   <211> 20
   <212> DNA
   <213> Artificial Sequence RPT3 P10R
<400> 108
   accccgatga ttgttcctgt 20
<210> 109
   <211> 21
   <212> DNA
   <213> Artificial Sequence RPT3P10Ra
<400> 109
   gaccccgatg attgttcctg t 21
<210> 110
   <211> 20
   <212> DNA
   <213> Artificial Sequence RPT3 P11F
<400> 110
   cactcacagg tgggacagga 20
<210> 111
   <211> 20
   <212> DNA
   <213> Artificial Sequence RPT3 P12R
<400> 111
   cctggacccc gatgattgtt 20
<210> 112
   <211> 20
   <212> DNA
   <213> Artificial Sequence RPT3 P15F
<400> 112
   ccaggacagt gacagtgaga 20
<210> 113
   <211> 20
   <212> DNA
   <213> Artificial Sequence RPT3 P16R
<400> 113
   tcggagtcgt ctgagtgtct 20
<210> 114
   <211> 20
   <212> DNA
   <213> Artificial Sequence RPT3 P17F
<400> 114
   acagtgagag acactcagac 20
<210> 115
   <211> 20
   <212> DNA
   <213> Artificial Sequence RPT3 P18R
<400> 115
   cagacaacct ctcggagtcg 20
<210> 116
   <211> 20
   <212> DNA
   <213> Artificial Sequence RPT3 P18R
<400> 116
   agagacactc agacgactcc 20
<210> 117
   <211> 20
   <212> DNA
   <213> Artificial Sequence RPT3 P20R
<400> 117
   cagacccaga caacctctcg 20
<210> 118
   <211> 20
   <212> DNA
   <213> Artificial Sequence RPT3 P21F
<400> 118
   tcagacgact ccgagaggtt 20
<210> 119
   <211> 20
   <212> DNA
   <213> Artificial Sequence RPT3 P22R
<400> 119
   tctggaagca gacccagaca 20
<210> 120
   <211> 20
   <212> DNA
   <213> Artificial Sequence
<400> 120
   cagaaaccat catggatctt 20
<210> 121
   <211> 20
   <212> DNA
   <213> Artificial Sequence RPT3 P24R
<400> 121
   tctcttgact gctcccgaga 20
<210> 122
   <211> 20
   <212> DNA
   <213> Artificial Sequence RPT3 P25F
<400> 122
   ggctccagac accctgggtt 20
<210> 123
   <211> 21
   <212> DNA
   <213> Artificial Sequence RPT3 P26R
<400> 123
   tggctctgtc ttcttgatgg a 21
<210> 124
   <211> 20
   <212> DNA
   <213> Artificial Sequence RPT3 P27F
<400> 124
   cacacagagt cttcctctca 20
<210> 125
   <211> 20
   <212> DNA
   <213> Artificial Sequence RPT3 P29F
<400> 125 20
   ggatgacaca gcctgtccat 20
<210> 126
   <211> 20
   <212> DNA
   <213> Artificial Sequence
<400> 126 20
   tcctctcatg gacaggctgt 20
<210> 127
   <211> 20
   <212> DNA
   <213> Artificial Sequence RPT3 P30R
<400> 127 20
   tgcctgttca tgggatgaca 20
<210> 128
   <211> 20
   <212> DNA
   <213> Artificial Sequence RPT3 P31F
<400> 128
   gggtccagtg gtagtcaggt 20
<210> 129
   <211> 20
   <212> DNA
   <213> Artificial Sequence RPT3 P32R
<400> 129 20
   atgtccctca ctgttagtga 20
<210> 130
   <211> 20
   <212> DNA
   <213> Artificial Sequence
<400> 130
   cagtggtagt caggtcacta 20
<210> 131
   <211> 20
   <212> DNA
   <213> Artificial Sequence RPT3 P34R
<400> 131
   tctgaatgtc cctcactgtt 20
<210> 132
   <211> 20
   <212> DNA
   <213> Artificial Sequence RPT3 P35F
<400> 132
   ggaaagctct ggacgttcaa 20
<210> 133
   <211> 20
   <212> DNA
   <213> Artificial Sequence RPT3 P36R
<400> 133
   acctggtaga ggaaagacct 20
<210> 134
   <211> 20
   <212> DNA
   <213> Artificial Sequence RPT3 P37F
<400> 134
   tttcctctac caggtgagct 20
<210> 135
   <211> 20
   <212> DNA
   <213> Artificial Sequence RPT3 P38R
<400> 135
   gactcagact gttcatgaga 20
<210> 136
   <211> 21
   <212> DNA
   <213> Artificial Sequence R1474X.R
<400> 136
   gagtgtccag acctatctac t 21
<210> 137
   <211> 21
   <212> DNA
   <213> Artificial Sequence RPT4.F1
<400> 137
   gacaagattc atctgtagtc g 21
<210> 138
   <211> 28
   <212> DNA
   <213> Artificial GS-RPT4.F1
<400> 138
   gtttcttgac aagattcatc tgtagtcg 28
<210> 139
   <211> 19
   <212> DNA
   <213> Artificial Sequence RPT4.F2

<400> 139
   gtagtcggag acagtggaa 19
<210> 140
   <211> 20
   <212> DNA
   <213> Artificial Sequence RPT4 P1F
<400> 140
   atgaacagtc tgagtccaca 20
<210> 141
   <211> 20
   <212> DNA
   <213> Artificial Sequence RPT4 P2R
<400> 141
   tgggtgcagt ctgtccgtgt 20
<210> 142
   <211> 20
   <212> DNA
   <213> Artificial Sequence RPT4 P3F
<400> 142
   aacagtctga gtccacacac 20
<210> 143
   <211> 20
   <212> DNA
   <213> Artificial Sequence RPT4 P4R
<400> 143
   tgctgggtgc agtctgtccg 20
<210> 144
   <211> 20
   <212> DNA
   <213> Artificial Sequence RPT4 P5F
<400> 144
   ccacacacgg acagactgca 20
<210> 145
   <211> 20
   <212> DNA
   <213> Artificial Sequence RPT4 P6R
<400> 145
   gtcttcctcc agtgctgggt 20
<210> 146
   <211> 20
   <212> DNA
   <213> Artificial Sequence RPT4 P7F
<400> 146
   ccgccatgag caggcacgaa 20
<210> 147
   <211> 20
   <212> DNA
   <213> Artificial Sequence RPT4 P8R
<400> 147
   gctgagtgcc tagagctgtt 20
<210> 148
   <211> 20
   <212> DNA
   <213> Artificial Sequence RPT4 P9F
<400> 148
   agcaggcacg aaacagctct 20
<210> 149
   <211> 20
   <212> DNA
   <213> Artificial Sequence RPT4 P10R
<400> 149 20
   cttgggatgc tgagtgccta 20
<210> 150
   <211> 20
   <212> DNA
   <213> Artificial Sequence RPT4 P11F
<400> 150 20
   aggaggaagg cagggatcct 20
<210> 151
   <211> 20
   <212> DNA
   <213> Artificial Sequence RPT4 P12R
<400> 151 20
   tctactgatt gctcgtggta 20
<210> 152
   <211> 20
   <212> DNA
   <213> Artificial sequence RPT4 P13F
<400> 152 20
   gatcctacca cgagcaatca 20
<210> 153
   <211> 20
   <212> DNA
   <213> Artificial Sequence RPT4 P14R
<400> 153
   agtgtccaga cctatctact 20
<210> 154
   <211> 20
   <212> DNA
   <213> Artificial Sequence RPT4 P15F
<400> 154
   aggtctggac actcagggta 20
<210> 155
   <211> 20
   <212> DNA
   <213> Artificial Sequence RPT4 P16R
<400> 155
   tgtggtgtgg ctgtgatggt 20
<210> 156
   <211> 20
   <212> DNA
   <213> Artificial Sequence RPT4 P17F
<400> 156
   ccatcacagc cacaccacac 20
<210> 157
   <211> 20
   <212> DNA
   <213> Artificial Sequence RPT4 P18R
<400> 157
   gcatcagacc ttccctgggg 20
<210> 158
   <211> 20
   <212> DNA
   <213> Artificial Sequence RPT4 P19F
<400> 158
   ctcccatggg cagtcaggac 20
<210> 159
   <211> 21
   <212> DNA
   <213> Artificial Sequence RPT4 P20R
<400> 159
   ttgcctgctt gcacttctgg g 21
<210> 160
   <211> 21
   <212> DNA
   <213> Artificial Sequence RPT4 P21F
<400> 160
   ggacccagaa gtgcaagcag g 21
<210> 161
   <211> 20
   <212> DNA
   <213> Artificial Sequence RPT4 P22R
<400> 161
   gttcctcatt tcttgtttgc 20
<210> 162
   <211> 20
   <212> DNA
   <213> Artificial Sequence RPT4 P23F
<400> 162
   aagtgcaagc aggcaaacaa 20
<210> 163
   <211> 20
   <212> DNA
   <213> Artificial Sequence RPT4 P24R
<400> 163
   cctgattgtt cctcatttct 20
<210> 164
   <211> 20
   <212> DNA
   <213> Artificial Sequence RPT4 P25F
<400> 164
   agggtcacgt caccatgaac 20
<210> 165
   <211> 20
   <212> DNA
   <213> Artificial Sequence RPT4 P26R
<400> 165
   ctgccggccc gagtggaagg 20
<210> 166
   <211> 20
   <212> DNA
   <213> Artificial Sequence RPT4 P27F
<400> 166
   gaaccttcca ctcgggccgg 20
<210> 167
   <211> 20
   <212> DNA
   <213> Artificial Sequence RPT4 P28R
<400> 167
   ctgtgagtgt ctagagctgc 20
<210> 168
   <211> 20
   <212> DNA
   <213> Artificial Sequence RPT4 P29F
<400> 168
   ctcacaggtg ggccagggag 20
<210> 169
   <211> 20
   <212> DNA
   <213> Artificial Sequence RPT4 P30R
<400> 169
   gtcttggacc ccgctgattc 20
<210> 170
   <211> 21
   <212> DNA
   <213> Artificial Sequence RPT4 P31F
<400> 170
   gggagaatca gcggggtcca a 21
<210> 171
   <211> 20
   <212> DNA
   <213> Artificial Sequence RPT4 P32R
<400> 171
   tccctggcgc ctgcttgtct 20
<210> 172
   <211> 20
   <212> DNA
   <213> Artificial Sequence RPT4 P33F
<400> 172
   gccagggatc cagtgttagt 20
<210> 173
   <211> 20
   <212> DNA
   <213> Artificial Sequence RPT4 P34R
<400> 173
   cctcactgtc cctgtcctga 20
<210> 174
   <211> 20
   <212> DNA
   <213> Artificial Sequence RPT4 P35F
<400> 174
   gtcggcttcc agaaaccatt 20
<210> 175
   <211> 20
   <212> DNA
   <213> Artificial Sequence RPT4 P36R
<400> 175
   tgctcccgag cagatccata 20
<210> 176
   <211> 20
   <212> DNA
   <213> Artificial Sequence RPT4 P37F
<400> 176
   agtcaagaca tggctccagg 20
<210> 177
   <211> 20
   <212> DNA
   <213> Artificial Sequence RPT4 P38R
<400> 177
   gaaccccagg tcccatcaag 20
<210> 178
   <211> 20
   <212> DNA
   <213> Artificial Sequence RPT4 P39F
<400> 178
   gtcaagacat ggctccagga 20
<210> 179
   <211> 20
   <212> DNA
   <213> Artificial Sequence RPT4 P40R
<400> 179
   gacaggctgc atcatcccag 20
<210> 180
   <211> 20
   <212> DNA
   <213> Artificial Sequence RPT4 P41F
<400> 180
   gacaggctgc atcatcccag 20
<210> 181
   <211> 20
   <212> DNA
   <213> Artificial Sequence RPT4 P42R
<400> 181
   gacttgacct tgcctgttcc 20
<210> 182
   <211> 20
   <212> DNA
   <213> Artificial Sequence RPT4 P43F
<400> 182
   catcccagga acaggcaagg 20
<210> 183
   <211> 20
   <212> DNA
   <213> Artificial sequence RPT4 P44R
<400> 183
   gtctttctcc tggacttgac 20
<210> 184
   <211> 20
   <212> DNA
   <213> Artificial Sequence RPT4 P45F
<400> 184
   cagtcagcag acagctccac 20
<210> 185
   <211> 20
   <212> DNA
   <213> Artificial Sequence RPT4 P46R
<400> 185
   gcgcccagtg cctgagtctg 20
<210> 186
   <211> 20
   <212> DNA
   <213> Artificial Sequence RPT4 P47F
<400> 186
   gtcagcagac agctccacag 20
<210> 187
   <211> 19
   <212> DNA
   <213> Artificial Sequence RPT4 P48R
<400> 187
   tgcgcccagt gcctgagtc 19
<210> 188
   <211> 20
<212> DNA
   <213> Artificial Sequence RPT4 P49F
<400> 188
   ggcactgggc gcagacaaga 20
<210> 189
   <211> 20
   <212> DNA
   <213> Artificial Sequence RPT4 P50R
<400> 189
   gtctccgact acagatgaat 20
<210> 190
   <211> 20
   <212> DNA
   <213> Artificial sequence RPT4 P51F
<400> 190
   cgcagacaag attcatctgt 20
<210> 191
   <211> 20
   <212> DNA
   <213> Artificial Sequence RPT4 P52R
<400> 191
   gtttccactg tctccgacta 20
<210> 192
   <211> 20
   <212> DNA
   <213> Artificial sequence RPT4 P53F
<400> 192
   acaagattca tctgtagtcg 20
<210> 193
   <211> 20
   <212> DNA
   <213> Artificial sequence RPT4 P54R
<400> 193 20
   cctcggtttc cactgtctcc 20
<210> 194
   <211> 20
   <212> DNA
   <213> Artificial Sequence RPT4 P55F
<400> 194
   tgtagtcgga gacagtggaa 20
<210> 195
   <211> 20
   <212> DNA
   <213> Artificial Sequence RPT4 P56R
<400> 195
   ctaccactgg accctcggtt 20
<210> 196
   <211> 20
   <212> DNA
   <213> Artificial Sequence RPT4 P57F
<400> 196
   accgagggtc cagtggtagc 20
<210> 197
   <211> 20
   <212> DNA
   <213> Artificial Sequence RPT4 P58R
<400> 197
   cctcgctgtc actggcctgg 20
<210> 198
   <211> 20
   <212> DNA
   <213> Artificial Sequence RPT4 P59F
<400> 198
   gtagccaggc cagtgacagc 20
<210> 199
   <211> 20
   <212> DNA
   <213> Artificial Sequence RPT4 P60R
<400> 199
   actcttctga gtgtccctcg 20
<210> 200
   <211> 20
   <212> DNA
   <213> Artificial Sequence RPT4 P61F
<400> 200
   ccagtgacag cgagggacac 20
<210> 201
   <211> 20
   <212> DNA
   <213> Artificial Sequence RPT4 P62R
<400> 201
   gtgtgtctga ctcttctgag 20
<210> 202
   <211> 19
   <212> DNA
   <213> Artificial Sequence RPT5.R2
<400> 202
   cctgctcgtg gcgggatct 19
<210> 203
   <211> 19
   <212> DNA
   <213> Artificial Sequence FAMRPT5.R2
<220>
   <221> misc_feature
   <222> (1)..(19)
   <223> carboxyfluorescein labelled cytosine
<400> 203
   nctgctcgtg gcgggatct 19
<210> 204
   <211> 21
   <212> DNA
   <213> Artificial Sequence FILEF
<400> 204
   gtcccaggaa aggtctgatg t 21
<210> 205
   <211> 21
   <212> DNA
   <213> Artificial Sequence FILER
<400> 205
   acatcagacc tttcctggga c 21
<210> 206
   <211> 22
   <212> DNA
   <213> Artificial Sequence FILr5.F1
<400> 206
   catggatccc accaccagct cc 22
<210> 207
   <211> 22
   <212> DNA
   <213> Artificial Sequence RPT5.F2
<400> 207
   agacacacag tcagtgtcag ca 22
<210> 208
   <211> 22
   <212> DNA
   <213> Artificial Sequence RPT5.F3
<400> 208
   cacacagtca gtgtcagcac ag 22
<210> 209
   <211> 20
   <212> DNA
   <213> Artificial Sequence RPT5.P1F
<400> 209
   ctgagtccgc ccatggacgc 20
<210> 210
   <211> 20
   <212> DNA
   <213> Artificial Sequence RPT5.P2R
<400> 210
   ctccagtgct gggccctgtg 20
<210> 211
   <211> 20
   <212> DNA
   <213> Artificial Sequence RPT5.P3F
<400> 211
   agtccgccca tggacgcaca 20
<210> 212
   <211> 20
   <212> DNA
   <213> Artificial Sequence RPT5.P4R
<400> 212
   ttcctccagt gctgggccct 20
<210> 213
   <211> 20
   <212> DNA
   <213> Artificial Sequence RPT5.P5F
<400> 213
   cagcactgga ggaagacaaa 20
<210> 214
   <211> 20
   <212> DNA
   <213> Artificial Sequence RPT5.P6R
<400> 214
   gcctgctcgt ggcgggatct 20
<210> 215
   <211> 20
   <212> DNA
   <213> Artificial Sequence RPT5.P7F
<400> 215
   acaccattcg tggacaccca 20
<210> 216
   <211> 20
   <212> DNA
   <213> Artificial Sequence RPT5.P8R
<400> 216
   ttcctcctct gcttgaccct 20
<210> 217
   <211> 20
   <212> DNA
   <213> Artificial Sequence RPT5.P9F
<400> 217
   gaaggcaggg atcccactat 20
<210> 218
   <211> 20
   <212> DNA
   <213> Artificial Sequence RPT5.P10R
<400> 218
   atctatctac cgattgctca 20
<210> 219
   <211> 20
   <212> DNA
   <213> Artificial Sequence RPT5.P11F
<400> 219
   atgagcaatc ggtagatagt 20
<210> 220
   <211> 20
   <212> DNA
   <213> Artificial Sequence RPT5.P12R
<400> 220
   gagaccctga gtgtccagaa 20
<210> 221
   <211> 20
   <212> DNA
   <213> Artificial Sequence RPT5.P13F
<400> 221
   ctcatcacag ccacaccacg 20
<210> 222
   <211> 20
   <212> DNA
   <213> Artificial sequence RPT5.P14R
<400> 222
   catcagacct ttcctgggac 20
<210> 223
   <211> 20
   <212> DNA
   <213> Artificial sequence RPT5.P15F
<400> 223
   agccacacca cgtcccagga 20
<210> 224
   <211> 20
   <212> DNA
   <213> Artificial Sequence RPT5.P16R
<400> 224
   acgggagaca tcagaccttt 20
<210> 225
   <211> 20
   <212> DNA
   <213> Artificial Sequence RPT5.P17F
<400> 225
   tcccaggaaa ggtctgatgt 20
<210> 226
   <211> 20
   <212> DNA
   <213> Artificial Sequence RPT5.P18R
<400> 226
   tcctgactgc ccacgggaga 20
<210> 227
   <211> 20
   <212> DNA
   <213> Artificial Sequence RPT5.P19F
<400> 227
   cagtcaggat ccagaagtgt 20
<210> 228
   <211> 20
   <212> DNA
   <213> Artificial Sequence RPT5.P20R
<400> 228
   tgtctggagc tgtctgctga 20
<210> 229
   <211> 20
   <212> DNA
   <213> Artificial Sequence RPT5.P21F
<400> 229
   agtcaggatc cagaagtgtc 20
<210> 230
   <211> 20
   <212> DNA
   <213> Artificial sequence RPT5.P22R
<400> 230
   gtgtctggag ctgtctgctg 20
<210> 231
   <211> 20
   <212> DNA
   <213> Artificial Sequence RPT5.P23F
<400> 231
   cagacaaaca cgtaatgaga 20
<210> 232
   <211> 20
   <212> DNA
   <213> Artificial Sequence
<400> 232
   gagccgtctc ctgattgttt 20
<210> 233
   <211> 20
   <212> DNA
   <213> Artificial Sequence RPT5.P25F
<400> 233
   ccgacagctc tagacactcg 20
<210> 234
   <211> 20
   <212> DNA
   <213> Artificial Sequence RPT5.P26R
<400> 234
   attgtccctg gcccacctgc 20
<210> 235
   <211> 20
   <212> DNA
   <213> Artificial sequence RPT5.P27F
<400> 235
   tgggccaggg acaatcatca 20
<210> 236
   <211> 20
   <212> DNA
   <213> Artificial Sequence RPT5.P28R
<400> 236 20
   tcctgcttgt cctgggccct 20
<210> 237
   <211> 20
   <212> DNA
   <213> Artificial Sequence RPT5.P29F
<400> 237 20
   tctgcttcca gaaaccatct 20
<210> 238
   <211> 20
   <212> DNA
   <213> Artificial Sequence RPT5.P30R
<400> 238 20
   ctgctcccaa gcagatccaa 20
<210> 239
   <211> 20
   <212> DNA
   <213> Artificial Sequence RPT5.P31F
<400> 239 20
   aaaccatctt ggatctgctt 20
<210> 240
   <211> 20
   <212> DNA
   <213> Artificial Sequence RPT5.P32R
<400> 240 20
   tgtcttcgtg atgggaccca 20

<210> 241
   <211> 20
   <212> DNA
   <213> Artificial Sequence RPT5.P33F
<400> 241
   gagatggctc cagacaccct 20
<210> 242
   <211> 20
   <212> DNA
   <213> Artificial Sequence RPT5.P34R
<400> 242
   tgtcttcgtg atgggaccca 20
<210> 243
   <211> 20
   <212> DNA
   <213> Artificial Sequence RPT5.P35F
<400> 243
   agatggctcc agacaccctg 20
<210> 244
   <211> 20
   <212> DNA
   <213> Artificial Sequence RPT5.P36R
<400> 244
   ctgtcttcgt gatgggaccc 20
<210> 245
   <211> 20
   <212> DNA
   <213> Artificial Sequence RPT5.P37F
<400> 245
   aggcactcgt cacacagagt 20
<210> 246
   <211> 20
   <212> DNA
   <213> Artificial Sequence RPT5.P38R
<400> 246
   gcctgtccac gagaggaaga 20
<210> 247
   <211> 20
   <212> DNA
   <213> Artificial Sequence RPT5.P39F
<400> 247
   catggatccc accaccagct 20
<210> 248
   <211> 20
   <212> DNA
   <213> Artificial Sequence RPT5.P40R
<400> 248
   ggagctgtct gctgactgga 20
<210> 249
   <211> 20
   <212> DNA
   <213> Artificial Sequence RPT5.P41F
<400> 249
   atggatccca ccaccagctc 20
<210> 250
   <211> 20
   <212> DNA
   <213> Artificial Sequence RPT5.P42R
<400> 250
   tggagctgtc tgctgactgg 20
<210> 251
   <211> 20
   <212> DNA
   <213> Artificial Sequence RPT5.P43F
<400> 251
   tggatcccac caccagctcc 20
<210> 252
   <211> 20
   <212> DNA
   <213> Artificial sequence RPT5.P44R
<400> 252
   ctggagctgt ctgctgactg 20
<210> 253
   <211> 20
   <212> DNA
   <213> Artificial sequence RPT5.P45F
<400> 253
   gacactcagg cattgggcat 20
<210> 254
   <211> 20
   <212> DNA
   <213> Artificial Sequence RPT5.P46R
<400> 254
   ctgcagatga agcttgtcca 20
<210> 255
   <211> 20
   <212> DNA
   <213> Artificial Sequence RPT5.P47F
<400> 255 20
   acacacagtc agtgtcagca 20
<210> 256
   <211> 20
   <212> DNA
   <213> Artificial Sequence RPT5.P48R
<400> 256 20
   ggggcccagc ttttccctgt 20
<210> 257
   <211> 20
   <212> DNA
   <213> Artificial sequence RPT5.P49F
<400> 257 20
   cacagtcagt gtcagcacag 20
<210> 258
   <211> 20
   <212> DNA
   <213> Artificial Sequence RPT5.P50R
<400> 258 20
   gatggggccc agcttttccc 20
<210> 259
   <211> 20
   <212> DNA
   <213> Artificial Sequence RPT5.P51F
<400> 259 20
   cagtgtcagc acagggaaaa 20
<210> 260
   <211> 20
   <212> DNA
   <213> Artificial Sequence RPT5.P52R
<400> 260
   tctgctgatg gggcccagct 20
<210> 261
   <211> 20
   <212> DNA
   <213> Artificial Sequence
<400> 261
   gccccatcag cagagccaca 20
<210> 262
   <211> 20
   <212> DNA
   <213> Artificial Sequence RPT5.P54R
<400> 262
   tggccacgtg cggactcttt 20
<210> 263
   <211> 20
   <212> DNA
   <213> Artificial Sequence
<400> 263
   gtggccagtc aggggaaagc 20
<210> 264
   <211> 20
   <212> DNA
   <213> Artificial Sequence RPT5.P56R
<400> 264
   aagaccctga acgtccagag 20
<210> 265
   <211> 21
   <212> DNA
   <213> Artificial Sequence FILF1F
<400> 265
   gaggaagaca aggatcccat t 21
<210> 266
   <211> 22
   <212> DNA
   <213> Artificial Sequence FILFIR
<400> 266
   gctgtcttgt gcctgatcat aa 22
<210> 267
   <211> 22
   <212> DNA
   <213> Artificial Sequence FILF2F
<400> 267
   gaagtgcaag cagaaaaaca ta 22
<210> 268
   <211> 22
   <212> DNA
   <213> Artificial Sequence FILF2R
<400> 268
   ctcctgattg ttccttgtca ta 22
<210> 269
   <211> 21
   <212> DNA
   <213> Artificial Sequence RPT6.F1
<400> 269
   gaacaatcag gagatggctc t 21
<210> 270
   <211> 22
   <212> DNA
   <213> Artificial Sequence RPT6.F2
<400> 270
   ccgacagctc tagacactca ct 22
<210> 271
   <211> 27
   <212> DNA
   <213> Artificial Sequence GSFIL6867del.F
<400> 271
   gtttcttaga ggcggtctgg gtctgcg 27
<210> 272
   <211> 22
   <212> DNA
   <213> Artificial sequence FIL6867delG.R
<400> 272
   ccagaggaat tctctgcatg at 22
<210> 273
   <211> 27
   <212> DNA
   <213> Artificial sequence GSRPT6P1F
<400> 273
   gtttcttcca cacgtggccg gtcagca 27
<210> 274
   <211> 20
   <212> DNA
   <213> Artificial sequence RPT6.P1F
<400> 274
   tcatgaacag tctgagtcca 20
<210> 275
   <211> 20
   <212> DNA
   <213> Artificial sequence RPT6.P2R
<400> 275
   ggcgcagact gtccatgggt 20
<210> 276
   <211> 20
   <212> DNA
   <213> Artificial Sequence RPT6.P3F
<400> 276
   tctgagtcca cccatggaca 20
<210> 277
   <211> 20
   <212> DNA
   <213> Artificial sequence RPT6.P4R
<400> 277
   tccagtgctg ggcgcagact 20
<210> 278
   <211> 20
   <212> DNA
   <213> Artificial Sequence RPT6.P5F
<400> 278
   tgagtccacc catggacagt 20
<210> 279
   <211> 20
   <212> DNA
   <213> Artificial Sequence RPT6.P6R
<400> 279
   cctccagtgc tgggcgcaga 20
<210> 280
   <211> 20
   <212> DNA
   <213> Artificial Sequence RPT6.P7F
<400> 280
   cctccagtgc tgggcgcaga 20
<210> 281
   <211> 20
   <212> DNA
   <213> Artificial Sequence RPT6.P8R
<400> 281
   ttcctccagt gctgggcgca 20
<210> 282
   <211> 20
   <212> DNA
   <213> Artificial sequence RPT6.P9F
<400> 282
   tccacccatg gacagtctgc 20
<210> 283
   <211> 20
   <212> DNA
   <213> Artificial sequence RPT6.P10R
<400> 283
   tcttcctcca gtgctgggcg 20
<210> 284
   <211> 20
   <212> DNA
   <213> Artificial Sequence RPT6.P11F
<400> 284 20
   tcagcatccc aagaggccat 20
<210> 285
   <211> 20
   <212> DNA
   <213> Artificial Sequence RPT6.P12R
<400> 285 20
   tgtcttgtgc ctgatcataa 20
<210> 286
   <211> 20
   <212> DNA
   <213> Artificial Sequence RPT6.P13F
<400> 286 20
   cagcatccca agaggccatt 20
<210> 287
   <211> 20
   <212> DNA
   <213> Artificial Sequence RPT6.P14R
<400> 287
   ctgtcttgtg cctgatcata 20
<210> 288
   <211> 20
   <212> DNA
   <213> Artificial Sequence RPT6.P15F
<400> 288 20
   gacaaggatc ccattatgat 20
<210> 289
   <211> 20
   <212> DNA
   <213> Artificial Sequence RPT6.P16R
<400> 289
   tggagctgtc ttgtgcctga 20
<210> 290
   <211> 20
   <212> DNA
   <213> Artificial Sequence RPT6.P17F
<400> 290
   cattcgtgga cacccggggc 20
<210> 291
   <211> 20
   <212> DNA
   <213> Artificial Sequence RPT6.P18R
<400> 291
   tgtcttcctc ctctgcttgg 20
<210> 292
   <211> 20
   <212> DNA
   <213> Artificial sequence RPT6.P19F
<400> 292
   ggccaagcag aggaggaaga 20
<210> 293
   <211> 20
   <212> DNA
   <213> Artificial Sequence RPT6.P20R
<400> 293
   gctcttggtg ggacccctgt 20
<210> 294
   <211> 20
   <212> DNA
   <213> Artificial Sequence
<400> 294
   gaagacaggg gtcccaccaa 20
<210> 295
   <211> 20
   <212> DNA
   <213> Artificial Sequence RPT6.P22R
<400> 295
   acctatctac cgattgctct 20
<210> 296
   <211> 20
   <212> DNA
   <213> Artificial Sequence RPT6.P23F
<400> 296
   aagtgcaagc agaaaaacat 20
<210> 297
   <211> 20
   <212> DNA
   <213> Artificial Sequence RPT6.P24R
<400> 297
   cctgattgtt ccttgtcata 20
<210> 298
   <211> 20
   <212> DNA
   <213> Artificial Sequence RPT6.P25F
<400> 298
   agtgcaagca gaaaaacata 20
<210> 299
   <211> 20
   <212> DNA
   <213> Artificial sequence RPT6.P26R
<400> 299
   tcctgattgt tccttgtcat 20
<210> 300
   <211> 20
   <212> DNA
   <213> Artificial sequence RPT6.P27F
<400> 300
   tgcaagcaga aaaacatatg 20
<210> 301
   <211> 20
   <212> DNA
   <213> Artificial Sequence RPT6.P28R
<400> 301
   tctcctgatt gttccttgtc 20
<210> 302
   <211> 20
   <212> DNA
   <213> Artificial sequence RPT6.P29F
<400> 302
   caagcagaaa aacatatgac 20
<210> 303
   <211> 20
   <212> DNA
   <213> Artificial Sequence RPT6.P30R
<400> 303
   catctcctga ttgttccttg 20
<210> 304
   <211> 20
   <212> DNA
   <213> Artificial Sequence RPT6.P31F
<400> 304
   aagcagaaaa acatatgaca 20
<210> 305
   <211> 20
   <212> DNA
   <213> Artificial sequence RPT6.P32R
<400> 305
   ccatctcctg attgttcctt 20
<210> 306
   <211> 20
   <212> DNA
   <213> Artificial Sequence RPT6.P33F
<400> 306
   acaaggaaca atcaggagat 20
<210> 307
   <211> 20
   <212> DNA
   <213> Artificial sequence RPT6.P34R
<400> 307
   accctgagtg cctagagcca 20
<210> 308
   <211> 20
   <212> DNA
   <213> Artificial sequence RPT6.P35F
<400> 308
   aacaatcagg agatggctct 20
<210> 309
   <211> 20
   <212> DNA
   <213> Artificial Sequence RPT6.P36R
<400> 309
   gatgcgaccc tgagtgccta 20
<210> 310
   <211> 20
   <212> DNA
   <213> Artificial Sequence RPT6.P37F
<400> 310
   tctaggcact cagggtcgca 20
<210> 311
   <211> 20
   <212> DNA
   <213> Artificial Sequence RPT6.P38R
<400> 311
   agaggaagct tcatgatgat 20
<210> 312
   <211> 20
   <212> DNA
   <213> Artificial Sequence RPT6.P39F
<400> 312
   ggcactcagg gtcgcatcat 20
<210> 313
   <211> 20
   <212> DNA
   <213> Artificial Sequence RPT6.P40R
<400> 313
   cccaagagga agcttcatga 20
<210> 314
   <211> 20
   <212> DNA
   <213> Artificial Sequence RPT6.P41F
<400> 314
   tcatcatgaa gcttcctctt 20
<210> 315
   <211> 20
   <212> DNA
   <213> Artificial Sequence RPT6.P42R
<400> 315
   tgtctagagc tgtcggccca 20
<210> 316
   <211> 20
   <212> DNA
   <213> Artificial Sequence RPT6.P43F
<400> 316
   gacagctcta gacactcact 20
<210> 317
   <211> 20
   <212> DNA
   <213> Artificial Sequence RPT6.P44R
<400> 317
   tgattgtccc tggcccacca 20
<210> 318
   <211> 20
   <212> DNA
   <213> Artificial Sequence RPT6.P45F
<400> 318
   tgggccaggg acaatcatca 20
<210> 319
   <211> 20
   <212> DNA
   <213> Artificial Sequence RPT6.P46R
<400> 319
   gcctgcttgt cctgggccct 20
<210> 320
   <211> 20
   <212> DNA
   <213> Artificial Sequence RPT6.P47F
<400> 320
   gggcccagga caagcaggcc 20
<210> 321
   <211> 20
   <212> DNA
   <213> Artificial Sequence RPT6.P48R
<400> 321
   gctaacactg gatccccggg 20
<210> 322
   <211> 20
   <212> DNA
   <213> Artificial Sequence RPT6.P49F
<400> 322
   cccaggacaa gcaggccccg 20
<210> 323
   <211> 20
   <212> DNA
   <213> Artificial Sequence RPT6.P50R
<400> 323
   ctggctaaca ctggatcccc 20
<210> 324
   <211> 20
   <212> DNA
   <213> Artificial Sequence RPT6.P51F
<400> 324
   agaggcggtc tgggtctgcg 20
<210> 325
   <211> 20
   <212> DNA
   <213> Artificial Sequence RPT6.P52R
<400> 325
   atccatgatg gtttctggac 20
<210> 326
   <211> 20
   <212> DNA
   <213> Artificial Sequence RPT6.P53F
<400> 326
   tccacacgtg gccggtcagc 20
<210> 327
   <211> 20
   <212> DNA
   <213> Artificial Sequence RPT6.P54R
<400> 327
   tgaacgtcca gaccttcctg 20
<210> 328
   <211> 20
   <212> DNA
   <213> Artificial Sequence RPT6.P55F

<400> 328
   ccacacgtgg ccggtcagca 20
<210> 329
   <211> 20
   <212> DNA
   <213> Artificial Sequence RPT6.P56R
<400> 329
   ctgaacgtcc agaccttcct 20
<210> 330
   <211> 19
   <212> DNA
   <213> Artificial Sequence RPT7.R1
<400> 330
   agctgtctcg tgcctgctt 19
<210> 331
   <211> 21
   <212> DNA
   <213> Artificial Sequence R2447X.R
<400> 331
   gtcctgaccc tcttgggacg t 21
<210> 332
   <211> 21
   <212> DNA
   <213> Artificial Sequence HEXR2447X.R
<220>
   <221> misc_feature
   <222> (1)..(21)
   <223> hexachlorofluorescein labelled guanine
<400> 332
   ntcctgaccc tcttgggacg t 21
<210> 333
   <211> 19
   <212> DNA
   <213> Artificial Sequence RPT7.F2
<400> 333
   cccaggacaa gcaggaact 19
<210> 334
   <211> 21
   <212> DNA
   <213> Artificial Sequence RPT7P1R
<400> 334
   ctggctaaaa ctggatcccc a 21
<210> 335
   <211> 19
   <212> DNA
   <213> Artificial Sequence RPT7.R2
<400> 335
   ggctaaaact ggatcccca 19
<210> 336
   <211> 20
   <212> DNA
   <213> Artificial Sequence RPT7.P1F
<400> 336
   aagacaagga tcccaccaca 20
<210> 337
   <211> 20
   <212> DNA
   <213> Artificial Sequence RPT/.P2R
<400> 337
   gagctgtctc gtgcctgctt 20
<210> 338
   <211> 20
   <212> DNA
   <213> Artificial Sequence RPT7.P3F
<400> 338
   agacagctcc aggcactcaa 20
<210> 339
   <211> 20
   <212> DNA
   <213> Artificial Sequence RPT7.P4R
<400> 339
   tcctgaccct cttgggacgt 20
<210> 340
   <211> 20
   <212> DNA
   <213> Artificial Sequence RPT7.P5F
<400> 340
   gagggtcagg acaccattca 20
<210> 341
   <211> 20
   <212> DNA
   <213> Artificial sequence RPT7.P6R
<400> 341
   gcttgacccc gggtgtccat 20
<210> 342
   <211> 20
   <212> DNA
   <213> Artificial Sequence RPT7.P7F
<400> 342
   gacacccggg gtcaagcagt 20
<210> 343
   <211> 20
   <212> DNA
   <213> Artificial Sequence RPT7.P8R
<400> 343
   gggatccctg ccttcctcca 20
<210> 344
   <211> 20
   <212> DNA
   <213> Artificial Sequence RPT7.P9F
<400> 344
   ggatcccact acgagcaatt 20
<210> 345
   <211> 20
   <212> DNA
   <213> Artificial Sequence RPT7.P10R
<400> 345
   gtgtccagat ctatctacca 20
<210> 346
   <211> 20
   <212> DNA
   <213> Artificial Sequence RPT7.P11F
<400> 346
   acgagcaatt ggtagataga 20
<210> 347
   <211> 20
   <212> DNA
   <213> Artificial Sequence RPT7.P12R
<400> 347
   gagaccctga gtgtccagat 20
<210> 348
   <211> 20
   <212> DNA
   <213> Artificial Sequence RPT7.P13F
<400> 348
   ctgatgcctc ccatgggcac 20
<210> 349
   <211> 20
   <212> DNA
   <213> Artificial Sequence RPT7.P14R
<400> 349
   ttgcacttct ggatcctgag 20
<210> 350
   <211> 20
   <212> DNA
   <213> Artificial Sequence RPT7.P15F
<400> 350
   caagcagaca aactcgtaac 20
<210> 351
   <211> 20
   <212> DNA
   <213> Artificial Sequence RPT7.P16R
<400> 351
   cgtctcctga ttgttcatcg 20
<210> 352
   <211> 20
   <212> DNA
   <213> Artificial Sequence RPT7.P17F
<400> 352
   gcagacaaac tcgtaacgat 20
<210> 353
   <211> 20
   <212> DNA
   <213> Artificial Sequence
<400> 353
   agccgtctcc tgattgttca 20
<210> 354
   <211> 20
   <212> DNA
   <213> Artificial Sequence RPT7.P19F
<400> 354
   agccgtctcc tgattgttca 20
<210> 355
   <211> 20
   <212> DNA
   <213> Artificial Sequence RPT7.P20R
<400> 355
   tggcccacct gcgagtgtcc 20
<210> 356
   <211> 20
   <212> DNA
   <213> Artificial Sequence RPT7.P21F
<400> 356
   ccgacagctc tggacactcg 20
<210> 357
   <211> 20
   <212> DNA
   <213> Artificial Sequence RPT7.P22R
<400> 357
   attgtccctg gcccacctgc 20
<210> 358
   <211> 20
   <212> DNA
   <213> Artificial Sequence RPT7.P23F
<400> 358
   gcccaggaca agcaggaact 20
<210> 359
   <211> 20
   <212> DNA
   <213> Artificial Sequence RPT7.P24R
<400> 359
   tggctaaaac tggatcccca 20
<210> 360
   <211> 20
   <212> DNA
   <213> Artificial Sequence RPT7.P25F
<400> 360
   cccaggacaa gcaggaactg 20
<210> 361
   <211> 20
   <212> DNA
   <213> Artificial Sequence RPT7.P26R
<400> 361
   ctggctaaaa ctggatcccc 20
<210> 362
   <211> 20
   <212> DNA
   <213> Artificial Sequence RPT7.P27F
<400> 362
   caggaactgg ggatccagtt 20
<210> 363
   <211> 20
   <212> DNA
   <213> Artificial Sequence RPT7.P28R
<400> 363
   ctgtcactgt cctggctaaa 20
<210> 364
   <211> 20
   <212> DNA
   <213> Artificial Sequence RPT7.P29F
<400> 364
   tggatctgct caggagcagc 20
<210> 365
   <211> 20
   <212> DNA
   <213> Artificial sequence RPT7.P30R
<400> 365
   tgtctggagc catctcttag 20
<210> 366
   <211> 20
   <212> DNA
   <213> Artificial Sequence RPT7.P31F
<400> 366
   ggatctgctc aggagcagct 20
<210> 367
   <211> 20
   <212> DNA
   <213> Artificial Sequence RPT7.P32R
<400> 367
   gtgtctggag ccatctctta 20
<210> 368
   <211> 20
   <212> DNA
   <213> Artificial Sequence RPT7.P33F
<400> 368
   ggtagtcagg ccagtgacaa 20
<210> 369
   <211> 20
   <212> DNA
   <213> Artificial Sequence RPT7.P34R
<400> 369
   gtcttctgaa tgtccctcat 20
<210> 370
   <211> 20
   <212> DNA
   <213> Artificial Sequence RPT7.P35F
<400> 370
   agcccacgga caggctgggt 20
<210> 371
   <211> 20
   <212> DNA
   <213> Artificial Sequence RPT7.P36R
<400> 371
   tggtggctct gctgatggga 20
<210> 372
   <211> 20
   <212> DNA
   <213> Artificial sequence RPT7.P37F
<400> 372
   cgtggccggt caggggaaac 20
<210> 373
   <211> 20
   <212> DNA
   <213> Artificial sequence
<400> 373
   agatcctgaa tgtccagacg 20
<210> 374
   <211> 20
   <212> DNA
   <213> Artificial Sequence RPT7.P39F
<400> 374
   tcaggggaaa cgtctggaca 20
<210> 375
   <211> 20
   <212> DNA
   <213> Artificial sequence RPT7.P40R
<400> 375
   gtagaggaaa gatcctgaat 20
<210> 376
   <211> 20
   <212> DNA
   <213> Artificial Sequence RPT7.P41F
<400> 376
   aaacgtctgg acattcagga 20
<210> 377
   <211> 20
   <212> DNA
   <213> Artificial Sequence RPT7.P42R
<400> 377
   tcacctggta gaggaaagat 20
<210> 378
   <211> 21
   <212> DNA.
   <213> Artificial Sequence RPT8.F1
<400> 378
   gatggacggg gcccagcact a 21
<210> 379
   <211> 21
   <212> DNA
   <213> Artificial Sequence RPT8.R1
<400> 379
   cttcctctag tgctgggccc c 21
<210> 380
   <211> 21
   <212> DNA
   <213> Artificial Sequence RPT8P1R
<400> 380
   ctaccgaatg ctcgtggtgg t 21
<210> 381
   <211> 21
   <212> DNA
   <213> Artificial Sequence RPT8.F2
<400> 381
   cacgagcatt cggtagatag c 21
<210> 382
   <211> 21
   <212> DNA
   <213> Artificial Sequence FILRPT8.F3
<400> 382
   aggcgccagg gatccagtgt g 21
<210> 383
   <211> 22
   <212> DNA
   <213> Artificial Sequence RPT8.R4
<400> 383
   cttggtggct ctgctgatgg ga 22
<210> 384
   <211> 21
   <212> DNA
   <213> Artificial Sequence FILr8.F1
<400> 384
   gaaacgtctg gacattcagg a 21
<210> 385
   <211> 20
   <212> DNA
   <213> Artificial Sequence RPT8.P1F
<400> 385
   agtcctccca tggatggacg 20
<210> 386
   <211> 20
   <212> DNA
   <213> Artificial Sequence RPT8.P2R
<400> 386
   ttcctctagt gctgggcccc 20
<210> 387
   <211> 20
   <212> DNA
   <213> Artificial Sequence RPT8.P3F
<400> 387
   atggacgggg cccagcacta 20
<210> 388
   <211> 20
   <212> DNA
   <213> Artificial Sequence RPT8.P4R
<400> 388
   cgggatcctt gtcttcctct 20
<210> 389
   <211> 20
   <212> DNA
   <213> Artificial Sequence RPT8.P5F
<400> 389
   agaggaggaa ggcaggggta 20
<210> 390
   <211> 20
   <212> DNA
   <213> Artificial Sequence RPT8.P6R
<400> 390
   taccgaatgc tcgtggtggt 20
<210> 391
   <211> 20
   <212> DNA
   <213> Artificial Sequence RPT8.P7F
<400> 391
   aggggtacca ccacgagcat 20
<210> 392
   <211> 20
   <212> DNA
   <213> Artificial Sequence RPT8.P8R
<400> 392
   gtccagagct atctaccgaa 20
<210> 393
   <211> 20
   <212> DNA
   <213> Artificial Sequence RPT8.P9F
<400> 393
   acgagcattc ggtagatagc 20
<210> 394
   <211> 20
   <212> DNA
   <213> Artificial Sequence RPT8.P10R
<400> 394
   gggaccctga gtgtccagag 20
<210> 395
   <211> 20
   <212> DNA
   <213> Artificial Sequence RPT8.P11F
<400> 395
   tctagacact cacaggcagt 20
<210> 396
   <211> 20
   <212> DNA
   <213> Artificial Sequence RPT8.P12R
<400> 396
   cccctctgat tgtccctgga 20
<210> 397
   <211> 20
   <212> DNA
   <213> Artificial Sequence RPT8.P13F
<400> 397
   caatcagagg ggtccaggag 20
<210> 398
   <211> 20
   <212> DNA
   <213> Artificial Sequence RPT8.P14R
<400> 398
   ggatccctgg cgcctgcttc 20
<210> 399
   <211> 20
   <212> DNA
   <213> Artificial Sequence RPT8.P15F
<400> 399
   ggcgccaggg atccagtgtg 20
<210> 400
   <211> 20
   <212> DNA
   <213> Artificial Sequence RPT8.P16R
<400> 400
   cactgtcact gtcctggctc 20
<210> 401
   <211> 20
   <212> DNA
   <213> Artificial Sequence RPT8.P17F
<400> 401
   tggatctgct caggagcagc 20
<210> 402
   <211> 20
   <212> DNA
   <213> Artificial Sequence RPT8.P18R
<400> 402
   tgtctggagc catctcttag 20
<210> 403
   <211> 20
   <212> DNA
   <213> Artificial Sequence RPT8.P19F
<400> 403
   ggatctgctc aggagcagct 20
<210> 404
   <211> 20
   <212> DNA
   <213> Artificial Sequence RPT8.P20R
<400> 404
   gtgtctggag ccatctctta 20
<210> 405
   <211> 20
   <212> DNA
   <213> Artificial Sequence RPT8.P21F
<400> 405
   ggtagtcagg ccagtgacaa 20
<210> 406
   <211> 20
   <212> DNA
   <213> Artificial sequence RPT8.P22R
<400> 406
   gtcttctgaa tgtccctcat 20
<210> 407
   <211> 20
   <212> DNA
   <213> Artificial sequence RPT8.P23F
<400> 407
   agcccacgga caggctgggt 20
<210> 408
   <211> 20
   <212> DNA
   <213> Artificial sequence RPT8.P24R
<400> 408
   tggtggctct gctgatggga 20
<210> 409
   <211> 20
   <212> DNA
   <213> Artificial Sequence RPT8.P25F
<400> 409
   cgtggccggt caggggaaac 20
<210> 410
   <211> 20
   <212> DNA
   <213> Artificial Sequence RPT8.P26R
<400> 410
   agatcctgaa tgtccagacg 20
<210> 411
   <211> 20
   <212> DNA
   <213> Artificial Sequence RPT8.P27F
<400> 411
   tcaggggaaa cgtctggaca 20
<210> 412
   <211> 20
   <212> DNA
   <213> Artificial Sequence RPT8.P28R
<400> 412
   gtagaggaaa gatcctgaat 20
<210> 413
   <211> 20
   <212> DNA
   <213> Artificial Sequence RPT8.P29F
<400> 413
   aaacgtctgg acattcagga 20
<210> 414
   <211> 20
   <212> DNA
   <213> Artificial sequence RPT8.P30R
<400> 414
   tcacctggta gaggaaagat 20
<210> 415
   <211> 21
   <212> DNA
   <213> Artificial Sequence FILRPT9.F1
<400> 415
   ccaggcactc agcatcccaa t 21
<210> 416
   <211> 21
   <212> DNA
   <213> Artificial Sequence FILr9.R1
<400> 416
   acgaatggtg tcctgaccgt a 21
<210> 417
   <211> 24
   <212> DNA
   <213> Artificial Sequence S3247X.F
<400> 417
   gtaatgagga acaatcagga gaca 24
<210> 418
   <211> 22
   <212> DNA
   <213> Artificial sequence RPT9.R1
<400> 418
   gtgacactga gtgcctggag ct 22
<210> 419
   <211> 21
   <212> DNA
   <213> Artificial Sequence FILr9.F1
<400> 419
   cagaaaccat cgtggatctg t 21
<210> 420
   <211> 21
   <212> DNA
   <213> Artificial Sequence FILr9.R2
<400> 420
   ctggggtgtc tggagccgtg c 21
<210> 421
   <211> 21
   <212> DNA
   <213> Artificial sequence FILr9.F2
<400> 421
   gtcacgggca ctctgcagac c 21
<210> 422
   <211> 20
   <212> DNA
   <213> Artificial Sequence RPT9.P1F
<400> 422
   agtcctccca tggatggacg 20
<210> 423
   <211> 20
   <212> DNA
   <213> Artificial Sequence RPT9.P2R
<400> 423
   ttcctctagt gctgggcccc 20
<210> 424
   <211> 20
   <212> DNA
   <213> Artificial Sequence RPT9.P3F
<400> 424
   atggacgggg cccagcacta 20

<210> 425
   <211> 20
   <212> DNA
   <213> Artificial Sequence RPT9.P4R
<400> 425
   cgggatcctt gtcttcctct 20
<210> 426
   <211> 20
   <212> DNA
   <213> Artificial Sequence RPT9.P5F
<400> 426
   tcccgccatg agcaggcaca 20
<210> 427
   <211> 20
   <212> DNA
   <213> Artificial Sequence RPT9.P6R
<400> 427
   tgagtgcctg gagctgtctt 20
<210> 428
   <211> 20
   <212> DNA
   <213> Artificial Sequence RPT9.P7F
<400> 428
   caggcactca gcatcccaat 20
<210> 429
   <211> 20
   <212> DNA
   <213> Artificial Sequence RPT9.P8R
<400> 429
   cgaatggtgt cctgaccgta 20
<210> 430
   <211> 20
   <212> DNA
   <213> Artificial Sequence RPT9.P9F
<400> 430
   agaggaggaa ggcaggggta 20
<210> 431
   <211> 20
   <212> DNA
   <213> Artificial Sequence RPT9.P10R
<400> 431
   taccgaatgc tcgtggtggt 20
<210> 432
   <211> 20
   <212> DNA
   <213> Artificial Sequence RPT9.P11F
<400> 432
   aggggtacca ccacgagcat 20
<210> 433
   <211> 20
   <212> DNA
   <213> Artificial Sequence RPT9.P12R
<400> 433
   gtccagagct atctaccgaa 20
<210> 434
   <211> 20
   <212> DNA
   <213> Artificial Sequence RPT9.P13F
<400> 434
   acgagcattc ggtagatagc 20
<210> 435
   <211> 20
   <212> DNA
   <213> Artificial Sequence RPT9.P14R
<400> 435
   gggaccctga gtgtccagag 20
<210> 436
   <211> 20
   <212> DNA
   <213> Artificial Sequence RPT9.P15F
<400> 436
   tgaggaacaa tcaggagaca 20
<210> 437
   <211> 20
   <212> DNA
   <213> Artificial Sequence RPT9.P16
<400> 437
   gacactgagt gcctggagct 20
<210> 438
   <211> 20
   <212> DNA
   <213> Artificial Sequence RPT9.P17F
<400> 438
   gacagctcca ggcactcagt 20
<210> 439
   <211> 20
   <212> DNA
   <213> Artificial Sequence RPT9.P18R
<400> 439
   agcttcatgg tgacgtgaca 20
<210> 440
   <211> 20
   <212> DNA
   <213> Artificial Sequence RPT9.P19F
<400> 440
   tctctagaca ctcacaggca 20
<210> 441
   <211> 20
   <212> DNA
   <213> Artificial Sequence RPT9.P20R
<400> 441
   cctctgattg tccctggact 20
<210> 442
   <211> 20
   <212> DNA
   <213> Artificial Sequence RPT9.P21F
<400> 442
   tctagacact cacaggcagt 20
<210> 443
   <211> 20
   <212> DNA
   <213> Artificial Sequence RPT9.P22R
<400> 443
   cccctctgat tgtccctgga 20
<210> 444
   <211> 20
   <212> DNA
   <213> Artificial Sequence RPT9.P23F
<400> 444 20
   ggcgccaggg atccagtgtg 20
<210> 445
   <211> 20
   <212> DNA
   <213> Artificial Sequence RPT9.P24R
<400> 445 '
   cactgtcact gtcctggctc 20
<210> 446
   <211> 20
   <212> DNA
   <213> Artificial Sequence RPT9.P25F
<400> 446
   agaaaccatc gtggatctgt 20
<210> 447
   <211> 20
   <212> DNA
   <213> Artificial Sequence RPT9.P26R
<400> 447
   gtgccttgac tgctcctgaa 20
<210> 448
   <211> 20
   <212> DNA
   <213> Artificial Sequence RPT9.P27F
<400> 448
   tcacgggcac tctgcagacc 20
<210> 449
   <211> 20
   <212> DNA
   <213> Artificial Sequence RPT9.P28R
<400> 449
   gtgcctgatt gtctggagcg 20
<210> 450
   <211> 20
   <212> DNA
   <213> Artificial Sequence RPT9.P29F
<400> 450
   ctccagacac tcaggcattc 20
<210> 451
   <211> 20
   <212> DNA
   <213> Artificial Sequence RPT9.P30R
<400> 451
   gatgaagctt gtccacgcgg 20
<210> 452
   <211> 20
   <212> DNA
   <213> Artificial Sequence RPT9.P31F
<400> 452
   tccagacact caggcattcc 20
<210> 453
   <211> 20
   <212> DNA
   <213> Artificial Sequence RPT9.P32R
<400> 453
   agatgaagct tgtccacgcg 20
<210> 454
   <211> 20
   <212> DNA
   <213> Artificial Sequence RPT9.P33F
<400> 454
   gacactcagg cattccgcgt 20
<210> 455
   <211> 20
   <212> DNA
   <213> Artificial Sequence RPT9.P34R
<400> 455
   ctgcagatga agcttgtcca 20
<210> 456
   <211> 20
   <212> DNA
   <213> Artificial Sequence RPT9.P35F
<400> 456
   atctgcagtc agagacagta 20
<210> 457
   <211> 20
   <212> DNA
   <213> Artificial Sequence RPT9.P36R
<400> 457
   ccactggacc cccagtgtct 20
<210> 458
   <211> 20
   <212> DNA
   <213> Artificial Sequence RPT9.P37F
<400> 458
   agtcagagac agtagacact 20
<210> 459
   <211> 20
   <212> DNA
   <213> Artificial Sequence RPT9.P38R
<400> 459
   tgactaccac tggaccccca 20
<210> 460
   <211> 20
   <212> DNA
   <213> Artificial Sequence RPT9.P39F
<400> 460
   gtggtagtca ggccagtgat 20
<210> 461
   <211> 20
   <212> DNA
   <213> Artificial Sequence RPT9.P40R
<400> 461
   cttctgaatg tccctcacta 20
<210> 462
   <211> 19
   <212> DNA
   <213> Artificial Sequence FILr10.F1
<400> 462
   gcccatgggc ggaccagga 19
<210> 463
   <211> 19
   <212> DNA
   <213> Artificial Sequence FILJF
<400> 463
   gatggctcca ggcactcat 19
<210> 464
   <211> 20
   <212> DNA
   <213> Artificial Sequence FILr10.F2
<400> 464
   gggcccagga caagcaggaa 20
<210> 465
   <211> 21
   <212> DNA
   <213> Artificial Sequence Q3683X.F
<400> 465
   gtcagagaca gtggacaccg a 21
<210> 466
   <211> 28
   <212> DNA
   <213> Artificial Sequence 11033del4.F
<400> 466
   gtttcttgtc agagacagtg gacaccga 28
<210> 467
   <211> 21
   <212> DNA
   <213> Artificial Sequence FILr10.R1
<400> 467
   ctgaacgtcc agaccttcct g 21
<210> 468
   <211> 20
   <212> DNA
   <213> Artificial Sequence RPT10.P1F
<400> 468
   agtctgagtc tgcccatggg 20
<210> 469
   <211> 20
   <212> DNA
   <213> Artificial Sequence RPT10.P2R
<400> 469
   cagtgctggt cctggtccgc 20
<210> 470
   <211> 20
   <212> DNA
   <213> Artificial sequence RPT10.P3F
<400> 470
   gtctgcccat gggcggacca 20
<210> 471
   <211> 20
   <212> DNA
   <213> Artificial Sequence RPT10.P4R
<400> 471
   cttcgtccag tgctggtcct 20
<210> 472
   <211> 20
   <212> DNA
   <213> Artificial Sequence RPT10.P5F
<400> 472
   gaccaggacc agcactggac 20
<210> 473
   <211> 20
   <212> DNA
   <213> Artificial Sequence RPT10.P6R
<400> 473
   tggtgggatc cttgtcttcg 20
<210> 474
   <211> 20
   <212> DNA
   <213> Artificial Sequence RPT10.P7F
<400> 474
   acacccgggg tcaagcagaa 20
<210> 475
   <211> 20
   <212> DNA
   <213> Artificial Sequence RPT10.P8R
<400> 475
   tgggatccct gccttcctct 20
<210> 476
   <211> 20
   <212> DNA
   <213> Artificial Sequence RPT10.P9F
<400> 476
   agtcaggatc cagaagtgcc 20
<210> 477
   <211> 20
   <212> DNA
   <213> Artificial Sequence RPT10.P10R
<400> 477
   cattacgagt ttgtctgctg 20
<210> 478
   <211> 20
   <212> DNA
   <213> Artificial Sequence RPT10.P11F
<400> 478
   gcagacaaac tcgtaatgac 20
<210> 479
   <211> 20
   <212> DNA
   <213> Artificial Sequence RPT10.P12R
<400> 479
   agccatctcc tgattgttcg 20
<210> 480
   <211> 20
   <212> DNA
   <213> Artificial Sequence RPT10.P13F
<400> 480
   atgacgaaca atcaggagat 20
<210> 481
   <211> 20
   <212> DNA
   <213> Artificial sequence RPT10.P14R
<400> 481
   accatgagtg cctggagcca 20
<210> 482
   <211> 20
   <212> DNA
   <213> Artificial Sequence RPT10.P15F
<400> 482
   agatggctcc aggcactcat 20
<210> 483
   <211> 20
   <212> DNA
   <213> Artificial Sequence RPT10.P16R
<400> 483
   gcttcatggt gatgcgacca 20
<210> 484
   <211> 20
   <212> DNA
   <213> Artificial Sequence RPT10.P17F
<400> 484
   tccaggcact catggtcgca 20
<210> 485
   <211> 20
   <212> DNA
   <213> Artificial Sequence RPT10.P18R
<400> 485
   agtggaagct tcatggtgat 20
<210> 486
   <211> 20
   <212> DNA
   <213> Artificial Sequence RPT10.P19F
<400> 486
   atgaagcttc cactcaggcg 20
<210> 487
   <211> 20
   <212> DNA
   <213> Artificial Sequence RPT10.P20R
<400> 487
   gtgagtgtct agagctgtcc 20
<210> 488
   <211> 20
   <212> DNA
   <213> Artificial sequence RPT10.P21F
<400> 488
   cagctctaga cactcacagt 20
<210> 489
   <211> 20
   <212> DNA
   <213> Artificial Sequence RPT10.P22R
<400> 489
   gctgattgtc cctggccgga 20
<210> 490
   <211> 20
   <212> DNA
   <213> Artificial Sequence RPT10.P23F
<400> 490
   gatggctcca gacaccccac 20
<210> 491
   <211> 20
   <212> DNA
   <213> Artificial Sequence RPT10.P24R
<400> 491
   tctgtcttcg tgatgggacg 20
<210> 492
   <211> 20
   <212> DNA
   <213> Artificial Sequence RPT10.P25F
<400> 492
   ccacacgtgg ccggtcagca 20
<210> 493
   <211> 20
   <212> DNA
   <213> Artificial Sequence RPT10.P26R
<400> 493
   ctgaacgtcc agaccttcct 20
<210> 494
   <211> 20
   <212> DNA
   <213> Artificial Sequence RPT8.1.P1F
<400> 494
   tgggcagtca ggatccagac 20
<210> 495
   <211> 20
   <212> DNA
   <213> Artificial Sequence RPT8.1.P2R
<400> 495
   cgtgttgttc tgcttgcacg 20
<210> 496
   <211> 20
   <212> DNA
   <213> Artificial Sequence RPT8.1.P3F
<400> 496
   cccaggtccc atcaagaagg 20
<210> 497
   <211> 20
   <212> DNA
   <213> Artificial Sequence RPT8.1.P4R
<400> 497
   gtgcccatga ccagctctgc 20
<210> 498
   <211> 20
   <212> DNA
   <213> Artificial Sequence RPT8.2.P1F
<400> 498
   ggcactcagc atcccaagat 20
<210> 499
   <211> 20
   <212> DNA
   <213> Artificial Sequence RPT8.2.P2R
<400> 499
   cacgaatggt gtcctgacca 20
<210> 500
   <211> 20
   <212> DNA
   <213> Artificial Sequence RPT8.2.P3F
<400> 500
   aggcactcgt cacacacagg 20
<210> 501
   <211> 20
   <212> DNA
   <213> Artificial Sequence RPT8.2.P4R
<400> 501
   gcctgtccac cagaggaagc 20
<210> 502
   <211> 22
   <212> DNA
   <213> Artificial Sequence FILr10/1.F1
<400> 502
   catggatccc accaccagct cc 22
<210> 503
   <211> 20
   <212> DNA
   <213> Artificial Sequence RPT10.1.P1F
<400> 503
   agacggctcc aggcactcat 20
<210> 504
   <211> 20
   <212> DNA
   <213> Artificial Sequence RPT10.1.P2R
<400> 504
   gcttcatggt gatgcgacca 20
<210> 505
   <211> 20
   <212> DNA
   <213> Artificial Sequence RPT10.1.P3F
<400> 505
   atgaagcttc cactcaggcg 20
<210> 506
   <211> 20
   <212> DNA
   <213> Artificial Sequence RPT10.1.P4R
<400> 506
   gtgagtgtct agagctgtcc 20
<210> 507
   <211> 20
   <212> DNA
   <213> Artificial Sequence RPT10.1.P5F
<400> 507
   cagctctaga cactcacagt 20
<210> 508
   <211> 20
   <212> DNA
   <213> Artificial Sequence RPT10.1.P6R
<400> 508
   gctgattgtc cctggccgga 20
<210> 509
   <211> 20
   <212> DNA
   <213> Artificial Sequence RPT10.1.P7F
<400> 509
   gacaatcagc ggggcccagt 20
<210> 510
   <211> 20
   <212> DNA
   <213> Artificial Sequence RPT10.1.P8R
<400> 510
   atccctggtt cctgcttgta 20
<210> 511
   <211> 20
   <212> DNA
   <213> Artificial Sequence RPT10.1.P9F
<400> 511
   cgggcactct gcagacagcc 20
<210> 512
   <211> 20
   <212> DNA
   <213> Artificial Sequence RPT10.1.P10R
<400> 512
   cgagtgcctg attgtctggg 20
<210> 513
   <211> 20
   <212> DNA
   <213> Artificial Sequence RPT10.1.P11F
<400> 513
   agcagacagc tccagacacg 20
<210> 514
   <211> 20
   <212> DNA
   <213> Artificial sequence RPT10.1.P12R
<400> 514
   tgtccgtgcc caatgcctgc 20
<210> 515
   <211> 20
   <212> DNA
   <213> Artificial Sequence RPT10.1.P13F
<400> 515
   acacacagtc agtgtcagca 20
<210> 516
   <211> 20
   <212> DNA
   <213> Artificial Sequence RPT10.1.P14R
<400> 516
   ggggcccagc ttttccctgt 20
<210> 517
   <211> 20
   <212> DNA
   <213> Artificial Sequence RPT10.1.P15F
<400> 517
   ccagtcaggg gaaagctcta 20
<210> 518
   <211> 20
   <212> DNA
   <213> Artificial Sequence RPT10.1.P16R
<400> 518
   aggaaagacc ctgaacgtct 20
<210> 519
   <211> 21
   <212> DNA
   <213> Artificial Sequence FILr10/2.F1
<400> 519
   gtccacccat ggacagtctg t 21
<210> 520
   <211> 20
   <212> DNA
   <213> Artificial Sequence RPT10.2.P1F
<400> 520
   tgagtccacc catggacagt 20
<210> 521
   <211> 20
   <212> DNA
   <213> Artificial Sequence RPT10.2.P2R
<400> 521
   cctccagtgc tgggcacaga 20

<210> 522
   <211> 20
   <212> DNA
   <213> Artificial Sequence RPT10.2.P3F
<400> 522
   gacaaggatc ccaccatgat 20
<210> 523
   <211> 20
   <212> DNA
   <213> Artificial Sequence RPT10.2.P4R
<400> 523
   tggagctgtc ttgtgcctga 20
<210> 524
   <211> 20
   <212> DNA
   <213> Artificial Sequence RPT10.2.P5F
<400> 524
   cattcgtgga cacccggggc 20
<210> 525
   <211> 20
   <212> DNA
   <213> Artificial Sequence RPT10.2.P6R
<400> 525
   tgtcttcctc ctctgcttgg 20
<210> 526
   <211> 20
   <212> DNA
   <213> Artificial Sequence RPT10.2.P7F
<400> 526
   ctcccataaa caatcaaaac 20
<210> 527
   <211> 20
   <212> DNA
   <213> Artificial Sequence RPT10.2.P8R
<400> 527
   cccagaagtg caagcagaca 20
<210> 528
   <211> 20
   <212> DNA
   <213> Artificial Sequence RPT10.2.P9F
<400> 528
   agtgcaagca gacaaacaca 20
<210> 529
   <211> 20
   <212> DNA
   <213> Artificial Sequence RPT10.2.P10R
<400> 529
   tcctgattgt tccttgtcat 20
<210> 530
   <211> 20
   <212> DNA
   <213> Artificial Sequence RPT10.2.P11F
<400> 530
   tgcaagcaga caaacacatg 20
<210> 531
   <211> 20
   <212> DNA
   <213> Artificial Sequence RPT10.2.P12R
<400> 531
   tctcctgatt gttccttgtc 20
<210> 532
   <211> 20
   <212> DNA
   <213> Artificial Sequence RPT10.2.P13F
<400> 532
   agcagacaaa cacatgacaa 20
<210> 533
   <211> 20
   <212> DNA
   <213> Artificial Sequence RPT10.2.P14R
<400> 533
   gccgtctcct gattgttcct 20
<210> 534
   <211> 20
   <212> DNA
   <213> Artificial Sequence RPT10.2.P15F
<400> 534
   aacaatcagg agacggctct 20
<210> 535
   <211> 20
   <212> DNA
   <213> Artificial Sequence RPT10.2.P16R
<400> 535
   gacgcgaccc tgagtgccta 20
<210> 536
   <211> 20
   <212> DNA
   <213> Artificial Sequence RPT10.2.P17F
<400> 536 20
   gaattcctct ggtggacagc 20
<210> 537
   <211> 20
   <212> DNA
   <213> Artificial Sequence RPT10.2.P18R
<400> 537
   tgttcatggg atgatgcagg 20
<210> 538
   <211> 20
   <212> DNA
   <213> Artificial Sequence RPT10.2.P19F
<400> 538
   ccacacgtgg ccggtcagca 20
<210> 539
   <211> 20
   <212> DNA
   <213> Artificial Sequence RPT10.2.P20R
<400> 539
   ctgaacgtcc agaccttcct 20
<210> 540
   <211> 21
   <212> DNA
   <213> Artificial Sequence FAM11033de14.R
<220>
   <221> misc_feature
   <222> (1)..(21)
   <223> carboxyfluorescein labelled guanine
<400> 540 21
   natccttgtc ttcctccagt a 21
<210> 541
   <211> 21
   <212> DNA
   <213> Artificial Sequence Q3683X.R
<400> 541
   gatccttgtc ttcctccagt a 21
<210> 542
   <211> 22
   <212> DNA
   <213> Artificial Sequence FLGendF1
<400> 542
   ctagtaccgc taaggaacat gg 22
<210> 543
   <211> 19
   <212> DNA
   <213> Artificial Sequence FILKF
<400> 543
   ggcagctatg gtagtgcag 19
<210> 544
   <211> 19
   <212> DNA
   <213> Artificial Sequence FILKR
<400> 544
   ctgcactacc atagctgcc 19
<210> 545
   <211> 21
   <212> DNA
   <213> Artificial Sequence FLGendR1
<400> 545 21
   tggctccttc gatatttctg a 21
<210> 546
   <211> 12186
   <212> DNA
   <213> Human Profilaggrin
<400> 546
   atgtctactc tcctggaaaa catctttgcc ataattaatc ttttcaagca atattcaaaa 60
   aaagataaaa acactgacac attgagtaaa aaagagctga aggaacttct ggaaaaggaa 120
   tttcggcaaa tcctgaagaa tccagatgac ccagatatgg ttgatgtctt catggatcac 180
   ttggatatag accacaacaa gaaaattgac ttcactgagt ttcttctgat ggtattcaag 240
   ttggctcaag catattatga gtctaccaga aaagagaatt taccgatatc aggacacaag 300
   cacagaaagc acagtcatca tgataaacat gaagataata aacaggaaga aaacaaagaa 360
   aacagaaaaa gaccctcaag tctggaaaga agaaacaata gaaaagggaa taagggaaga 420
   tccaagagcc caagagaaac aggggggaaa aggcatgaat ctagttctga aaaaaaagaa 480
   agaaaaggat attcacctac tcatagagaa gaagaatatg gaaaaaacca tcataactca 540
   agtaaaaaag agaaaaacaa gactgaaaat actagattag gagacaatag gaagaggcta 600
   agtgaaagac ttgaagagaa agaagacaat gaagaaggag tatatgatta tgaaaataca 660
   ggaagaatga ctcaaaaatg gatacaatca ggccatattg ccacatatta cacaatccag 720
   gatgaagcct atgacaccac tgatagtcta ttagaagaaa acaaaatata tgaaagatca 780
   aggtcatctg atggcaaatc atcatctcaa gtgaacaggt caagacatga aaatacaagc 840
   caggtaccat tgcaggagtc caggacaaga aagcgtaggg gatccagagt tagccaggac 900
   agggacagtg agggacactc agaagactct gagaggcact ctgggtcggc ttccagaaac 960
   catcatggat ctgcgtggga gcagtcaaga gatggctcca gacaccccag gtcccatgat 1020
   gaagacagag ccagtcatgg gcactctgca gacagctcca gacaatcagg cactcgtcac 1080
   gcagagactt cctctcgtgg acagactgca tcatcccatg aacaggcaag atcaagtcca 1140
   ggagaaagac atggatccgg ccaccagcag tcagcagaca gctccagaca ctcagccact 1200
   gggcgcgggc aagcttcatc tgcagtcagc gatcgtggac accgggggtc tagcggtagt 1260
   caggccagtg acagtgaggg acattcagaa aactcagaca cacaatcagt gtcaggccac 1320
   ggaaaggctg ggctgagaca gcagagccac caagagtcca cacgtggccg gtcaggggaa 1380
   cggtctggac gttcagggtc ttccctctac caggtgagca ctcatgaaca gcctgactct 1440
   gcccatggac ggaccgggac cagcactgga ggaagacaag gatcgcacca cgagcaggca 1500
   cgagacagct ccaggcattc agcgtcccaa gagggtcagg acaccattcg tggacacccg 1560
   gggtcaagca gaggaggaag gcagggatcc caccacgagc aatcggtaaa taggtctgga 1620
   cactcaggtt cccatcacag ccacaccaca tcccagggaa ggtctgatgc ctcccatggg 1680
   cagtcaggat ccagaagtgc aagcagacaa acacgaaatg aggaacaatc aggagacggc 1740
   accaggcact cagggtcacg tcatcatgaa gcttcctctc aggctgacag ctctagacac 1800
   tcacaggtgg gccagggaca atcatcgggg cccaggacaa gtaggaacca gggatccagt 1860
   gttagccagg acagtgacag tcagggacac tcagaagact ctgagaggtg gtctgggtct 1920
   gcttccagaa accatcatgg atctgctcag gagcagtcaa gagatggctc cagacacccc 1980
   aggtcccatc acgaagacag agctggtcat gggcactctg cagacagctc cagaaaatca 2040
   ggcactcgtc acacacagaa ttcctctagt ggacaggctg cgtcatccca tgaacaggca 2100
   agatcaagtg caggagaaag acatggatcc cgccaccagc tccagtcagc agacagctcc 2160
   agacactcag gcactgggca cggacaagct tcatctgcag tcagagacag tggacaccga 2220
   gggtccagtg gtagtcaggc cactgacagt gagggacatt cagaagactc agacacacag 2280
   tcagtgtcag gccatggaca ggctggtcac catcagcaga gccaccaaga gtccgcacgt 2340
   gaccggtcag gggaaaggtc tcgacgttca gggtctttcc tctaccaggt gagcactcat 2400
   aaacagtctg agtcctccca tggatggaca gggcccagca ctggagtaag acaaggatcc 2460
   caccatgagc aggcacgaga caactccagg cactcagcat cccaagatgg tcaggacacc 2520
   attcgtggac acccggggtc aagcagaaga ggaaggcagg ggtcccacca cgagcaatcg 2580
   gtagataggt ctggacactc agggtcccat cacagccaca ccacatccca gggaaggtct 2640
   gatgcctccc gtgggcagtc aggatccaga agtgcaagca gaacaacacg taatgaggaa 2700
   caatcaagag acggctccag gcactcaggg tcacgtcacc atgaagcttc ctctcatgcc 2760
   gacatctcta gacactcaca ggcaggccag ggacaatcag aggggtccag gacaagcagg 2820
   cgccagggat ccagtgttag ccaggacagt gacagtgagg gacattcaga agactctgag 2880
   aggtggtctg ggtctgcttc cagaaaccat cgtggatctg ctcaggagca gtcaagacat 2940
   ggctccagac accccaggtc ccatcacgaa gacagagccg gtcacgggca ctctgcagac 3000
   agctccagac aatcaggaac tcctcacgca gagacttcct ctggtggaca ggctgcgtca 3060
   tcccatgaac aggcaagatc aagtccagga gaaagacacg gatcccgcca ccagcagtca 3120
   gcagacagct ccagacactc aggcattccg cgcagacaag cttcatctgc agtcagagac 3180
   agtggacact gggggtccag tggtagtcag gccagtgata gtgagggaca ttcagaggag 3240
   tcagacacac agtcagtgtc aggccatgga caggatgggc cccatcagca gagccaccaa 3300
   gagtccgcac gtgactggtc agggggaagg tctggacgtt cagggtcttt catctaccag 3360
   gtgagcactc atgaacagtc tgagtctgcc catgggcgga ccaggaccag cactggacga 3420
   agacaaggat cccaccacga gcaggcacga gacagctcca ggcactcagc gtcccaagag 3480
   ggtcaggaca ccattcgtgc acacccgggg tcaaggagag gaggaaggca gggatcccac 3540
   catgagcaat cggtagatag atctggacac tcagggtccc atcacagcca caccacatcc 3600
   cagggaaggt ctgatgcctc ccatgggcag tcaggatcca gaagtgcaag cagacaaact 3660
   cgtaaggaca aacaatcagg agacggctcc aggcactcag ggtcacgtca ccatgaagct 3720
   gcctcttggg ctgacagctc tagacactca caggtgggac aggaacaatc atcggggtcc 3780
   aggacaagca ggcaccaggg atccagtgtt agccaggaca gtgacagtga gagacactca 3840
   gacgactccg agaggttgtc tgggtctgct tccagaaacc atcatggatc ttctcgggag 3900
   cagtcaagag atggctccag acaccctggg ttccatcaag aagacagagc cagtcacggg 3960
   cactctgcag acagctccag acaatcaggc actcatcaca cagagtcttc ctctcatgga 4020
   caggctgtgt catcccatga acaggcaaga tcaagtccag gagaaagaca tggatcccgc 4080
   caccagcagt cagcagacag ctccagacac tcaggcattg ggcacagaca agcttcatct 4140
   gcagtcagag acagtggaca ccgagggtcc agtggtagtc aggtcactaa cagtgaggga 4200
   cattcagaag actcagacac acagtcagtg tcagcccacg gacaagctgg gccccatcag 4260
   cagagccaca aagagtccgc acgtggccag tcaggggaaa gctctggacg ttcaaggtct 4320
   ttcctctacc aggtgagctc tcatgaacag tctgagtcca cacacggaca gactgcaccc 4380
   agcactggag gaagacaagg atcccgccat gagcaggcac gaaacagctc taggcactca 4440
   gcatcccaag acggtcagga caccattcgt ggacacccgg ggtcaagcag aggaggaagg 4500
   cagggatcct accacgagca atcagtagat aggtctggac actcagggta ccatcacagc 4560
   cacaccacac cccagggaag gtctgatgcc tcccatgggc agtcaggacc cagaagtgca 4620
   agcaggcaaa caagaaatga ggaacaatca ggagacggct ccaggcactc agggtcacgt 4680
   caccatgaac cttccactcg ggccggcagc tctagacact cacaggtggg ccagggagaa 4740
   tcagcggggt ccaagacaag caggcgccag ggatccagtg ttagtcagga cagggacagt 4800
   gagggacact cagaagactc tgagaggcgg tctgagtcgg cttccagaaa ccattatgga 4860
   tctgctcggg agcagtcaag acatggctcc aggaacccca ggtcccatca agaagataga 4920
   gccagtcatg ggcactctgc agagagctcc agacaatcag gcactcgtca tgcagagact 4980
   tcctctggtg gacaggctgc atcatcccag gaacaggcaa ggtcaagtcc aggagaaaga 5040
   catggatccc gccaccagca gtcagcagac agctccacag actcaggcac tgggcgcaga 5100
   caagattcat ctgtagtcgg agacagtgga aaccgagggt ccagtggtag ccaggccagt 5160
   gacagcgagg gacactcaga agagtcagac acacagtcag tgtcagccca cggacaggct 5220
   gggccccatc agcagagcca ccaagagtcc acacgtggcc agtcagggga aaggtctgga 5280
   cgttcagggt ctttcctcta ccaggtgagc actcatgaac agtctgagtc cgcccatgga 5340
   cgcacagggc ccagcactgg aggaagacaa agatcccgcc acgagcaggc acgagacagc 5400
   tccaggcact cagcgtccca agagggtcag gacaccattc gtggacaccc agggtcaagc 5460
   agaggaggaa ggcagggatc ccactatgag caatcggtag atagttctgg acactcaggg 5520
   tctcatcaca gccacaccac gtcccaggaa aggtctgatg tctcccgtgg gcagtcagga 5580
   tccagaagtg tcagcagaca aacacgtaat gagaaacaat caggagacgg ctccaggcac 5640
   tcagggtcgc gtcaccatga agcttcctct cgggccgaca gctctagaca ctcgcaggtg 5700
   ggccagggac aatcatcagg gcccaggaca agcaggaacc agggatccag tgttagccag 5760
   gacagtgaca gtcagggaca ctcagaagac tctgagaggt ggtctgggtc tgcttccaga 5820
   aaccatcttg gatctgcttg ggagcagtca agagatggct ccagacaccc tgggtcccat 5880
   cacgaagaca gagccggtca cgggcactct gcagacagct ccagacaatc aggcactcgt 5940
   cacacagagt cttcctctcg tggacaggct gcgtcatccc atgaacaggc aagatcaagt 6000
   gcaggagaaa gacatggatc ccaccaccag ctccagtcag cagacagctc cagacactca 6060
   ggcattgggc atggacaagc ttcatctgca gtcagagaca gtggacaccg agggtacagt 6120
   ggtagtcagg ccagtgacag tgagggacat tcagaagact cagacacaca gtcagtgtca 6180
   gcacagggaa aagctgggcc ccatcagcag agccacaaag agtccgcacg tggccagtca 6240
   ggggaaagct ctggacgttc agggtctttc ctctaccagg tgagcactca tgaacagtct 6300
   gagtccaccc atggacagtc tgcgcccagc actggaggaa gacaaggatc ccattatgat 6360
   caggcacaag acagctccag gcactcagca tcccaagagg gtcaggacac cattcgtgga 6420
   cacccggggc caagcagagg aggaagacag gggtcccacc aagagcaatc ggtagatagg 6480
   tctggacact cagggtctca tcacagccac accacatccc agggaaggtc tgatgcctcc 6540
   cgtgggcagt caggatccag aagtgcaagc agaaaaacat atgacaagga acaatcagga 6600
   gatggctcta ggcactcagg gtcgcatcat catgaagctt cctcttgggc cgacagctct 6660
   agacactcac tggtgggcca gggacaatca tcagggccca ggacaagcag gccccgggga 6720
   tccagtgtta gccaggacag tgacagtgag ggacactcag aagattctga gaggcggtct 6780
   gggtctgcgt ccagaaacca tcatggatct gctcaggagc agtcaagaga tggctccaga 6840
   caccccaggt cccatcacga agacagagcc ggtcatgggc actctgcaga gagctccaga 6900
   caatcaggca ctcatcatgc agagaattcc tctggtggac aggctgcatc atcccatgaa 6960
   caggcaagat caagtgcagg agagagacac ggatcccacc accagcagtc agcagacagc 7020
   tccagacact caggcattgg gcacggacaa gcttcatctg cagtcagaga cagtggacac 7080
   cgagggtcca gtggtagtca ggccagtgac agtgagggac attcagaaga ctcagacaca 7140
   cagtcagtgt cagcccacgg acaggctggg ccccatcagc agagccacca agagtccaca 7200
   cgtggccggt cagcaggaag gtctggacgt tcagggtctt tcctctacca ggtgagcact 7260
   catgaacagt ctgagtccgc ccatggacgg accgggacca gcactggagg aagacaagga 7320
   tcccaccaca agcaggcacg agacagctcc aggcactcaa cgtcccaaga gggtcaggac 7380
   accattcatg gacacccggg gtcaagcagt ggaggaaggc agggatccca ctacgagcaa 7440
   ttggtagata gatctggaca ctcagggtct catcacagcc acaccacatc ccagggaagg 7500
   tctgatgcct cccatgggca ctcaggatcc agaagtgcaa gcagacaaac tcgtaacgat 7560
   gaacaatcag gagacggctc caggcactca gggtcgcgtc accatgaagc ttcctctcgg 7620
   gccgacagct ctggacactc gcaggtgggc cagggacaat cagaggggcc caggacaagc 7680
   aggaactggg gatccagttt tagccaggac agtgacagtc agggacactc agaagactct 7740
   gagaggtggt ctgggtctgc ttccagaaac catcatggat ctgctcagga gcagctaaga 7800
   gatggctcca gacaccccag gtcccatcaa gaagacagag ctggtcatgg gcactctgca 7860
   gacagctcca gacaatcagg cactcgtcac acacagactt cctctggtgg acaggctgca 7920
   tcatcccatg aacaggcaag atcaagtgca ggagaaagac atggatccca ccaccagcag 7980
   tcagcagaca gctccagaca ctcaggcatt gggcacggac aagcttcatc tgcagtcaga 8040
   gacagtggac accgagggta cagtggtagt caggccagtg acaatgaggg acattcagaa 8100
   gactcagaca cacagtcagt gtcagcccac ggacaggctg ggtcccatca gcagagccac 8160
   caagagtccg cacgtggccg gtcaggggaa acgtctggac attcaggatc tttcctctac 8220
   caggtgagca ctcatgaaca gtctgagtcc tcccatggat ggacggggcc cagcactaga 8280
   ggaagacaag gatcccgcca tgagcaggca caagacagct ccaggcactc agcatcccaa 8340
   gacggtcagg acaccattcg tggacacccg gggtcaagca gaggaggaag gcaggggtac 8400
   caccacgagc attcggtaga tagctctgga cactcagggt cccatcacag ccacaccaca 8460
   tcccagggaa ggtctgatgc ctcccgtggg cagtcaggat ccagaagtgc aagcagaaca 8520
   acacgtaatg aggaacaatc aggagacggc tccaggcact cagggtcgcg tcaccatgaa 8580
   gcttccactc atgccgacat ctctagacac tcacaggcag tccagggaca atcagagggg 8640
   tccaggagaa gcaggcgcca gggatccagt gtgagccagg acagtgacag tgagggacat 8700
   tcagaagact ctgagaggtg gtctgggtct gcttccagaa accatcatgg atctgctcag 8760
   gagcagctaa gagatggctc cagacacccc aggtcccatc aagaagacag agctggtcat 8820
   gggcactctg cagacagctc cagacaatca ggcactcgtc acacacagac ttcctctggt 8880
   ggacaggctg catcatccca tgaacaggca agatcaagtg caggagaaag acatggatcc 8940
   caccaccagc agtcagcaga cagctccaga cactcaggca ttgggcacgg acaagcttca 9000
   tctgcagtca gagacagtgg acaccgaggg tacagtggta gtcaggccag tgacaatgag 9060
   ggacattcag aagactcaga cacacagtca gtgtcagccc acggacaggc tgggtcccat 9120
   cagcagagcc accaagagtc cgcacgtggc cggtcagggg aaacgtctgg acattcagga 9180
   tctttcctct accaggtgag cactcatgaa cagtctgagt cctcccatgg atggacgggg 9240
   cccagcacta gaggaagaca aggatcccgc catgagcagg cacaagacag ctccaggcac 9300
   tcagcatccc aatacggtca ggacaccatt cgtggacacc cggggtcaag cagaggagga 9360
   aggcaggggt accaccacga gcattcggta gatagctctg gacactcagg gtcccatcac 9420
   agccacacca catcccaggg aaggtctgat gcctcccgtg ggcagtcagg atccagaagt 9480
   gcaagcagaa caacacgtaa tgaggaacaa tcaggagaca gctccaggca ctcagtgtca 9540
   cgtcaccatg aagcttccac tcatgccgac atctctagac actcacaggc agtccaggga 9600
   caatcagagg ggtccaggag aagcaggcgc cagggatcca gtgtgagcca ggacagtgac 9660
   agtgagggac attcagaaga ctctgagagg tggtctgggt ctgcttccag aaaccatcgt 9720
   ggatctgttc aggagcagtc aaggcacggc tccagacacc ccaggtccca tcacgaagac 9780
   agagccggtc acgggcactc tgcagaccgc tccagacaat caggcactcg tcacgcagag 9840
   acttcctctg gtggacaggc tgcatcatcc catgaacagg caagatcaag tccaggagag 9900
   agacacggat cccgccacca gcagtcagca gacagctcca gacactcagg cattccgcgt 9960
   ggacaagctt catctgcagt cagagacagt agacactggg ggtccagtgg tagtcaggcc 10020
   agtgatagtg agggacattc agaagagtca gacacacagt cagtgtcagg ccatggacag 10080
   gctgggcccc atcagcagag ccaccaagag tccgcacgtg accggtcagg gggaaggtct 10140
   ggacgttcag ggtctttcct ctaccaggtg agcactcatg aacagtctga gtctgcccat 10200
   gggcggacca ggaccagcac tggacgaaga caaggatccc accacgagca ggcacgagac 10260
   agctccaggc actcagcgtc ccaagagggt caggacacca ttcgtggaca cccggggtca 10320
   agcagaagag gaaggcaggg atcccactac gagcaatcgg tagataggtc tggacactca 10380
   gggtcccatc acagccacac cacatcccag ggaaggtctg atgcctcccg tgggcagtca 10440
   ggatccagaa gtgccagcag acaaactcgt aatgacgaac aatcaggaga tggctccagg 10500
   cactcatggt cgcatcacca tgaagcttcc actcaggcgg acagctctag acactcacag 10560
   tccggccagg gacaatcagc ggggcccagg acaagcagga accagggatc cagtgttagc 10620
   caggacagtg acagtcaggg acactcagaa gactctgaga ggtggtctgg gtctgcttcc 10680
   agaaaccatc gtggatctgc tcaggagcag tcaagagatg gctccagaca ccccacgtcc 10740
   catcacgaag acagagccgg tcacgggcac tctgcagaga gctccagaca atcaggcact 10800
   catcatgcag agaattcctc tggtggacag gctgcatcat cccatgaaca ggcaagatca 10860
   agtgcaggag agagacatgg atcccaccac cagcagtcag cagacagctc cagacactca 10920
   ggcattgggc acggacaagc ttcatctgca gtcagagaca gtggacaccg agggtccagt 10980
   ggtagtcagg ccagtgacag tgagggacat tcagaagact cagacacaca gtcagtgtca 11040
   gcccacggac aggctgggcc ccatcagcag agccaccaag agtccacacg tggccggtca 11100
   gcaggaaggt ctggacgttc agggtctttc ctctaccagg tgagcactca tgaacagtct 11160
   gagtctgccc atggacgggc tgggcccagt actggaggaa gacaaggatc ccgccacgag 11220
   caggcacgag acagctccag gcactcagcg tcccaagagg gtcaggacac cattcgtgga 11280
   cacccggggt caaggagagg aggaagacag ggatcctacc acgagcaatc ggtagatagg 11340
   tctggacact cagggtccca tcacagccac accacatccc agggaaggtc tgatgcctcc 11400
   catgggcagt caggatccag aagtgcaagc agagaaacac gtaatgagga acagtcagga 11460
   gacggctcca ggcactcagg gtcgcgtcac catgaagctt ccactcaggc tgacagctct 11520
   agacactcac agtccggcca gggtgaatca gcggggtcca ggagaagcag gcgccaggga 11580
   tccagtgtta gccaggacag tgacagtgag gcatacccag aggactctga gaggcgatct 11640
   gagtctgctt ccagaaacca tcatggatct tctcgggagc agtcaagaga tggctccaga 11700
   caccccggat cctctcaccg cgatacagcc agtcatgtac agtcttcacc tgtacagtca 11760
   gactctagta ccgctaagga acatggtcac tttagtagtc tttcacaaga ttctgcgtat 11820
   cactcaggaa tacagtcacg tggcagtcct cacagttcta gttcttatca ttatcaatct 11880
   gagggcactg aaaggcaaaa aggtcaatca ggtttagttt ggagacatgg cagctatggt 11940
   agtgcagatt atgattatgg tgaatccggg tttagacact ctcagcacgg aagtgttagt 12000
   tacaattcca atcctgttgt tttcaaggaa agatctgata tctgtaaagc aagtgcgttt 12060
   ggtaaagatc atccaaggta ttatgcaacg tatattaata aggacccagg tttatgtggc 12120
   cattctagtg atatatcgaa acaactggga tttagtcagt cacagagata ctattactat 12180
   gagtaa 12186
<210> 547
   <211> 975
   <212> DNA
   <213> Human Profilaggrin Repeat 1
<400> 547
   cctgactctg cccatggacg gaccgggacc agcactggag gaagacaagg atcgcaccac 60
   gagcaggcac gagacagctc caggcattca gcgtcccaag agggtcagga caccattcgt 120
   ggacacccgg ggtcaagcag aggaggaagg cagggatccc accacgagca atcggtaaat 180
   aggtctggac actcaggttc ccatcacagc cacaccacat cccagggaag gtctgatgcc 240
   tcccatgggc agtcaggatc cagaagtgca agcagacaaa cacgaaatga ggaacaatca 300
   ggagacggca ccaggcactc agggtcacgt catcatgaag cttcctctca ggctgacagc 360
   tctagacact cacaggtggg ccagggacaa tcatcggggc ccaggacaag taggaaccag 420
   ggatccagtg ttagccagga cagtgacagt cagggacact cagaagactc tgagaggtgg 480
   tctgggtctg cttccagaaa ccatcatgga tctgctcagg agcagtcaag agatggctcc 540
   agacacccca ggtcccatca cgaagacaga gctggtcatg ggcactctgc agacagctcc 600
   agaaaatcag gcactcgtca cacacagaat tcctctagtg gacaggctgc gtcatcccat 660
   gaacaggcaa gatcaagtgc aggagaaaga catggatccc gccaccagct ccagtcagca 720
   gacagctcca gacactcagg cactgggcac ggacaagctt catctgcagt cagagacagt 780
   ggacaccgag ggtccagtgg tagtcaggcc actgacagtg agggacattc agaagactca 840
   gacacacagt cagtgtcagg ccatggacag gctggtcacc atcagcagag ccaccaagag 900
   tccgcacgtg accggtcagg ggaaaggtct cgacgttcag ggtctttcct ctaccaggtg 960
   agcactcata aacag 975
<210> 548
   <211> 325
   <212> PRT
   <213> Human Profilaggrin Repeat 1
<400> 548
   Pro Asp Ser Ala His Gly Arg Thr Gly Thr Ser Thr Gly Gly Arg Gln
   1               5                   10                  15
   Gly Ser His His Glu Gln Ala Arg Asp Ser Ser Arg His Ser Ala Ser
               20                  25                  30
   Gln Glu Gly Gln Asp Thr Ile Arg Gly His Pro Gly Ser Ser Arg Gly
           35                  40                  45
   Gly Arg Gln Gly Ser His His Glu Gln Ser Val Asn Arg Ser Gly His
       50                  55                  60
   Ser Gly Ser His His Ser His Thr Thr Ser Gln Gly Arg Ser Asp Ala
   65                  70                  75                  80
   Ser His Gly Gln Ser Gly Ser Arg Ser Ala Ser Arg Gln Thr Arg Asn
                   85                  90                  95
   Glu Glu Gln Ser Gly Asp Gly Thr Arg His Ser Gly Ser Arg His His
               100                 105                 110
   Glu Ala Ser Ser Gln Ala Asp Ser Ser Arg His Ser Gln Val Gly Gln
           115                 120                 125
   Gly Gln Ser Ser Gly Pro Arg Thr Ser Arg Asn Gln Gly Ser Ser Val
       130                 135                 140
   Ser Gln Asp Ser Asp Ser Gln Gly His Ser Glu Asp Ser Glu Arg Trp
   145                 150                 155                 160
   Ser Gly Ser Ala Ser Arg Asn His His Gly Ser Ala Gln Glu Gln Ser
                   165                 170                 175
   Arg Asp Gly Ser Arg His Pro Arg Ser His His Glu Asp Arg Ala Gly
               180                 185                 190
   His Gly His Ser Ala Asp Ser Ser Arg Lys Ser Gly Thr Arg His Thr
           195                 200                 205
   Gln Asn Ser Ser Ser Gly Gln Ala Ala Ser Ser His Glu Gln Ala Arg
       210                 215                 220
   Ser Ser Ala Gly Glu Arg His Gly Ser Arg His Gln Leu Gln Ser Ala
   225                 230                 235                 240
   Asp Ser Ser Arg His Ser Gly Thr Gly His Gly Gln Ala Ser Ser Ala
   245 250 255
   Val Arg Asp Ser Gly His Arg Gly Ser Ser Gly Ser Gln Ala Thr Asp
   260 265 270
   Ser Glu Gly His Ser Glu Asp Ser Asp Thr Gln Ser Val Ser Gly His
   275 280 285
   Gly Gln Ala Gly His His Gln Gln Ser His Gln Glu Ser Ala Arg Asp
   290 295 300
   Arg Ser Gly Glu Arg Ser Arg Arg Ser Gly Ser Phe Leu Tyr Gln Val
   305 310 315 320
   Ser Thr His Lys Gln
   325
<210> 549
   <211> 971
   <212> DNA
   <213> Human Profilaggrin Repeat 1 - 2282de14
<400> 549
   cctgactctg cccatggacg gaccgggacc agcactggag gaagacaagg atcgcaccac 60
   gagcaggcac gagacagctc caggcattca gcgtcccaag agggtcagga caccattcgt 120
   ggacacccgg ggtcaagcag aggaggaagg cagggatccc accacgagca atcggtaaat 180
   aggtctggac actcaggttc ccatcacagc cacaccacat cccagggaag gtctgatgcc 240
   tcccatgggc agtcaggatc cagaagtgca agcagacaaa cacgaaatga ggaacaatca 300
   ggagacggca ccaggcactc agggtcacgt catcatgaag cttcctctca ggctgacagc 360
   tctagacact cacaggtggg ccagggacaa tcatcggggc ccaggacaag taggaaccag 420
   ggatccagtg ttagccagga cagtgacagt cagggacact cagaagactc tgagaggtgg 480
   tctgggtctg cttccagaaa ccatcatgga tctgctcagg agcagtcaag agatggctcc 540
   agacacccca ggtcccatca cgaagacaga gctggtcatg ggcactctgc agacagctcc 600
   agaaaatcag gcactcgtca cacacagaat tcctctagtg gacaggctgc gtcatcccat 660
   gaacaggcaa gatcaagtgc aggagaaaga catggatccc gccaccagct ccagtcagca 720
   gacagctcca gacactcagg cactgggcac ggacaagctt catctgcagt cagagacagt 780
   ggacaccgag ggtccagtgg tagtcaggcc actgacagtg agggacattc agaagactca 840
   gacacacagt gtcaggccat ggacaggctg gtcaccatca gcagagccac caagagtccg 900
   cacgtgaccg gtcaggggaa aggtctcgac gttcagggtc tttcctctac caggtgagca 960
   ctcataaaca g 971
<210> 550
   <211> 323
   <212> PRT
   <213> Human Profilaggrin Repeat 1 - 2282de14
<220>
   <221> MISC_FEATURE
   <222> (319)..()
   <223> X = STOP
<220>
   <221> MISC_FEATURE
   <222> (1)..(323)
   <223> x = STOP
<400> 550
   Pro Asp Ser Ala His Gly Arg Thr Gly Thr Ser Thr Gly Gly Arg Gln
   1               5                   10                  15
   Gly Ser His His Glu Gln Ala Arg Asp Ser Ser Arg His Ser Ala Ser
               20                  25                  30
   Gln Glu Gly Gln Asp Thr Ile Arg Gly His Pro Gly Ser Ser Arg Gly
           35                  40                  45
   Gly Arg Gln Gly Ser His His Glu Gln Ser Val Asn Arg Ser Gly His
       50                  55                  60
   Ser Gly Ser His His Ser His Thr Thr Ser Gln Gly Arg Ser Asp Ala
   65                  70                  75                  80
   Ser His Gly Gln Ser Gly Ser Arg Ser Ala Ser Arg Gln Thr Arg Asn
                   85                  90                  95
   Glu Glu Gln Ser Gly Asp Gly Thr Arg His Ser Gly Ser Arg His His
               100                 105                 110
   Glu Ala Ser Ser Gln Ala Asp Ser Ser Arg His Ser Gln Val Gly Gln
           115                 120                 125
   Gly Gln Ser Ser Gly Pro Arg Thr Ser Arg Asn Gln Gly Ser Ser Val
       130                 135                 140
   Ser Gln Asp Ser Asp Ser Gln Gly His Ser Glu Asp Ser Glu Arg Trp
   145 150 155 160
   Ser Gly Ser Ala Ser Arg Asn His His Gly Ser Ala Gln Glu Gln Ser
   165 170 175
   Arg Asp Gly Ser Arg His Pro Arg Ser His His Glu Asp Arg Ala Gly
   180 185 190
   His Gly His Ser Ala Asp Ser Ser Arg Lys Ser Gly Thr Arg His Thr
   195 200 205
   Gln Asn Ser Ser Ser Gly Gln Ala Ala Ser Ser His Glu Gln Ala Arg
   210 215 220
   Ser Ser Ala Gly Glu Arg His Gly Ser Arg His Gln Leu Gln Ser Ala
   225 230 235 240
   Asp Ser Ser Arg His Ser Gly Thr Gly His Gly Gln Ala Ser Ser Ala
   245 250 255
   Val Arg Asp Ser Gly His Arg Gly Ser Ser Gly Ser Gln Ala Thr Asp
   260 265 270
   Ser Glu Gly His Ser Glu Asp Ser Asp Thr Gln Cys Gln Ala Met Asp
   275 280 285
   Arg Leu Val Thr Ile Ser Arg Ala Thr Lys Ser Pro His Val Thr Gly
   290 295 300
   Gln Gly Lys Gly Leu Asp Val Gln Gly Leu Ser Ser Thr Arg Xaa Ala
   305 310 315 320
   Leu Ile Asn
<210> 551
   <211> 975
   <212> DNA
   <213> Human Profilaggrin repeat 1 - R501X C>T
<400> 551
   cctgactctg cccatggacg gaccgggacc agcactggag gaagacaagg atcgcaccac 60
   gagcaggcat gagacagctc caggcattca gcgtcccaag agggtcagga caccattcgt 120
   ggacacccgg ggtcaagcag aggaggaagg cagggatccc accacgagca atcggtaaat 180
   aggtctggac actcaggttc ccatcacagc cacaccacat cccagggaag gtctgatgcc 240
   tcccatgggc agtcaggatc cagaagtgca agcagacaaa cacgaaatga ggaacaatca    300
   ggagacggca ccaggcactc agggtcacgt catcatgaag cttcctctca ggctgacagc    360
   tctagacact cacaggtggg ccagggacaa tcatcggggc ccaggacaag taggaaccag    420
   ggatccagtg ttagccagga cagtgacagt cagggacact cagaagactc tgagaggtgg    480
   tctgggtctg cttccagaaa ccatcatgga tctgctcagg agcagtcaag agatggctcc    540
   agacacccca ggtcccatca cgaagacaga gctggtcatg ggcactctgc agacagctcc    600
   agaaaatcag gcactcgtca cacacagaat tcctctagtg gacaggctgc gtcatcccat    660
   gaacaggcaa gatcaagtgc aggagaaaga catggatccc gccaccagct ccagtcagca    720
   gacagctcca gacactcagg cactgggcac ggacaagctt catctgcagt cagagacagt    780
   ggacaccgag ggtccagtgg tagtcaggcc actgacagtg agggacattc agaagactca    840
   gacacacagt cagtgtcagg ccatggacag gctggtcacc atcagcagag ccaccaagag    900
   tccgcacgtg accggtcagg ggaaaggtct cgacgttcag ggtctttcct ctaccaggtg    960
   agcactcata aacag                                                     975
<210> 552
   <211> 325
   <212> PRT
   <213> Human Profilaggrin Repeat 1 R501X C>T
<220>
   <221> MISC_FEATURE
   <222> (1)..(325)
   <223> X = STOP
<400> 552 <211> 5251
   <212> DNA
   <213> Homo sapiens
<400> 553
<210> 554
   <211> 1750
   <212> PRT
   <213> Homo sapiens
<400> 554
   Arg Thr Ser Arg Asn Arg Gly Ser Ser Phe Ser Gln Asp Ser Asp Ser
   1 5 10 15
   Gln Gly His Ser Glu Asp Ser Glu Arg Trp Ser Gly Ser Ala Ser Arg
   20 25 30
   Asn His His Gly Ser Ala Gln Glu Gln Leu Arg Asp Gly Ser Arg His
   35 40 45
   Pro Arg Ser His Gln Glu Asp Arg Ala Gly His Gly His Ser Ala Asp
   50 55 60
   Ser Ser Arg Gln Ser Gly Thr Arg His Thr Gln Thr Ser Ser Gly Gly
   65 70 75 80
   Gln Ala Ala Ser Ser His Glu Gln Ala Arg Ser Ser Ala Gly Glu Arg
   85 90 95
   His Gly Ser His His Gln Gln Ser Ala Asp Ser Ser Arg His Ser Gly
   100 105 110
   Ile Gly His Gly Gln Ala Ser Ser Ala Val Arg Asp Ser Gly His Arg
   115 120 125
   Gly Tyr Ser Gly Ser Gln Ala Ser Asp Asn Glu Gly His Ser Glu Asp
   130 135 140
   Ser Asp Thr Gln Ser Val Ser Ala His Gly Gln Ala Gly Ser His Gln
   145 150 155 160
   Gln Ser His Gln Glu Ser Ala Arg Gly Arg Ser Gly Glu Thr Ser Gly
   165 170 175
   His Ser Gly Ser Phe Leu Tyr Gln Val Ser Thr His Glu Gln Ser Glu
   180 185 190
   Ser Ser His Gly Trp Thr Gly Pro Ser Thr Arg Gly Arg Gln Gly Ser
   195 200 205
   Arg His Glu Gln Ala Gln Asp Ser Ser Arg His Ser Ala Ser Gln Asp
   210 215 220
   Gly Gln Asp Thr Ile Arg Gly His Pro Gly Ser Ser Arg Gly Gly Arg
   225 230 235 240
   Gln Gly Tyr His His Glu His Ser Val Asp Ser Ser Gly His Ser Gly
   245 250 255
   Ser His His Ser His Thr Thr Ser Gln Gly Arg Ser Asp Ala Ser Arg
   260 265 270
   Gly Gln Ser Gly Ser Arg Arg Ala Ser Arg Thr Thr Arg Asn Glu Glu
   275 280 285
   Gln Ser Gly Asp Gly Ser Arg His Ser Gly Ser Arg His His Glu Ala
   290 295 300
   Ser Thr His Ala Asp Ile Ser Arg His Ser Gln Ala Val Gln Gly Gln
   305 310 315 320
   Ser Glu Gly Ser Arg Arg Ser Arg Arg Gln Gly Ser Ser Val Ser Gln
   325 330 335
   Asp Ser Asp Ser Glu Gly His Ser Glu Asp Ser Glu Arg Trp Ser Gly
   340 345 350
   Ser Ala Ser Arg Asn His His Gly Ser Ala Gln Glu Gln Leu Arg Asp
   355 360 365
   Gly Ser Arg His Pro Arg Ser His Gln Glu Gly Arg Ala Gly His Gly
   370 375 380
   His Ser Ala Asp Ser Ser Arg Gln Ser Gly Thr Arg His Thr Gln Thr
   385 390 395 400
   Ser Ser Gly Gly Gln Ala Ala Ser Ser His Glu Gln Ala Arg Ser Ser
   405 410 415
   Ala Gly Glu Arg His Gly Ser His His Gln Gln Ser Ala Asp Ser Ser
   420 425 430
   Arg His Ser Gly Ile Gly His Gly Gln Ala Ser Ser Ala Val Arg Asp
   435 440 445
   Ser Gly His Arg Gly Tyr Ser Gly Ser Gln Ala Ser Asp Asn Glu Gly
   450 455 460
   His Ser Glu Asp Ser Asp Thr Gln Ser Val Ser Ala His Gly Gln Ala
   465 470 475 480
   Gly Ser His Gln Gln Ser His Gln Glu Ser Ala Arg Gly Arg Ser Gly
   485 490 495
   Glu Thr Ser Gly His Ser Gly Ser Phe Leu Tyr Gln Val Ser Thr His
   500 505 510
   Glu Gln Ser Glu Ser Ser His Gly Trp Thr Gly Pro Ser Thr Arg Gly
   515 520 525
   Arg Gln Gly Ser Arg His Glu Gln Ala Gln Asp Ser Ser Arg His Ser
   530 535 540
   Ala Ser Gln Asp Gly Gln Asp Thr Ile Arg Gly His Pro Gly Ser Ser
   545 550 555 560
   Arg Gly Gly Arg Gln Gly Tyr His His Glu His Ser Val Asp Ser Ser
   565 570 575
   Gly His Ser Gly Ser His His Ser His Thr Thr Ser Gln Gly Arg Ser
               580                 585                 590
   Asp Ala Ser Arg Gly Gln Ser Gly Ser Arg Ser Ala Ser Arg Thr Thr
           595                 600                 605
   Arg Asn Glu Glu Gln Ser Gly Asp Gly Ser Arg His Ser Gly Ser Arg
       610                 615                 620
   His His Glu Ala Ser Thr His Ala Asp Ile Ser Arg His Ser Gln Ala
   625                 630                 635                 640
   Val Gln Gly Gln Ser Glu Gly Ser Arg Arg Ser Arg Arg Gln Gly Ser
                   645                 650                 655
   Ser Val Ser Gln Asp Ser Asp Ser Glu Gly His Ser Glu Asp Ser Glu
               660                 665                 670
   Arg Trp Ser Gly Ser Ala Ser Arg Asn His His Gly Ser Ala Gln Glu
           675                 680                 685
   Gln Leu Arg Asp Gly Ser Arg His Pro Arg Ser His Gln Glu Asp Arg
       690                 695                 700
   Ala Gly His Gly His Ser Ala Asp Ser Ser Arg Gln Ser Gly Thr Arg
   705                 710                 715                 720
   His Thr Gln Ala Ser Ser Gly Gly Gln Ala Ala Ser Ser His Glu Gln
                   725                 730                 735
   Ala Arg Ser Ser Ala Gly Glu Arg His Gly Ser His His Gln Gln Ser
               740                 745                 750
   Ala Asp Ser Ser Arg His Ser Gly Ile Gly His Gly Gln Ala Ser Ser
           755                 760                 765
   Ala Val Arg Asp Ser Gly His Arg Gly Tyr Ser Gly Ser Gln Ala Ser
       770                 775                 780
   Asp Asn Glu Gly His Ser Glu Asp Ser Asp Thr Gln Ser Val Ser Ala
   785                 790                 795                 800
   His Gly Gln Ala Gly Ser His Gln Gln Ser His Gln Glu Ser Ala Arg
                   805                 810                 815
   Gly Arg Ser Gly Glu Thr Ser Gly His Ser Gly Ser Phe Leu Tyr Gln
               820                 825                 830
   Val Ser Thr His Glu Gln Ser Glu Ser Ser His Gly Trp Thr Gly Pro
           835                 840                 845
   Ser Thr Arg Gly Arg Gln Gly Ser Arg His Glu Gln Ala Gln Asp Ser
       850                 855                 860
   Ser Arg His Ser Ala Ser Gln Asp Gly Gln Asp Thr Ile Arg Gly His
   865                 870                 875                 880
   Pro Gly Ser Ser Arg Gly Gly Arg Gln Gly Tyr His His Glu His Ser
                   885                 890                 895
   Val Asp Ser Ser Gly His Ser Gly Ser His His Ser His Thr Thr Ser
               900                 905                 910
   Gln Gly Arg Ser Asp Ala Ser Arg Gly Gln Ser Gly Ser Arg Ser Ala
           915                 920                 925
   Ser Arg Thr Thr Arg Asn Glu Glu Gln Ser Gly Asp Ser Ser Arg His
       930                 935                 940
   Ser Gly Ser Arg His His Glu Ala Ser Thr His Ala Asp Ile Ser Arg
   945                 950                 955                 960
   His Ser Gln Ala Val Gln Gly Gln Ser Glu Gly Ser Arg Arg Ser Arg
                   965                 970                 975
   Arg Gln Gly Ser Ser Val Ser Gln Asp Ser Asp Ser Glu Gly His Ser
               980                 985                 990
   Glu Asp Ser Glu Arg Trp Ser Gly  Ser Ala Ser Arg Asn  His Arg Gly
           995                 1000                1005
   Ser Val  Gln Glu Gln Ser Arg  His Gly Ser Arg His  Pro Arg Ser
       1010                 1015                 1020
   His His  Glu Asp Arg Ala Gly  His Gly His Ser Ala  Asp Arg Ser
       1025                 1030                 1035
   Arg Gln  Ser Gly Thr Arg His  Ala Glu Thr Ser Ser  Gly Gly Gln
       1040                 1045                 1050
   Ala Ala  Ser Ser His Glu Gln  Ala Arg Ser Ser Pro  Gly Glu Arg
       1055                 1060                 1065
   His Gly  Ser Arg His Gln Gln  Ser Ala Asp Ser Ser  Arg His Ser
       1070                 1075                 1080
   Gly Ile  Pro Arg Gly Gln Ala  Ser Ser Ala Val Arg  Asp Ser Arg
       1085                 1090                 1095
   His Trp  Gly Ser Ser Gly Ser  Gln Ala Ser Asp Ser  Glu Gly His
       1100                 1105                 1110
   Ser Glu Glu Ser Asp Thr Gln Ser Val Ser Gly His Gly Gln Ala
   1115 1120 1125
   Gly Pro His Gln Gln Ser His Gln Glu Ser Ala Arg Asp Arg Ser
   1130 1135 1140
   Gly Gly Arg Ser Gly Arg Ser Gly Ser Phe Leu Tyr Gln Val Ser
   1145 1150 1155
   Thr His Glu Gln Ser Glu Ser Ala His Gly Arg Thr Arg Thr Ser
   1160 1165 1170
   Thr Gly Arg Arg Gln Gly Ser His His Glu Gln Ala Arg Asp Ser
   1175 1180 1185
   Ser Arg His Ser Ala Ser Gln Glu Gly Gln Asp Thr Ile Arg Ala
   1190 1195 1200
   His Pro Gly Ser Ser Arg Arg Gly Arg Gln Gly Ser His Tyr Glu
   1205 1210 1215
   Gln Ser Val Asp Arg Ser Gly His Ser Gly Ser His His Ser His
   1220 1225 1230
   Thr Thr Ser Gln Gly Arg Ser Asp Ala Ser Arg Gly Gln Ser Gly
   1235 1240 1245
   Ser Arg Ser Ala Ser Arg Gln Thr Arg Asn Asp Glu Gln Ser Gly
   1250 1255 1260
   Asp Gly Ser Arg His Ser Trp Ser His His His Glu Ala Ser Thr
   1265 1270 1275
   Gln Ala Asp Ser Ser Arg His Ser Gln Ser Gly Gln Gly Gln Ser
   1280 1285 1290
   Ala Gly Pro Ser Thr Ser Arg Asn Gln Gly Ser Ser Val Ser Gln
   1295 1300 1305
   Asp Ser Asp Ser Gln Gly His Ser Glu Asp Ser Glu Arg Trp Ser
   1310 1315 1320
   Gly Ser Ala Ser Arg Asn His His Gly Ser Ala Gly Glu Gln Ser
   1325 1330 1335
   Arg Asp Gly Ser Arg His Pro Gly Ser His Gln Glu Asp Arg Ala
   1340 1345 1350
   Gly His Gly His Ser Ala Asp Ser Pro Arg Gln Ser Gly Thr Arg
   1355 1360 1365
   His Thr Glu Ser Ser Ser Arg Gly Gln Ala Ala Ser Ser His Glu
   1370 1375 1380
   Gln Ala Arg Ser Ser Ala Gly Glu Arg His Gly Ser His His Gln
   1385 1390 1395
   Leu Gln Ser Ala Asp Ser Ser Arg His Ala Gly Ile Gly His Gly
   1400 1405 1410
   Gln Ala Ser Ser Ala Val Arg Asp Ser Gly His Arg Gly Tyr Ser
   1415 1420 1425
   Gly Ser Gln Ala Thr Asp Ser Glu Gly His Ser Glu Asp Ser Asp
   1430 1435 1440
   Thr Gln Ser Val Ser Ala Gln Gly Lys Ala Gly Pro His Gln Gln
   1445 1450 1455
   Ser His Lys Glu Ser Ala Arg Gly Gln Ser Gly Glu Ser Ser Arg
   1460 1465 1470
   Arg Ser Gly Ser Phe Leu Tyr Gln Val Ser Thr His Glu Gln Ser
   1475 1480 1485
   Glu Ser Ala His Gly Arg Ala Gly Pro Ser Thr Gly Gly Arg Gln
   1490 1495 1500
   Gly Ser His His Glu Gln Ala Arg Asp Ser Ser Arg His Ser Ala
   1505 1510 1515
   Ser Gln Glu Gly Gln Asp Thr Ile Arg Gly His Pro Gly Ser Arg
   1520 1525 1530
   Arg Gly Gly Arg Gln Gly Ser Tyr His Glu Gln Ser Val Asp Arg
   1535 1540 1545
   Ser Gly His Ser Gly Ser His His Ser His Thr Thr Ser Gln Gly
   1550 1555 1560
   Arg Ser Asp Ala Ser His Gly Gln Ser Gly Ser Arg Ser Ala Ser
   1565 1570 1575
   Arg Glu Thr Arg Asn Glu Glu Gln Ser Gly Asp Gly Ser Arg His
   1580 1585 1590
   Ser Gly Ser Arg His His Glu Ala Ser Thr Gln Ala Asp Ser Ser
   1595 1600 1605
   Arg His Ser Gln Ser Gly Gln Gly Glu Ser Ala Gly Ser Arg Arg
   1610 1615 1620
   Ser Arg Arg Gln Gly Ser Ser Val Ser Gln Asp Ser Asp Ser Glu
   1625 1630 1635
   Ala Tyr Pro Glu Asp Ser Glu Arg Arg Ser Glu Ser Ala Ser Arg
   1640 1645 1650
   Asn His His Gly Ser Ser Arg Glu Gln Ser Arg Asp Gly Ser Arg
   1655 1660 1665
   His Pro Gly Ser Ser His Arg Asp Thr Ala Ser His Val Gln Ser
   1670 1675 1680
   Ser Pro Val Gln Ser Asp Ser Ser Thr Ala Lys Glu His Gly His
   1685 1690 1695
   Phe Ser Ser Leu Ser Gln Asp Ser Ala Tyr His Ser Gly Ile Gln
   1700 1705 1710
   Ser Arg Gly Ser Pro His Ser Ser Ser Ser Tyr His Tyr Gln Ser
   1715 1720 1725
   Glu Gly Thr Glu Arg Gln Lys Gly Gln Ser Gly Leu Val Trp Arg
   1730 1735 1740
   His Gly Ser Tyr Gly Ser Ala
   1745 1750
<210> 555
   <211> 5251
   <212> DNA
   <213> Homo sapiens
<400> 555
   aggacaagca ggaaccgggg atccagtttt agccaggaca gtgacagtca gggacactca 60
   gaagactctg agaggtggtc tgggtctgct tccagaaacc atcatggatc tgctcaggag 120
   cagctaagag atggctccag acaccccagg tcccatcaag aagacagagc tggtcatggg 180
   cactctgcag acagctccag acaatcaggc actcgtcaca cacagacttc ctctggtgga 240
   caggctgcat catcccatga acaggcaaga tcaagtgcag gagaaagaca tggatcccac 300
   caccagcagt cagcagacag ctccagacac tcaggcattg ggcacggaca agcttcatct 360
   gcagtcagag acagtggaca ccgagggtac agtggtagtc aggccagtga caatgaggga 420
   cattcagaag actcagacac acagtcagtg tcagcccacg gacaggctgg gtcccatcag 480
   cagagccacc aagagtccgc acgtggccgg tcaggggaaa cgtctggaca ttcagggtct 540
   ttcctctacc aggtgagcac tcatgaacag tctgagtcct cccatggatg gacggggccc 600
   agcactagag gaagacaagg atcccgccat gagcaggcac aagacagctc caggcactca 660
   gcatcccaag acggtcagga caccattcgt ggacacccgg ggtcaagcag aggaggaagg 720
   caggggtacc accacgagca ttcggtagat agctctggac actcagggtc ccatcacagc 780
   cacaccacat cccagggaag gtctgatgcc tcccgtgggc agtcaggatc cagacgtgca 840
   agcagaacaa cacgtaatga ggaacaatca ggagacggct ccaggcactc agggtcgcgt 900
   caccatgaag cttccactca tgccgacatc tctagacact cacaggcagt ccagggacaa 960
   tcagaggggt ccaggagaag caggcgccag ggatccagtg ttagccagga cagtgacagt 1020
   gagggacatt cagaagactc tgagaggtgg tctgggtctg cttccagaaa ccatcatgga 1080
   tctgctcagg agcagctaag agatggctcc agacacccca ggtcccatca agaaggcaga 1140
   gctggtcatg ggcactctgc agacagctcc agacaatcag gcactcgtca cacacagact 1200
   tcctctggtg gacaggctgc atcatcycat gaacaggcaa gatcaagtgc aggagaaaga 1260
   catggatccc accaccagca gtcagcagac agctccagac actcaggcat tgggcacgga 1320
   caagcttcat ctgcagtcag agacagtgga caccgagggt acagtggtag tcaggccagt 1380
   gacaatgagg gacattcaga agactcagac acacagtcag tgtcagccca cggacaggct 1440
   gggtcccatc agcagagcca ccaagagtcc gcacgtggcc ggtcagggga aacgtctgga 1500
   cattcaggat ctttcctcta ccaggtgagc actcatgaac agtctgagtc ctcccatgga 1560
   tggacggggc ccagcactag aggaagacaa ggatcccgcc atgagcaggc acaagacagc 1620
   tccaggcact cagcatccca agacggtcag gacaccattc gtggacaccc ggggtcaagc 1680
   agaggaggaa ggcaggggta ccaccacgag cattcggtag atagctctgg acactcaggg 1740
   tcccatcaca gccacaccac atcccaggga aggtctgatg cctcccgtgg gcagtcagga 1800
   tccagaagtg caagcagaac aacacgtaat gaggaacaat caggagacag ctccaggcac 1860
   tcagggtcgc gtcaccatga agcttccact catgccgaca tctctagaca ctcacaggca 1920
   gtccagggac aatcagaggg gtccaggaga agcaggcgcc agggatccag tgttagccag 1980
   gacagtgaca gtgagggaca ttcagaagac tctgagaggt ggtctgggtc tgcttccaga 2040
   aaccatcgtg gatctgttca ggagcagtca aggcacggct ccagacaccc caggtcccat 2100
   cacgaagaca gagccggtca cgggcactct gcagaccgct ccagacaatc aggcactcgt 2160
   cacgcagaga cttcctctgg tggacaggct gcatcatccc atgaacaggc aagatcaagt 2220
   ccaggagaga gacacggatc ccgccaccag cagtcagcag acagctccag acactcaggc 2280
   attccgcgtg gacaggcttc atctgcagtc agagacagta gacactgggg gtccagtggt 2340
   agtcaagcca gtgatagtga gggacattca gaagagtcag acacacagtc agtgtcaggc 2400
   catggacagg ctgggcccca tcagcagagc caccaagagt ccgcacgtga ccggtcaggg 2460
   ggaaggtctg gacgttcagg gtctttcctc taccaggtga gcactcatga acagtctgag 2520
   tccgcccatg ggcggaccag gaccagcact ggacgaagac aaggatccca ccacgagcag 2580
   gcacgagaca gctccaggca ctcagcgtcc caagagggtc aggacaccat tcgtgcacac 2640
   ccggggtcaa gcagaagagg aaggcaggga tcccactacg agcaatcggt agataggtct 2700
   ggacactcag ggtcccatca cagccacacc acatcccagg gaaggtctga tgcctcccgt 2760
   gggcagtcag gatccagaag tgccagcaga caaactcgta acgacgaaca atcaggagac 2820
   ggctccaggc actcatggtc gcatcaccat gaagcttcca ctcaggcgga cagctctaga 2880
   cactcacagt ccggccaggg acaatcagcg gggcccagta caagcaggaa ccagggatcc 2940
   agtgttagcc aggacagtga cagtcaggga cactcagaag actctgagag gtggtctggg 3000
   tctgcttcca gaaaccatca tggatctgct ggggagcagt caagagatgg ctccagacac 3060
   cctgggtccc atcaagaaga cagagccggt cacgggcact ctgcagacag ccccagacaa 3120
   tcaggcactc gtcacacaga gtcttcctct cgtggacagg ctgcgtcatc ccatgaacag 3180
   gcaagatcaa gtgcaggaga aagacatgga tcccaccacc agctccagtc agcagacagc 3240
   tccagacacg caggcattgg gcacggacaa gcttcatctg cagtcagaga cagtggacac 3300
   cgagggtaca gtggtagtca ggccactgac agtgagggac attcagaaga ctcagacaca 3360
   cagtcagtgt cagcacaggg aaaagctggg ccccatcagc agagccacaa agagtccgca 3420
   cgtggccagt caggggaaag ctctagacgt tcagggtctt tcctctacca ggtgagcact 3480
   catgaacagt ctgagtccac ccatggacag tctgtgccca gcactggagg aagacaagga 3540
   tcccaccatg atcaggcaca agacagctcc aggcactcag catcccaaga gggtcaggac 3600
   accattcgtg gacacccggg gccaagcaga ggaggaagac aggggtccca ccacgagcaa 3660
   tcggtagata ggtctggaca ctcagggtcc catcacagcc acaccacatc ccagggaagg 3720
   tctgatgcct cccgtgggca gtcaggaccc agaagtgcaa gcagacaaac acatgacaag 3780
   gaacaatcag gagacggctc taggcactca gggtcgcgtc atcatgaagc ttcctcttgg 3840
   gccgacagct ctagacactc acaggcagtc cagggacaat cagaggggtc caggagaagc 3900
   aggcgccagg gatccagtgt tagccaggac agtgacagtc agggacactc agaagactct 3960
   gagaggcggt ctgggtctgc ttccagaaac catcgtggat ctgctcagga gcagtcaaga 4020
   gatggctcca gacaccccag gtcccatcac gaagacagag ccggtcatgg gcactctgca 4080
   gacagctcca gacaatcagg cactcatcat gcagagaatt cctctggtgg acagcctgca 4140
   tcatcccatg aacaggcaag atcaagtgca ggagagagac atggatccca ccaccagcag 4200
   tcagcagaca gctccagaca ctcaggcatt gggcacggac aagcttcatc tgcagtcaga 4260
   gacagtggac accgagggtc cagtggtagt caggccagtg acagtgaggg acattcagaa 4320
   gactcagaca cacagtcagt gtcagcccac ggacaggctg ggccccatca gcagagccac 4380
   caagagtcca cacgtggccg gtcagcagga aggtctggac gttcagggtc tttcctctac 4440
   caggtgagca ctcatgaaca gtctgagtct gcccatggac gggctgggcc cagtactgga 4500
   ggaagacaag gatcccacca cgagcaggca cgagacagct ccaggcactc agcgtcccaa 4560
   gagggtcagg acaccattcg tggacacccg gggtcaagga gaggaggaag acagggatcc 4620
   taccacgagc aatcggtaga taggtctgga cactcagggt cccatcacag ccacaccaca 4680
   tcccagggaa ggtctgatgc ctcccatggg cagtcaggat ccagaagtgc aagcagagaa   4740
   acacgtaatg aggaacagtc aggagacggc tccaggcact cagggtcgcg tcaccatgaa   4800
   gcttccactc aggctgacag ctctagacac tcacagtccg gccagggtga atcagcgggg   4860
   tccaggagaa gcaggcgcca gggatccagt gttagccagg acagtgacag tgaggcatac   4920
   ccagaggact ctgagaggcg atctgagtct gcttccagaa accatcatgg atcttctcgg   4980
   gagcagtcaa gagatggctc cagacacccc ggatcctctc accgcgatac agccagtcat   5040
   gtacagtctt cacctgtaca gtcagactct agtaccgcta aggaacatgg tcactttagt   5100
   agtctttcac aagattctgc gtatcactca ggaatacagt cacgtggcag tcctcacagt   5160
   tctagttctt atcattatca atctgagggc actgaaaggc aaaaaggtca atcaggttta   5220
   gtttggagac atggcagcta tggtagtgca g                                   5251
<210> 556
   <211> 1750
   <212> PRT
   <213> Homo sapiens
<400> 556
   Arg Thr Ser Arg Asn Arg Gly Ser Ser Phe Ser Gln Asp Ser Asp Ser
   1               5                   10                  15
   Gln Gly His Ser Glu Asp Ser Glu Arg Trp Ser Gly Ser Ala Ser Arg
               20                  25                  30
   Asn His His Gly Ser Ala Gln Glu Gln Leu Arg Asp Gly Ser Arg His
           35                  40                  45
   Pro Arg Ser His Gln Glu Asp Arg Ala Gly His Gly His Ser Ala Asp
       50                  55                  60
   Ser Ser Arg Gln Ser Gly Thr Arg His Thr Gln Thr Ser Ser Gly Gly
   65                  70                  75                  80
   Gln Ala Ala Ser Ser His Glu Gln Ala Arg Ser Ser Ala Gly Glu Arg
                   85                  90                  95
   His Gly Ser His His Gln Gln Ser Ala Asp Ser Ser Arg His Ser Gly
               100                 105                 110
   Ile Gly His Gly Gln Ala Ser Ser Ala Val Arg Asp Ser Gly His Arg
           115                 120                 125
   Gly Tyr Ser Gly Ser Gln Ala Ser Asp Asn Glu Gly His Ser Glu Asp
       130                 135                 140
   Ser Asp Thr Gln Ser Val Ser Ala His Gly Gln Ala Gly Ser His Gln
   145 150 155 160
   Gln Ser His Gln Glu Ser Ala Arg Gly Arg Ser Gly Glu Thr Ser Gly
   165 170 175
   His Ser Gly Ser Phe Leu Tyr Gln Val Ser Thr His Glu Gln Ser Glu
   180 185 190
   Ser Ser His Gly Trp Thr Gly Pro Ser Thr Arg Gly Arg Gln Gly Ser
   195 200 205
   Arg His Glu Gln Ala Gln Asp Ser Ser Arg His Ser Ala Ser Gln Asp
   210 215 220
   Gly Gln Asp Thr Ile Arg Gly His Pro Gly Ser Ser Arg Gly Gly Arg
   225 230 235 240
   Gln Gly Tyr His His Glu His Ser Val Asp Ser Ser Gly His Ser Gly
   245 250 255
   Ser His His Ser His Thr Thr Ser Gln Gly Arg Ser Asp Ala Ser Arg
   260 265 270
   Gly Gln Ser Gly Ser Arg Arg Ala Ser Arg Thr Thr Arg Asn Glu Glu
   275 280 285
   Gln Ser Gly Asp Gly Ser Arg His Ser Gly Ser Arg His His Glu Ala
   290 295 300
   Ser Thr His Ala Asp Ile Ser Arg His Ser Gln Ala Val Gln Gly Gln
   305 310 315 320
   Ser Glu Gly Ser Arg Arg Ser Arg Arg Gln Gly Ser Ser Val Ser Gln
   325 330 335
   Asp Ser Asp Ser Glu Gly His Ser Glu Asp Ser Glu Arg Trp Ser Gly
   340 345 350
   Ser Ala Ser Arg Asn His His Gly Ser Ala Gln Glu Gln Leu Arg Asp
   355 360 365
   Gly Ser Arg His Pro Arg Ser His Gln Glu Gly Arg Ala Gly His Gly
   370 375 380
   His Ser Ala Asp Ser Ser Arg Gln Ser Gly Thr Arg His Thr Gln Thr
   385 390 395 400
   Ser Ser Gly Gly Gln Ala Ala Ser Ser His Glu Gln Ala Arg Ser Ser
   405 410 415
   Ala Gly Glu Arg His Gly Ser His His Gln Gln Ser Ala Asp Ser Ser
               420                 425                 430
   Arg His Ser Gly Ile Gly His Gly Gln Ala Ser Ser Ala Val Arg Asp
           435                 440                 445
   Ser Gly His Arg Gly Tyr Ser Gly Ser Gln Ala Ser Asp Asn Glu Gly
       450                 455                 460
   His Ser Glu Asp Ser Asp Thr Gln Ser Val Ser Ala His Gly Gln Ala
   465                 470                 475                 480
   Gly Ser His Gln Gln Ser His Gln Glu Ser Ala Arg Gly Arg Ser Gly
                   485                 490                 495
   Glu Thr Ser Gly His Ser Gly Ser Phe Leu Tyr Gln Val Ser Thr His
               500                 505                 510
   Glu Gln Ser Glu Ser Ser His Gly Trp Thr Gly Pro Ser Thr Arg Gly
           515                 520                 525
   Arg Gln Gly Ser Arg His Glu Gln Ala Gln Asp Ser Ser Arg His Ser
       530                 535                 540
   Ala Ser Gln Asp Gly Gln Asp Thr Ile Arg Gly His Pro Gly Ser Ser
   545                 550                 555                 560
   Arg Gly Gly Arg Gln Gly Tyr His His Glu His Ser Val Asp Ser Ser
                   565                 570                 575
   Gly His Ser Gly Ser His His Ser His Thr Thr Ser Gln Gly Arg Ser
               580                 585                 590
   Asp Ala Ser Arg Gly Gln Ser Gly Ser Arg Ser Ala Ser Arg Thr Thr
           595                 600                 605
   Arg Asn Glu Glu Gln Ser Gly Asp Ser Ser Arg His Ser Gly Ser Arg
       610                 615                 620
   His His Glu Ala Ser Thr His Ala Asp Ile Ser Arg His Ser Gln Ala
   625                 630                 635                 640
   Val Gln Gly Gln Ser Glu Gly Ser Arg Arg Ser Arg Arg Gln Gly Ser
                   645                 650                 655
   Ser Val Ser Gln Asp Ser Asp Ser Glu Gly His Ser Glu Asp Ser Glu
               660                 665                 670
   Arg Trp Ser Gly Ser Ala Ser Arg Asn His Arg Gly Ser Val Gln Glu
           675                 680                 685
   Gln Ser Arg His Gly Ser Arg His Pro Arg Ser His His Glu Asp Arg
   690 695 700
   Ala Gly His Gly His Ser Ala Asp Arg Ser Arg Gln Ser Gly Thr Arg
   705 710 715 720
   His Ala Glu Thr Ser Ser Gly Gly Gln Ala Ala Ser Ser His Glu Gln
   725 730 735
   Ala Arg Ser Ser Pro Gly Glu Arg His Gly Ser Arg His Gln Gln Ser
   740 745 750
   Ala Asp Ser Ser Arg His Ser Gly Ile Pro Arg Gly Gln Ala Ser Ser
   755 760 765
   Ala Val Arg Asp Ser Arg His Trp Gly Ser Ser Gly Ser Gln Ala Ser
   770 775 780
   Asp Ser Glu Gly His Ser Glu Glu Ser Asp Thr Gln Ser Val Ser Gly
   785 790 795 800
   His Gly Gln Ala Gly Pro His Gln Gln Ser His Gln Glu Ser Ala Arg
   805 810 815
   Asp Arg Ser Gly Gly Arg Ser Gly Arg Ser Gly Ser Phe Leu Tyr Gln
   820 825 830
   Val Ser Thr His Glu Gln Ser Glu Ser Ala His Gly Arg Thr Arg Thr
   835 840 845
   Ser Thr Gly Arg Arg Gln Gly Ser His His Glu Gln Ala Arg Asp Ser
   850 855 860
   Ser Arg His Ser Ala Ser Gln Glu Gly Gln Asp Thr Ile Arg Ala His
   865 870 875 880
   Pro Gly Ser Ser Arg Arg Gly Arg Gln Gly Ser His Tyr Glu Gln Ser
   885 890 895
   Val Asp Arg Ser Gly His Ser Gly Ser His His Ser His Thr Thr Ser
   900 905 910
   Gln Gly Arg Ser Asp Ala Ser Arg Gly Gln Ser Gly Ser Arg Ser Ala
   915 920 925
   Ser Arg Gln Thr Arg Asn Asp Glu Gln Ser Gly Asp Gly Ser Arg His
   930 935 940
   Ser Trp Ser His His His Glu Ala Ser Thr Gln Ala Asp Ser Ser Arg
   945 950 955 960
   His Ser Gln Ser Gly Gln Gly Gln Ser Ala Gly Pro Ser Thr Ser Arg
   965 970 975
   Asn Gln Gly Ser Ser Val Ser Gln Asp Ser Asp Ser Gln Gly His Ser
   980 985 990
   Glu Asp Ser Glu Arg Trp Ser Gly Ser Ala Ser Arg Asn His His Gly
   995 1000 1005
   Ser Ala Gly Glu Gln Ser Arg Asp Gly Ser Arg His Pro Gly Ser
   1010 1015 1020
   His Gln Glu Asp Arg Ala Gly His Gly His Ser Ala Asp Ser Pro
   1025 1030 1035
   Arg Gln Ser Gly Thr Arg His Thr Glu Ser Ser Ser Arg Gly Gln
   1040 1045 1050
   Ala Ala Ser Ser His Glu Gln Ala Arg Ser Ser Ala Gly Glu Arg
   1055 1060 1065
   His Gly Ser His His Gln Leu Gln Ser Ala Asp Ser Ser Arg His
   1070 1075 1080
   Ala Gly Ile Gly His Gly Gln Ala Ser Ser Ala Val Arg Asp Ser
   1085 1090 1095
   Gly His Arg Gly Tyr Ser Gly Ser Gln Ala Thr Asp Ser Glu Gly
   1100 1105 1110
   His Ser Glu Asp Ser Asp Thr Gln Ser Val Ser Ala Gln Gly Lys
   1115 1120 1125
   Ala Gly Pro His Gln Gln Ser His Lys Glu Ser Ala Arg Gly Gln
   1130 1135 1140
   Ser Gly Glu Ser Ser Arg Arg Ser Gly Ser Phe Leu Tyr Gln Val
   1145 1150 1155
   Ser Thr His Glu Gln Ser Glu Ser Thr His Gly Gln Ser Val Pro
   1160 1165 1170
   Ser Thr Gly Gly Arg Gln Gly Ser His His Asp Gln Ala Gln Asp
   1175 1180 1185
   Ser Ser Arg His Ser Ala Ser Gln Glu Gly Gln Asp Thr Ile Arg
   1190 1195 1200
   Gly His Pro Gly Pro Ser Arg Gly Gly Arg Gln Gly Ser His His
   1205 1210 1215
   Glu Gln Ser Val Asp Arg Ser Gly His Ser Gly Ser His His Ser
   1220 1225 1230
   His Thr Thr Ser Gln Gly Arg Ser Asp Ala Ser Arg Gly Gln Ser
   1235 1240 1245
   Gly Pro Arg Ser Ala Ser Arg Gln Thr His Asp Lys Glu Gln Ser
   1250 1255 1260
   Gly Asp Gly Ser Arg His Ser Gly Ser Arg His His Glu Ala Ser
   1265 1270 1275
   Ser Trp Ala Asp Ser Ser Arg His Ser Gln Ala Val Gln Gly Gln
   1280 1285 1290
   Ser Glu Gly Ser Arg Arg Ser Arg Arg Gln Gly Ser Ser Val Ser
   1295 1300 1305
   Gln Asp Ser Asp Ser Gln Gly His Ser Glu Asp Ser Glu Arg Arg
   1310 1315 1320
   Ser Gly Ser Ala Ser Arg Asn His Arg Gly Ser Ala Gln Glu Gln
   1325 1330 1335
   Ser Arg Asp Gly Ser Arg His Pro Arg Ser His His Glu Asp Arg
   1340 1345 1350
   Ala Gly His Gly His Ser Ala Asp Ser Ser Arg Gln Ser Gly Thr
   1355 1360 1365
   His His Ala Glu Asn Ser Ser Gly Gly Gln Pro Ala Ser Ser His
   1370 1375 1380
   Glu Gln Ala Arg Ser Ser Ala Gly Glu Arg His Gly Ser His His
   1385 1390 1395
   Gln Gln Ser Ala Asp Ser Ser Arg His Ser Gly Ile Gly His Gly
   1400 1405 1410
   Gln Ala Ser Ser Ala Val Arg Asp Ser Gly His Arg Gly Ser Ser
   1415 1420 1425
   Gly Ser Gln Ala Ser Asp Ser Glu Gly His Ser Glu Asp Ser Asp
   1430 1435 1440
   Thr Gln Ser Val Ser Ala His Gly Gln Ala Gly Pro His Gln Gln
   1445 1450 1455
   Ser His Gln Glu Ser Thr Arg Gly Arg Ser Ala Gly Arg Ser Gly
   1460 1465 1470
   Arg Ser Gly Ser Phe Leu Tyr Gln Val Ser Thr His Glu Gln Ser
   1475 1480 1485
   Glu Ser Ala His Gly Arg Ala Gly Pro Ser Thr Gly Gly Arg Gln
   1490 1495 1500
   Gly Ser His His Glu Gln Ala Arg Asp Ser Ser Arg His Ser Ala
   1505 1510 1515
   Ser Gln Glu Gly Gln Asp Thr Ile Arg Gly His Pro Gly Ser Arg
   1520 1525 1530
   Arg Gly Gly Arg Gln Gly Ser Tyr His Glu Gln Ser Val Asp Arg
   1535 1540 1545
   Ser Gly His Ser Gly Ser His His Ser His Thr Thr Ser Gln Gly
   1550 1555 1560
   Arg Ser Asp Ala Ser His Gly Gln Ser Gly Ser Arg Ser Ala Ser
   1565 1570 1575
   Arg Glu Thr Arg Asn Glu Glu Gln Ser Gly Asp Gly Ser Arg His
   1580 1585 1590
   Ser Gly Ser Arg His His Glu Ala Ser Thr Gln Ala Asp Ser Ser
   1595 1600 1605
   Arg His Ser Gln Ser Gly Gln Gly Glu Ser Ala Gly Ser Arg Arg
   1610 1615 1620
   Ser Arg Arg Gln Gly Ser Ser Val Ser Gln Asp Ser Asp Ser Glu
   1625 1630 1635
   Ala Tyr Pro Glu Asp Ser Glu Arg Arg Ser Glu Ser Ala Ser Arg
   1640 1645 1650
   Asn His His Gly Ser Ser Arg Glu Gln Ser Arg Asp Gly Ser Arg
   1655 1660 1665
   His Pro Gly Ser Ser His Arg Asp Thr Ala Ser His Val Gln Ser
   1670 1675 1680
   Ser Pro Val Gln Ser Asp Ser Ser Thr Ala Lys Glu His Gly His
   1685 1690 1695
   Phe Ser Ser Leu Ser Gln Asp Ser Ala Tyr His Ser Gly Ile Gln
   1700 1705 1710
   Ser Arg Gly Ser Pro His Ser Ser Ser Ser Tyr His Tyr Gln Ser
   1715 1720 1725
   Glu Gly Thr Glu Arg Gln Lys Gly Gln Ser Gly Leu Val Trp Arg
   1730 1735 1740
   His Gly Ser Tyr Gly Ser Ala
   1745 1750
<210> 557
   <211> 6173
   <212> DNA
   <213> Homo sapiens

<400> 557
   aggacaagca ggaaccgggg atccagtttt agccaggaca gtgacagtca gggacactca 60
   gaagactctg agaggtggtc tgggtctgct tccagaaacc atcatggatc tgctcaggag 120
   cagctaagag atggctccag acaccccagg tcccatcaag aagacagagc tggtcatggg 180
   cactctgcag acagctccag acaatcaggc actcgtcaca cacagacttc ctctggtgga 240
   caggctgcat catcccatga acaggcaaga tcaagtgcag gagaaagaca tggatcccac 300
   caccagcagt cagcagacag ctccagacac tcaggcattg ggcacggaca agcttcatct 360
   gcagtcagag acagtggaca ccgagggtac agtggtagtc aggccagtga caatgaggga 420
   cattcagaag actcagacac acagtcagtg tcagcccacg gacaggctgg gtcccatcag 480
   cagagccacc aagagtccgc acgtggccgg tcaggggaaa cgtctggaca ttcagggtct 540
   ttcctctacc aggtgagcac tcatgaacag tctgagtcct cccatggatg gacggggccc 600
   agcactagag gaagacaagg atcccgccat gagcaggcac aagacagctc caggcactca 660
   gcatcccaag acggtcagga caccattcgt ggacacccgg ggtcaagcag aggaggaagg 720
   caggggtacc accacgagca ttcggtagat agctctggac actcagggtc ccatcacagc 780
   cacaccacat cccagggaag gtctgatgcc tcccgtgggc agtcaggatc cagacgtgca 840
   agcagaacaa cacgtaatga ggaacaatca ggagacggct ccaggcactc agggtcgcgt 900
   caccatgaag cttccactca tgccgacatc tctagacact cacaggcagt ccagggacaa 960
   tcagaggggt ccaggagaag caggcgccag ggatccagtg ttagccagga cagtgacagt 1020
   gagggacatt cagaagactc tgagaggtgg tctgggtctg cttccagaaa ccatcatgga 1080
   tctgctcagg agcagctaag agatggctcc agacacccca ggtcccatca agaaggcaga 1140
   gctggtcatg tcctctggtg gacaggctgc atcatcccat gaacaggcaa gatcaagtgc 1200
   aggagaaaga catggatccc accaccagca gtcagcagac agctccagac actcaggcat 1260
   tgggcacgga caagcttcat ctgcagtcag agacagtgga caccgagggt acagtggtag 1320
   tcaggccagt gacaatgagg gacattcaga agactcagac acacagtcag tgtcagccca 1380
   cggacaggct gggtcccatc agcagagcca ccaagagtcc gcacgtggcc ggtcagggga 1440
   aacgtctgga cattcaggat ctttcctcta ccaggtgagc actcatgaac agtctgagtc 1500
   ctcccatgga tggacggggc ccagcactag aggaagacaa ggatcccgcc atgagcaggc 1560
   acaagacagc tccaggcact cagcatccca agatggtcag gacaccattc gtggacaccc 1620
   ggggtcaagc agaggaggaa ggcaggggta ccaccacgag cattcggtag atagctctgg 1680
   acactcaggg tcccatcaca gccacaccac atcccaggga aggtctgatg cctcccgtgg 1740
   gcagtcagga tccagaagtg caagcagaac aacacgtaat gaggaacaat caggagacgg 1800
   ctccaggcac tcagggtcgc gtcaccatga agcttccact catgccgaca tctctagaca 1860
   ctcacaggca gtccagggac aatcagaggg gtccaggaga agcaggcgcc agggatccag 1920
   tgttagccag gacagtgaca gtgagggaca ttcagaagac tctgagaggt ggtctgggtc 1980
   tgcttccaga aaccatcatg gatctgctca ggagcagcta agagatggct ccagacaccc 2040
   caggtcccat caagaagaca gagctggtca tgggcactct gcagacagct ccagacaatc 2100
   aggcactcgt cacacacagg cttcctctgg tggacaggct gcatcatccc atgaacaggc 2160
   aagatcaagt gcaggagaaa gacatggatc ccaccaccag cagtcagcag acagctccag 2220
   acactcaggc attgggcacg gacaagcttc atctgcagtc agagacagtg gacaccgagg 2280
   gtacagtggt agtcaggcca gtgacaatga gggacattca gaagactcag acacacagtc 2340
   agtgtcagcc cacggacagg ctgggtccca tcagcagagc caccaagagt ccgcacgtgg 2400
   ccggtcaggg gaaacgtctg gacattcagg atctttcctc taccaggtga gcactcatga 2460
   acagtctgag tcctcccatg gatggacggg gcccagcact agaggaagac aaggatcccg 2520
   ccatgagcag gcacaagaca gctccaggca ctcagcatcc caagacggtc aggacaccat 2580
   tcgtggacac ccggggtcaa gcagaggagg aaggcagggg taccaccacg agcattcggt 2640
   agatagctct ggacactcag ggtcccatca cagccacacc acatcccagg gaaggtctga 2700
   tgcctcccgt gggcagtcag gatccagaag tgcaagcaga acaacacgta atgaggaaca 2760
   atcaggagac agctccaggc actcagggtc gcgtcaccat gaagcttcca ctcatgccga 2820
   catctctaga cactcacagg cagtccaggg acaatcagag gggtccagga gaagcaggcg 2880
   ccagggatcc agtgttagcc aggacagtga cagtgaggga cattcagaag actctgagag 2940
   gtggtctggg tctgcttcca gaaaccatcg tggatctgtt caggagcagt caaggcacgg 3000
   ctccagacac cccaggtccc atcacgaaga cagagccggt cacgggcact ctgcagaccg 3060
   ctccagacaa tcaggcactc gtcacgcaga gacttcctct ggtggacagg ctgcatcatc 3120
   ccatgaacag gcaagatcaa gtccaggaga gagacacgga tcccgccacc agcagtcagc 3180
   agacagctcc agacactcag gcattccgcg tggacaggct tcatctgcag tcagagacag 3240
   tagacactgg gggtccagtg gtagtcaagc cagtgatagt gagggacatt cagaagagtc 3300
   agacacacag tcagtgtcag gccatggaca ggctgggccc catcagcaga gccaccaaga 3360
   gtccgcacgt gaccggtcag ggggaaggtc tggacgttca gggtctttcc tctaccaggt 3420
   gagcactcat gaacagtctg agtccgccca tgggcggacc aggaccagca ctggacgaag 3480
   acaaggatcc caccacgagc aggcacgaga cagctccagg cactcagcgt cccaagaggg 3540
   tcaggacacc attcgtgcac acccggggtc aagcagaaga ggaaggcagg gatcccacta 3600
   cgagcaatcg gtagataggt ctggacactc agggtcccat cacagccaca ccacatccca 3660
   gggaaggtct gatgcctccc gtgggcagtc aggatccaga agtgccagca gacaaactcg 3720
   taacgacgaa caatcaggag acggctccag gcactcatgg tcgcatcacc atgaagcttc 3780
   cactcaggcg gacagctcta gacactcaca gtccggccag ggacaatcag cggggcccag 3840
   tacaagcagg aaccagggat ccagtgttag ccaggacagt gacagtcagg gacactcaga 3900
   agactctgag aggtggtctg ggtctgcttc cagaaaccat catggatctg ctggggagca 3960
   gtcaagagat ggctccagac accctgggtc ccatcaagaa gacagagccg gtcacgggca 4020
   ctctgcagac agccccagac aatcaggcac tcgtcacaca gagtcttcct ctcgtggaca 4080
   ggctgcgtca tcccatgaac aggcaagatc aagtgcagga gaaagacatg gatcccacca 4140
   ccagctccag tcagcagaca gctccagaca cgcaggcatt gggcacggac aagcttcatc 4200
   tgcagtcaga gacagtggac accgagggta cagtggtagt caggccactg acagtgaggg 4260
   acattcagaa gactcagaca cacagtcagt gtcagcacag ggaaaagctg ggccccatca 4320
   gcagagccac aaagagtccg cacgtggcca gtcaggggaa agctctagac gttcagggtc 4380
   tttcctctac caggtgagca ctcatgaaca gtctgagtcc acccatggac agtctgtgcc 4440
   cagcactgga ggaagacaag gatcccacca tgatcaggca caagacagct ccaggcactc 4500
   agcatcccaa gagggtcagg acaccattcg tggacacccg gggccaagca gaggaggaag 4560
   acaggggtcc caccacgagc aatcggtaga taggtctgga cactcagggt cccatcacag 4620
   ccacaccaca tcccagggaa ggtctgatgc ctcccgtggg cagtcaggac ccagaagtgc 4680
   aagcagacaa acacatgaca aggaacaatc aggagacggc tctaggcact cagggtcgcg 4740
   tcatcatgaa gcttcctctt gggccgacag ctctagacac tcacaggcag tccagggaca 4800
   atcagagggg tccaggagaa gcaggcgcca gggatccagt gttagccagg acagtgacag 4860
   tcagggacac tcagaagact ctgagaggcg gtctgggtct gcttccagaa accatcgtgg 4920
   atctgctcag gagcagtcaa gagatggctc cagacacccc aggtcccatc acgaagacag 4980
   agccggtcat gggcactctg cagacagctc cagacaatca ggcactcatc atgcagagaa 5040
   ttcctctggt ggacagcctg catcatccca tgaacaggca agatcaagtg caggagagag 5100
   acatggatcc caccaccagc agtcagcaga cagctccaga cactcaggca ttgggcacgg 5160
   acaagcttca tctgcagtca gagacagtgg acaccgaggg tccagtggta gtcaggccag 5220
   tgacagtgag ggacattcag aagactcaga cacacagtca gtgtcagccc acggacaggc 5280
   tgggccccat cagcagagcc accaagagtc cacacgtggc cggtcagcag gaaggtctgg 5340
   acgttcaggg tctttcctct accaggtgag cactcatgaa cagtctgagt ctgcccatgg 5400
   acgggctggg cccagtactg gaggaagaca aggatcccac cacgagcagg cacgagacag 5460
   ctccaggcac tcagcgtccc aagagggtca ggacaccatt cgtggacacc cggggtcaag 5520
   gagaggagga agacagggat cctaccacga gcaatcggta gataggtctg gacactcagg 5580
   gtcccatcac agccacacca catcccaggg aaggtctgat gcctcccatg ggcagtcagg 5640
   atccagaagt gcaagcagag aaacacgtaa tgaggaacag tcaggagacg gctccaggca 5700
   ctcagggtcg cgtcaccatg aagcttccac tcaggctgac agctctagac actcacagtc 5760
   cggccagggt gaatcagcgg ggtccaggag aagcaggcgc cagggatcca gtgttagcca 5820
   ggacagtgac agtgaggcat acccagagga ctctgagagg cgatctgagt ctgcttccag 5880
   aaaccatcat ggatcttctc gggagcagtc aagagatggc tccagacacc ccggatcctc 5940
   tcaccgcgat acagccagtc atgtacagtc ttcacctgta cagtcagact ctagtaccgc 6000
   taaggaacat ggtcacttta gtagtctttc acaagattct gcgtatcact caggaataca 6060
   gtcacgtggc agtcctcaca gttctagttc ttatcattat caatctgagg gcactgaaag 6120
   gcaaaaaggt caatcaggtt tagtttggag acatggcagc tatggtagtg cag 6173
<210> 558
   <211> 2074
   <212> PRT
   <213> Homo sapiens
<400> 558
   Arg Thr Ser Arg Asn Arg Gly Ser Ser Phe Ser Gln Asp Ser Asp Ser
   1 5 10 15
   Gln Gly His Ser Glu Asp Ser Glu Arg Trp Ser Gly Ser Ala Ser Arg
   20 25 30
   Asn His His Gly Ser Ala Gln Glu Gln Leu Arg Asp Gly Ser Arg His
   35 40 45
   Pro Arg Ser His Gln Glu Asp Arg Ala Gly His Gly His Ser Ala Asp
   50 55 60
   Ser Ser Arg Gln Ser Gly Thr Arg His Thr Gln Thr Ser Ser Gly Gly
   65 70 75 80
   Gln Ala Ala Ser Ser His Glu Gln Ala Arg Ser Ser Ala Gly Glu Arg
   85 90 95
   His Gly Ser His His Gln Gln Ser Ala Asp Ser Ser Arg His Ser Gly
   100 105 110
   Ile Gly His Gly Gln Ala Ser Ser Ala Val Arg Asp Ser Gly His Arg
   115 120 125
   Gly Tyr Ser Gly Ser Gln Ala Ser Asp Asn Glu Gly His Ser Glu Asp
   130 135 140
   Ser Asp Thr Gln Ser Val Ser Ala His Gly Gln Ala Gly Ser His Gln
   145 150 155 160
   Gln Ser His Gln Glu Ser Ala Arg Gly Arg Ser Gly Glu Thr Ser Gly
   165 170 175
   His Ser Gly Ser Phe Leu Tyr Gln Val Ser Thr His Glu Gln Ser Glu
   180 185 190
   Ser Ser His Gly Trp Thr Gly Pro Ser Thr Arg Gly Arg Gln Gly Ser
   195 200 205
   Arg His Glu Gln Ala Gln Asp Ser Ser Arg His Ser Ala Ser Gln Asp
   210 215 220
   Gly Gln Asp Thr Ile Arg Gly His Pro Gly Ser Ser Arg Gly Gly Arg
   225 230 235 240
   Gln Gly Tyr His His Glu His Ser Val Asp Ser Ser Gly His Ser Gly
   245 250 255
   Ser His His Ser His Thr Thr Ser Gln Gly Arg Ser Asp Ala Ser Arg
   260 265 270
   Gly Gln Ser Gly Ser Arg Arg Ala Ser Arg Thr Thr Arg Asn Glu Glu
   275 280 285
   Gln Ser Gly Asp Gly Ser Arg His Ser Gly Ser Arg His His Glu Ala
   290 295 300
   Ser Thr His Ala Asp Ile Ser Arg His Ser Gln Ala Val Gln Gly Gln
   305 310 315 320
   Ser Glu Gly Ser Arg Arg Ser Arg Arg Gln Gly Ser Ser Val Ser Gln
   325 330 335
   Asp Ser Asp Ser Glu Gly His Ser Glu Asp Ser Glu Arg Trp Ser Gly
   340 345 350
   Ser Ala Ser Arg Asn His His Gly Ser Ala Gln Glu Gln Leu Arg Asp
   355 360 365
   Gly Ser Arg His Pro Arg Ser His Gln Glu Gly Arg Ala Gly His Gly
   370 375 380
   His Ser Ala Asp Ser Ser Arg Gln Ser Gly Thr Arg His Thr Gln Thr
   385 390 395 400
   Ser Ser Gly Gly Gln Ala Ala Ser Ser His Glu Gln Ala Arg Ser Ser
   405 410 415
   Ala Gly Glu Arg His Gly Ser His His Gln Gln Ser Ala Asp Ser Ser
   420 425 430
   Arg His Ser Gly Ile Gly His Gly Gln Ala Ser Ser Ala Val Arg Asp
   435 440 445
   Ser Gly His Arg Gly Tyr Ser Gly Ser Gln Ala Ser Asp Asn Glu Gly
   450 455 460
   His Ser Glu Asp Ser Asp Thr Gln Ser Val Ser Ala His Gly Gln Ala
   465 470 475 480
   Gly Ser His Gln Gln Ser His Gln Glu Ser Ala Arg Gly Arg Ser Gly
   485 490 495
   Glu Thr Ser Gly His Ser Gly Ser Phe Leu Tyr Gln Val Ser Thr His
   500 505 510
   Glu Gln Ser Glu Ser Ser His Gly Trp Thr Gly Pro Ser Thr Arg Gly
   515 520 525
   Arg Gln Gly Ser Arg His Glu Gln Ala Gln Asp Ser Ser Arg His Ser
   530 535 540
   Ala Ser Gln Asp Gly Gln Asp Thr Ile Arg Gly His Pro Gly Ser Ser
   545 550 555 560
   Arg Gly Gly Arg Gln Gly Tyr His His Glu His Ser Val Asp Ser Ser
   565 570 575
   Gly His Ser Gly Ser His His Ser His Thr Thr Ser Gln Gly Arg Ser
   580 585 590
   Asp Ala Ser Arg Gly Gln Ser Gly Ser Arg Ser Ala Ser Arg Thr Thr
   595 600 605
   Arg Asn Glu Glu Gln Ser Gly Asp Gly Ser Arg His Ser Gly Ser Arg
   610 615 620
   His His Glu Ala Ser Thr His Ala Asp Ile Ser Arg His Ser Gln Ala
   625 630 635 640
   Val Gln Gly Gln Ser Glu Gly Ser Arg Arg Ser Arg Arg Gln Gly Ser
   645 650 655
   Ser Val Ser Gln Asp Ser Asp Ser Glu Gly His Ser Glu Asp Ser Glu
   660 665 670
   Arg Trp Ser Gly Ser Ala Ser Arg Asn His His Gly Ser Ala Gln Glu
   675 680 685
   Gln Leu Arg Asp Gly Ser Arg His Pro Arg Ser His Gln Glu Asp Arg
   690 695 700
   Ala Gly His Gly His Ser Ala Asp Ser Ser Arg Gln Ser Gly Thr Arg
   705 710 715 720
   His Thr Gln Ala Ser Ser Gly Gly Gln Ala Ala Ser Ser His Glu Gln
   725 730 735
   Ala Arg Ser Ser Ala Gly Glu Arg His Gly Ser His His Gln Gln Ser
   740 745 750
   Ala Asp Ser Ser Arg His Ser Gly Ile Gly His Gly Gln Ala Ser Ser
   755 760 765
   Ala Val Arg Asp Ser Gly His Arg Gly Tyr Ser Gly Ser Gln Ala Ser
   770 775 780
   Asp Asn Glu Gly His Ser Glu Asp Ser Asp Thr Gln Ser Val Ser Ala
   785 790 795 800
   His Gly Gln Ala Gly Ser His Gln Gln Ser His Gln Glu Ser Ala Arg
   805 810 815
   Gly Arg Ser Gly Glu Thr Ser Gly His Ser Gly Ser Phe Leu Tyr Gln
   820 825 830
   Val Ser Thr His Glu Gln Ser Glu Ser Ser His Gly Trp Thr Gly Pro
   835 840 845
   Ser Thr Arg Gly Arg Gln Gly Ser Arg His Glu Gln Ala Gln Asp Ser
   850 855 860
   Ser Arg His Ser Ala Ser Gln Asp Gly Gln Asp Thr Ile Arg Gly His
   865 870 875 880
   Pro Gly Ser Ser Arg Gly Gly Arg Gln Gly Tyr His His Glu His Ser
   885 890 895
   Val Asp Ser Ser Gly His Ser Gly Ser His His Ser His Thr Thr Ser
   900 905 910
   Gln Gly Arg Ser Asp Ala Ser Arg Gly Gln Ser Gly Ser Arg Ser Ala
   915 920 925
   Ser Arg Thr Thr Arg Asn Glu Glu Gln Ser Gly Asp Ser Ser Arg His
   930 935 940
   Ser Gly Ser Arg His His Glu Ala Ser Thr His Ala Asp Ile Ser Arg
   945 950 955 960
   His Ser Gln Ala Val Gln Gly Gln Ser Glu Gly Ser Arg Arg Ser Arg
   965 970 975
   Arg Gln Gly Ser Ser Val Ser Gln Asp Ser Asp Ser Glu Gly His Ser
   980 985 990
   Glu Asp Ser Glu Arg Trp Ser Gly Ser Ala Ser Arg Asn His Arg Gly
   995 1000 1005
   Ser Val Gln Glu Gln Ser Arg His Gly Ser Arg His Pro Arg Ser
   1010 1015 1020
   His His Glu Asp Arg Ala Gly His Gly His Ser Ala Asp Arg Ser
   1025 1030 1035
   Arg Gln Ser Gly Thr Arg His Ala Glu Thr Ser Ser Gly Gly Gln
   1040 1045 1050
   Ala Ala Ser Ser His Glu Gln Ala Arg Ser Ser Pro Gly Glu Arg
   1055 1060 1065
   His Gly Ser Arg His Gln Gln Ser Ala Asp Ser Ser Arg His Ser
   1070 1075 1080
   Gly Ile Pro Arg Gly Gln Ala Ser Ser Ala Val Arg Asp Ser Arg
   1085 1090 1095
   His Trp Gly Ser Ser Gly Ser Gln Ala Ser Asp Ser Glu Gly His
   1100 1105 1110
   Ser Glu Glu Ser Asp Thr Gln Ser Val Ser Gly His Gly Gln Ala
   1115 1120 1125
   Gly Pro His Gln Gln Ser His Gln Glu Ser Ala Arg Asp Arg Ser
   1130 1135 1140
   Gly Gly Arg Ser Gly Arg Ser Gly Ser Phe Leu Tyr Gln Val Ser
   1145 1150 1155
   Thr His Glu Gln Ser Glu Ser Ala His Gly Arg Thr Arg Thr Ser
   1160 1165 1170
   Thr Gly Arg Arg Gln Gly Ser His His Glu Gln Ala Arg Asp Ser
   1175 1180 1185
   Ser Arg His Ser Ala Ser Gln Glu Gly Gln Asp Thr Ile Arg Ala
   1190 1195 1200
   His Pro Gly Ser Ser Arg Arg Gly Arg Gln Gly Ser His Tyr Glu
   1205 1210 1215
   Gln Ser Val Asp Arg Ser Gly His Ser Gly Ser His His Ser His
   1220 1225 1230
   Thr Thr Ser Gln Gly Arg Ser Asp Ala Ser Arg Gly Gln Ser Gly
   1235 1240 1245
   Ser Arg Ser Ala Ser Arg Gln Thr Arg Asn Asp Glu Gln Ser Gly
   1250 1255 1260
   Asp Gly Ser Arg His Ser Trp Ser His His His Glu Ala Ser Thr
   1265 1270 1275
   Gln Ala Asp Ser Ser Arg His Ser Gln Ser Gly Gln Gly Gln Ser
   1280 1285 1290
   Ala Gly Pro Ser Thr Ser Arg Asn Gln Gly Ser Ser Val Ser Gln
   1295 1300 1305
   Asp Ser Asp Ser Gln Gly His Ser Glu Asp Ser Glu Arg Trp Ser
   1310 1315 1320
   Gly Ser Ala Ser Arg Asn His His Gly Ser Ala Gly Glu Gln Ser
   1325 1330 1335
   Arg Asp Gly Ser Arg His Pro Gly Ser His Gln Glu Asp Arg Ala
   1340 1345 1350
   Gly His Gly His Ser Ala Asp Ser Pro Arg Gln Ser Gly Thr Arg
   1355 1360 1365
   His Thr Glu Ser Ser Ser Arg Gly Gln Ala Ala Ser Ser His Glu
   1370 1375 1380
   Gln Ala Arg Ser Ser Ala Gly Glu Arg His Gly Ser His His Gln
   1385 1390 1395
   Leu Gln Ser Ala Asp Ser Ser Arg His Ala Gly Ile Gly His Gly
   1400 1405 1410
   Gln Ala Ser Ser Ala Val Arg Asp Ser Gly His Arg Gly Tyr Ser
   1415 1420 1425
   Gly Ser Gln Ala Thr Asp Ser Glu Gly His Ser Glu Asp Ser Asp
   1430 1435 1440
   Thr Gln Ser Val Ser Ala Gln Gly Lys Ala Gly Pro His Gln Gln
   1445 1450 1455
   Ser His Lys Glu Ser Ala Arg Gly Gln Ser Gly Glu Ser Ser Arg
   1460 1465 1470
   Arg Ser Gly Ser Phe Leu Tyr Gln Val Ser Thr His Glu Gln Ser
   1475 1480 1485
   Glu Ser Thr His Gly Gln Ser Val Pro Ser Thr Gly Gly Arg Gln
   1490 1495 1500
   Gly Ser His His Asp Gln Ala Gln Asp Ser Ser Arg His Ser Ala
   1505 1510 1515
   Ser Gln Glu Gly Gln Asp Thr Ile Arg Gly His Pro Gly Pro Ser
   1520 1525 1530
   Arg Gly Gly Arg Gln Gly Ser His His Glu Gln Ser Val Asp Arg
   1535 1540 1545
   Ser Gly His Ser Gly Ser His His Ser His Thr Thr Ser Gln Gly
   1550 1555 1560
   Arg Ser Asp Ala Ser Arg Gly Gln Ser Gly Pro Arg Ser Ala Ser
   1565 1570 1575
   Arg Gln Thr His Asp Lys Glu Gln Ser Gly Asp Gly Ser Arg His
   1580 1585 1590
   Ser Gly Ser Arg His His Glu Ala Ser Ser Trp Ala Asp Ser Ser
   1595 1600 1605
   Arg His Ser Gln Ala Val Gln Gly Gln Ser Glu Gly Ser Arg Arg
   1610 1615 1620
   Ser Arg Arg Gln Gly Ser Ser Val Ser Gln Asp Ser Asp Ser Gln
   1625 1630 1635
   Gly His Ser Glu Asp Ser Glu Arg Arg Ser Gly Ser Ala Ser Arg
   1640 1645 1650
   Asn His Arg Gly Ser Ala Gln Glu Gln Ser Arg Asp Gly Ser Arg
   1655 1660 1665
   His Pro Arg Ser His His Glu Asp Arg Ala Gly His Gly His Ser
   1670 1675 1680
   Ala Asp Ser Ser Arg Gln Ser Gly Thr His His Ala Glu Asn Ser
   1685 1690 1695
   Ser Gly Gly Gln Pro Ala Ser Ser His Glu Gln Ala Arg Ser Ser
   1700 1705 1710
   Ala Gly Glu Arg His Gly Ser His His Gln Gln Ser Ala Asp Ser
   1715 1720 1725
   Ser Arg His Ser Gly Ile Gly His Gly Gln Ala Ser Ser Ala Val
   1730 1735 1740
   Arg Asp Ser Gly His Arg Gly Ser Ser Gly Ser Gln Ala Ser Asp
   1745 1750 1755
   Ser Glu Gly His Ser Glu Asp Ser Asp Thr Gln Ser Val Ser Ala
   1760 1765 1770
   His Gly Gln Ala Gly Pro His Gln Gln Ser His Gln Glu Ser Thr
   1775 1780 1785
   Arg Gly Arg Ser Ala Gly Arg Ser Gly Arg Ser Gly Ser Phe Leu
   1790 1795 1800
   Tyr Gln Val Ser Thr His Glu Gln Ser Glu Ser Ala His Gly Arg
   1805 1810 1815
   Ala Gly Pro Ser Thr Gly Gly Arg Gln Gly Ser His His Glu Gln
   1820 1825 1830
   Ala Arg Asp Ser Ser Arg His Ser Ala Ser Gln Glu Gly Gln Asp
   1835 1840 1845
   Thr Ile Arg Gly His Pro Gly Ser Arg Arg Gly Gly Arg Gln Gly
   1850 1855 1860
   Ser Tyr His Glu Gln Ser Val Asp Arg Ser Gly His Ser Gly Ser
   1865 1870 1875
   His His Ser His Thr Thr Ser Gln Gly Arg Ser Asp Ala Ser His
   1880 1885 1890
   Gly Gln Ser Gly Ser Arg Ser Ala Ser Arg Glu Thr Arg Asn Glu
   1895 1900 1905
   Glu Gln Ser Gly Asp Gly Ser Arg His Ser Gly Ser Arg His His
   1910 1915 1920
   Glu Ala Ser Thr Gln Ala Asp Ser Ser Arg His Ser Gln Ser Gly
   1925 1930 1935
   Gln Gly Glu Ser Ala Gly Ser Arg Arg Ser Arg Arg Gln Gly Ser
   1940 1945 1950
   Ser Val Ser Gln Asp Ser Asp Ser Glu Ala Tyr Pro Glu Asp Ser
   1955 1960 1965
   Glu Arg Arg Ser Glu Ser Ala Ser Arg Asn His His Gly Ser Ser
   1970 1975 1980
   Arg Glu Gln Ser Arg Asp Gly Ser Arg His Pro Gly Ser Ser His
   1985 1990 1995
   Arg Asp Thr Ala Ser His Val Gln Ser Ser Pro Val Gln Ser Asp
   2000 2005 2010
   Ser Ser Thr Ala Lys Glu His Gly His Phe Ser Ser Leu Ser Gln
   2015 2020 2025
   Asp Ser Ala Tyr His Ser Gly Ile Gln Ser Arg Gly Ser Pro His
   2030 2035 2040
   Ser Ser Ser Ser Tyr His Tyr Gln Ser Glu Gly Thr Glu Arg Gln
   2045 2050 2055
   Lys Gly Gln Ser Gly Leu Val Trp Arg His Gly Ser Tyr Gly Ser
   2060 2065 2070
   Ala